# EUROPEAN PATENT APPLICATION

(11) **EP 3 456 300 A1**
(43) Date of publication of application: **20.03.2019**
(21) Application number: 18186369.7
(22) Date of filing: 02.05.2014
(51) Int. Cl.: A61F 13/00, A61F 15/00, A61B 17/00, A61B 17/04

(54) **MICRODRESSINGS FOR SKIN TREATMENT**

(30) Priority: 03.05.2013 US 201361819190 P
(62) Divisional of application: 14791060.8
(71) Applicant: Cytrellis Biosystems, Inc., Woburn, MA 01801 (US)
(72) Inventor: Levinson, Douglas, Sherborn, Massachusetts 01770 (US); Stone, David, Acton, Massachusetts 01720 (US); Ginggen, Alec, Medford, Massachusetts 02155 (US)
(74) Representative: Bösl, Raphael Konrad

(57) **Abstract**

The present invention relates to microclosures and methods for treating microwounds in the skin (e.g., after incising or excising tissue portions from a subject). Exemplary microclosures include a material having at least one dimension of from about 10 um to about 1 mm after application to a microwound. The microclosure maintains a first compressive force when applied to the microwound.

## Description

### Cross-Reference to Related Applications

This application claims benefit to U.S. Provisional Application No. 61/819,190, filed May 3, 2013, which is hereby incorporated by reference.

### Background of the Invention

This invention relates to methods and devices for treating skin, such as skin tightening or for treating diseases, disorders, and conditions that would benefit from tissue area or volume reduction, skin restoration, or skin tightening.

Many human health issues arise from the damage or loss of tissue due to disease, advanced age, and/or injury. In aesthetic medicine, elimination of excess tissue and/or skin laxity is an important concern that affects more than 25% of the U.S. population. Conventional surgical therapies (e.g., a face lift, brow lift, or breast lift) can be effective but are often invasive, inconvenient, and expensive, while scarring limits its applicability.

Although minimally invasive methods are available, such methods are generally less effective than surgical methods. Methods using energy sources (e.g., laser, non-coherent light, radiofrequency, or ultrasound) can be effective at improving the architecture and the texture of the skin but are much less effective at tightening the skin or reducing skin laxity. Neurotoxins, such as botulinum toxin, reduce the formation of dynamic wrinkles by paralysis of the injected muscles, but such toxins have minimal or no effect on skin tightness or laxity. Finally, dermal fillers, such as hyaluronic acid, are injected in the dermal layer to smooth out wrinkles and improve contours, but such fillers do not tighten or reduce laxity of the skin. Thus, surgical therapies remain the gold standard for lifting and/or tightening skin, as compared to energy-based techniques (e.g., with laser, radiofrequency, or ultrasound ablation) and injection-based techniques (e.g., with botulinum toxin or hyaluronic acid- or collagen-based fillers).

Accordingly, there is a need for improved methods and devices that increase the effectiveness of minimally-invasive techniques while maintaining convenience, affordability, and/or accessibility to patients requiring tissue restoration.

### Summary of the Invention

This invention relates to methods and devices (e.g., a microclosure) for treating skin by selective opening or closing a plurality of small slits or holes (e.g., microwounds) formed by incision or excision of tissue portions. For example, tissue excision can be performed by fractional ablation of the epidermal and/or dermal layer of the skin with a hollow coring needle, by fractional laser ablation, by fractional radiofrequency ablation, or by fractional ultrasonic ablation. Various methods and devices (e.g., microclosures) are provided to close small wounds, which may include smart or tunable microclosures, that allow for titration of the tightening effect after application to the skin of a subject.

The present invention features a microclosure including a material having at least one dimension of from about 10 µm to about 1 mm (e.g., including any ranges described herein) after application to a microwound, where the microclosure maintains a first compressive force when applied to the microwound. Specifically, such microclosures have no dimension that is larger than 5 mm (e.g., no dimension larger than 4, 3, 2, or 1 mm). In some embodiments, the microclosure has an areal dimension of less than about 4 mm² (e.g., including any ranges described herein).

In some embodiments, the microclosure includes a microstaple (e.g., having a circular geometry), a microdressing, a microweld, a suture, or a sealant (e.g., a resorbable or non-resorbable sealant). In other embodiments, the microstaple further includes one or more (e.g., two, three, four, five, six, seven, eight, or more) tips and/or one or more (e.g., two, three, four, five, six, seven, eight, or more) sharp edges. In some embodiments, the microstaple is pre-constrained prior to application to the microwound.

In cetain embodiments where the microclosure is a staple, the dimensions can be, e.g., 200 µm by 200 µm by 2 mm. In certain embodiments where the microclosure is a disc shaped microdressing, the disc can have a diameter of, less than 2 mm (e.g., 1 mm, or less). In certain embodiments where the microclosure is a suture, the suture can have a diameter of 100 µm, or less.

In some embodiments, the microdressing includes an adhesive layer and a regulatable layer that includes one or more materials where exposure of the regulatable layer to one or more external stimuli results in a change in a physical characteristic in the one or more materials in at least a portion of the microdressing. In other embodiments, the change in a physical characteristic includes an increase in tension of the microdressing, a decrease in tension of the microdressing, an increase in compressive force exerted by the microdressing, a decrease in compressive force exerted by the microdressing, compression in one or more directions of the microdressing, and/or expansion in one or more directions of the microdressing. In yet other embodiments, the microdressing includes an adhesive layer and a pre-stretched or unstretched layer.

In any of the devices, apparatuses, and methods described herein, the material (e.g., of the microclosure, either in the bulk material or a portion thereof) includes one or more of a metal, a metal alloy, a plastic, a polymer, a shape-memory polymer, a shape-memory alloy, a thermal-responsive material, a pH-responsive material, a light-responsive material, a moisture-responsive material, a solvent-responsive or chemical exposure-responsive material, an electric field-responsive material, a magnetic field-responsive material, an actuator-embedded material, an unstretched material, a pre-stretched material, an adhesive, a biocompatible matrix, a photosensitizer, a photochemical agent, a synthetic glue, a biologic sealant, a biodegradable adhesive, a tissue glue, or a resorbable material.

In any of the devices, apparatuses, and methods described herein, the first compressive force includes a compression in one or more directions and/or an expansion in one or more directions (e.g., in the x-, y-, z-, xy-, xz-, yz-, and/or xyz-directions), as compared to before application to the microwound. In some embodiments, the compression or the expansion is an increase or decrease (e.g., an increase or decrease of at least about 0.5% (e.g., at least about 0.6%, 0.7%, 0.8%, 0.9%, 1.0%, 1.1%, 1.2%, 1.5%, 1.7%, 2.0%, 2.2%, 2.5%, 2.7%, 3%, 3.5%, 4%, 4.5%, 5%, 5.5%, 6%, 6.5%, 7%, 7.5%, 8%, 8.5%, 9%, 9.5%, 10%, 10.5%, 15%, 20%, 30%, 40%, 50% or more) or from about 0.5% to 50%, as described herein) in the x-axis, y-axis, and/or z-axis of the microclosure, as compared to before application to the microwound.

In some embodiments, the microclosure further includes an attachment component adapted to facilitate removal of the microclosure. In further embodiments, the attachment component includes a hook, a pincher, an eye, a loop, a post, a microfastener, a slot, a snap fastener, or a combination thereof.

The present invention also features an array including a plurality (e.g., about 2, 3, 4, 5, 6, 7, 8, 9, 10, 15, 20, 25, 30, 35, 40, 45, 50, 75, 100, 400 per cm² or more, such as between about 2 and 400 per cm², (e.g., between 2 and 10, 2 and 15, 2 and 20, 2 and 25, 2 and 30, 2 and 35, 2 and 40, 2 and 45, 2 and 50, 2 and 75, 5 and 10, 5 and 15, 5 and 20, 5 and 25, 5 and 30, 5 and 35, 5 and 40, 5 and 45, 5 and 50, 5 and 75, 5 and 100, 10 and 20, 10 and 25, 10 and 30, 10 and 35, 10 and 40, 10 and 45, 10 and 50, 10 and 75, 10 and 100, 15 and 20, 15 and 25, 15 and 30, 15 and 35, 15 and 40, 15 and 45, 15 and 50, 15 and 75, 15 and 100, 20 and 25, 20 and 30, 20 and 35, 20 and 40, 20 and 45, 20 and 50, 20 and 75, 20 and 100, 25 and 30, 25 and 35, 25 and 40, 25 and 45, 25 and 50, 25 and 75, 25 and 100, 30 and 35, 30 and 40, 30 and 45, 30 and 50, 30 and 75, 30 and 100, 35 and 40, 35 and 45, 35 and 50, 35 and 75, 35 and 100, 40 and 45, 40 and 50, 40 and 75, 40 and 100, 50 and 75, 100 and 200, 100 and 300, 100 and 400, 200 and 300, 200 and 400, and 300 and 400, or 50 and 100)) of microclosures (e.g., any described herein). In some embodiments, each of the microclosures is separated by at least about 10 µm (e.g., about 15 µm, 20 µm, 25 µm, 30 µm, 40 µm, 45 µm, 50 µm, 75 µm, 100 µm, 150 µm, 200 µm, 300 µm, 400 µm, 500 µm, 750 µm, 1 mm, 1.25 mm, 1.75 mm, 2 mm, 3 mm, 4 mm, 5 mm, 7 mm, 10 mm or more) or between about 10 µm and about 5 mm (e.g., between about 0.01 mm and 5 mm, 0.01 mm and 3 mm, 0.01 mm and 2 mm, 0.01 mm and 1 mm, 0.01 mm and 0.5 mm, 0.01 mm and 0.3 mm, 0.01 mm and 0.1 mm, 0.05 mm and 5 mm, 0.05 mm and 3 mm, 0.05 mm and 2 mm, 0.05 mm and 1 mm, 0.05 mm and 0.5 mm, 0.05 mm and 0.3 mm, 0.05 mm and 0.1 mm, 0.1 mm and 5 mm, 0.1 mm and 3 mm, 0.1 mm and 2 mm, 0.1 mm and 1 mm, 0.1 mm and 0.5 mm, 0.1 mm and 0.3 mm, 0.5 mm and 5 mm, 0.5 mm and 3 mm, 0.5 mm and 2 mm, 0.5 mm and 1 mm, 1 mm and 5 mm, 1 mm and 3 mm, 3 mm and 5 mm).

The present invention also features a skin treatment device including an applicator, where the applicator is adapted to apply one or more microclosures (e.g., any described herein) or an array (e.g., any described herein, including one or more microclosures releasably attached to any solid substrate, such as a liner, a polymer, or a dressing, such as described herein).

In some embodiments, the applicator includes a needle (e.g., any described herein) and a pin (e.g., any described herein), where the needle or the pin is adapted to releasably attach the microclosure or the array. In further embodiments, the device or the apparatus includes a holder that is co-axial to the needle and is adapted to releasably attach the microclosure or the array. In some embodiments, the holder is adapted to apply a second compressive force to the microwound or a skin region.

In other embodiments, the applicator includes a dispenser adapted to dispense a volume of sealant that is less than or equal to about 3 mm³ (e.g., including any ranges described herein).

In some embodiments, the applicator is further adapted to make a plurality of microwounds in a skin region. In other embodiments, the applicator is further adapted to apply a second compressive force in a skin region. In yet other embodiments, the applicator is further adapted to remove the microclosure or the array.

In some embodiments, the microclosure or the array further includes one or more attachment components adapted to facilitate removal of the microclosure.

In some embodiments, the device further includes an apparatus for making a plurality of microwounds in a skin region (e.g., a microablation tool or any apparatus described herein). In further embodiments, the device further includes an apparatus for applying a second compressive force in a skin region. In yet other embodiments, the device further includes an apparatus for removing the microclosure or the array (e.g., a remover, such as any described herein). In some embodiments, the remover or the mechanical lifting device is configured to detach all the microclosure devices once the wound is healed or to detach some of the microclosure to titrate the tightening effect immediately after application of the microclosures.

The present invention also features a kit including one or more microclosures (e.g., any described herein) or an array (e.g., any described herein); and a device (e.g., any described herein), where the kit optionally includes a sanitizing wipe, an antibiotic ointment, a macrowound dressing, and/or instructions for use.

In some embodiments, the kit further includes a remover for removing the microclosure or the array, where the remover is selected from the group consisting of an apparatus, a chemical agent, a biological agent, a polymeric material, an abrasive material, a macrodressing (e.g., a tunable dressing), an adhesive material, and a mechanical lifting device. In other embodiments, one or more microclosures includes an attachment component adapted to attach to the mechanical lifting device. In yet other embodiments, the apparatus or the mechanical lifting device includes a heating component, an optical component, a radiofrequency component, a mechanical component, and/or an ultrasound component.

The present invention also features a method of treating skin including: forming a plurality of microwounds, each having at least one dimension that is less than about 1 mm (e.g., less than or equal to about 1 mm or between about 10 µm to about 1 mm, as described herein) and/or an areal dimension that is less than about 1 mm² (e.g., less than about 1 mm² or a range of about 0.2 mm² to about 4 mm², as described herein) in the region of the skin and/or a volumetric dimension that is less than about 4 mm³ (e.g., less than about 3 mm³ or between about 0.001 mm³ and 6 mm³, as described herein) in the region of the skin; and applying a plurality of microclosures to the plurality of microwounds. In some embodiments, the microclosure includes a material having at least one dimension that is less than about 2 mm (e.g., less than or equal to about 1.75 mm, about 1.5 mm, about 1.25 mm, about 1.0 mm, 0.75 mm, about 0.5 mm, about 0.3 mm, about 0.2 mm, about 0.1 mm, or about 0.05 mm) or between about 10 µm to about 2 mm (e.g., including ranges described herein) after application, where the microclosure maintains a first compressive force, thereby treating the skin.

In the above methods, prior to the application of a microclosure, a drug is administered into the microwound, e.g., as the microwounds are being formed.

Any of the microclosures described herein can include, e.g., a skin pigmentation modifying compound (e.g., a bleaching agent or lightening agent, e.g., hydroquinone, or a tyrosinase inhibitor).

In some embodiments, treating includes reducing tissue volume or area, promoting beneficial tissue growth, tightening skin, rejuvenating skin, improving skin texture or appearance, removing skin laxity, and/or expanding tissue volume or area.

In further embodiments, the method includes applying a second compressive force to the skin region (e.g., in one or more directions, such as in the x-, y-, z-, xy-, xz-, yz-, and/or xyz-direction) and/or removing the microclosure or the array after treating the skin.

In any of the devices, apparatuses, and methods described herein, the dressing (e.g., microdressing or macrodressing, which may be tunable) may include (i) an adhesive layer and (ii) a regulatable layer that includes one or more materials, where exposure of the regulatable layer to one or more external stimuli (e.g., any described herein) results in a change in a physical characteristic (e.g., any described herein) in the one or more materials in at least a portion of the dressing (e.g., including planar or non-planar changes across the entire device or in a portion of the device).

In any of the devices, apparatuses, and methods described herein, the dressing (e.g., microdressing or macrodressing, which may be tunable) includes (i) an adhesive layer and (ii) an unstretched layer that includes one or more materials, where exposure of the unstretched layer to one or more external stimuli (e.g., any described herein) results in contraction or expansion in one or more directions (e.g., in the x-, y-, z-, xy-, xz-, yz-, and/or xyz-directions) in at least a portion of the area of the dressing. In some embodiments, the contraction or expansion is in the x-axis, y-axis, and/or z-axis of the dressing, as compared to before the exposure (e.g., in the xy-, xz-, yz-, and/or xyz-plane of the dressing, as compared to before the exposure). In further embodiments, the contraction or expansion is uniform or non-uniform.

In some embodiments, the change in a physical characteristic includes an increase in tension of the device (e.g., of any device described herein, such as a microclosure, a macrodressing, or any substrate attached to a microclosure), a decrease in tension of the device (e.g., of any device described herein, such as a microclosure, a macrodressing, or any substrate attached to a microclosure), an increase in compressive force exerted by the device (e.g., of any device described herein, such as a microclosure, a macrodressing, or any substrate attached to a microclosure), a decrease in compressive force exerted by the device (e.g., of any device described herein, such as a microclosure, a macrodressing, or any substrate attached to a microclosure), compression in one or more directions of the device (e.g., of any device described herein, such as a microclosure, a macrodressing, or any substrate attached to a microclosure), and/or expansion in one or more directions of the device (e.g., of any device described herein, such as a microclosure, a macrodressing, or any substrate attached to a microclosure). In some embodiments, such an increase or decrease is in the x-axis, y-axis, and/or z-axis or in the xy-, xz-, xy-, and/or xyz-plane of the device, as compared to before the exposure. In particular embodiments, the increase or decrease in tension or compressive force and/or the expansion or compression of the device is an increase or decrease of intensity of at least about 0.5% after exposure of the one or more external stimuli, as compared to before the exposure (e.g., an increase or decrease of at least about 0.5% (e.g., at least about 0.6%, 0.7%, 0.8%, 0.9%, 1.0%, 1.1%, 1.2%, 1.5%, 1.7%, 2.0%, 2.2%, 2.5%, 2.7%, 3%, 3.5%, 4%, 4.5%, 5%, 5.5%, 6%, 6.5%, 7%, 7.5%, 8%, 8.5%, 9%, 9.5%, 10%, 10.5%, 15%, 20%, or more) or from about 0.5% to 20%, as described herein). In some embodiments, the physical characteristic is one or more of compression, expansion, tension, structure, size, porosity, surface chemistry, bending modulus, fracture or failure strain, resilience, permeability, swelling ratio, elasticity, electric conductivity, plasticity, resilience, resistance (e.g., creep resistance), strength (e.g., as measured by Young's modulus, tensile strength, compressive strength, impact strength, or yield strength), stress (e.g., compressive stress, shear stress, or tensile stress), load, and/or strain (e.g., as measured by deflection, deformation, strain at failure, or ultimate strain).

In any embodiment described herein, the change in a physical characteristic occurs in a portion of the device or across the entire device. In other embodiments, the change in a physical characteristic is non-uniform across the entire device or in a portion of the device. In yet other embodiments, the change in a physical characteristic is uniform across the entire device or in a portion of the device.

In any of the devices, apparatuses, and methods described herein, the one or more materials are configured in a random, non-geometric, and/or geometric arrangement to provide contraction and/or expansion in one or more directions in at least a portion of the area of the microclosure. In particular embodiments, the arrangement is geometric (e.g., a uniform or non-uniform arrangement). In some embodiments, the geometric arrangement includes a first material arranged in a first direction and optionally a second material arranged in a second direction (e.g., where the second direction is approximately orthogonal to the first direction). In further embodiments, each of the first material or the second material is, independently, a shape-memory polymer, a shape-memory alloy, a thermal-responsive material, a pH-responsive material, a light-responsive material, a moisture-responsive material, a solvent-responsive or chemical exposure-responsive material, an electric field-responsive material, a magnetic field-responsive material, an actuator-embedded material, a pre-stretched material, or an unstretched material (e.g., any described herein).

In any of the devices, apparatuses, and methods described herein, the one or more external stimuli is, independently, a change in temperature, pH, light, moisture, solvent, chemical exposure, electric field, and/or magnetic field (e.g., which can optionally result in mechanical, hydraulic, and/or pneumatic tuning).

In any embodiment described herein, exposure of the device (e.g., microclosures, dressing, or a layer of the device, as well as portions thereof) to two or more external stimuli (e.g., three, four, five, six, seven, eight, nine, ten, or more external stimuli) results in a change in two or more physical characteristics (e.g., three, four, five, six, seven, eight, nine, ten, or more changes in physical characteristics).

In any embodiment described herein, the microclosure, the regulatable layer, pre-stretched layer, or the unstretched layer includes two or more materials (e.g., three, four, five, six, seven, eight, nine, ten, or more materials). In particular embodiments, at least one of the materials (e.g., at least two, three, four, five, or more in one, two, three, four, or more layers) is a stimulus-responsive material (e.g., any described herein). Exemplary materials include a shape-memory polymer (e.g., including shape-memory polyurethane; block copolymers including poly(ethylene terephthalate), polystyrene, polyethylene glycol, poly(1,4-butadiene), polynorbornene, polyacrylate, and/or polyurethane, as well as shape-memory composites and shape-memory hybrids), a shape-memory alloy (e.g., any alloy described herein, such as a NiTi alloy), a thermal-responsive material (e.g., any such material described herein, such as polymers including poly-N-isopropylacrylamide, poly-N-vinylcaprolactam, poly-N,N-diethylacrylamide, and/or a polyalkylacrylamide), a pH-responsive material (e.g., any described herein, such as polymers and copolymers including one or more polyacrylic acid, polymethacrylic acid, methacrylic acid/methyl methacrylate, and carboxylic derivatives of any monomer described herein), a light-responsive material (e.g., a polymer including one or more light-responsive switches, as described herein), a moisture-responsive material (e.g., a polymer including one or more ionic monomers, as described herein), a solvent-responsive or chemical exposure-responsive material (e.g., a polymer composite, as described herein), an electric field-responsive material (e.g., a polymer including one or more electric field-responsive switches, as described herein), a magnetic field-responsive material (e.g., a polymer including one or more magnetic field-responsive switches, as described herein), an actuator-embedded material (e.g., a material including one or more MEMS actuators, carbon nanotubes, piezoceramic actuators (e.g., optionally having one or more interdigitated electrodes), multilayered actuators, optical fibers, piezopolymeric films, piezoplates, piezofibers, shape-memory polymers, or shape-memory alloys). In other embodiments, at least one of the materials (e.g., at least two, three, four, five, or more in one, two, three, four, or more layers) is a conventional material and/or a rigid material (e.g., any described herein, such as alginate, benzyl hyaluronate, carboxymethylcellulose, cellulose acetate, chitosan, collagen, dextran, epoxy, gelatin, hyaluronic acid, hydrocolloids, nylon (e.g., nylon 6 or PA6), pectin, poly (3-hydroxyl butyrate-co- poly (3-hydroxyl valerate), polyacrylate (PA), polyacrylonitrile (PAN), polybenzimidazole (PBI), polycarbonate (PC), polycaprolactone (PCL), polyester (PE), polyethylene glycol (PEG), polyethylene oxide (PEO), PEO/polycarbonate/polyurethane (PEO/PC/PU), poly(ethylene-co-vinyl acetate) (PEVA), PEVA/polylactic acid (PEVA/PLA), poly (ethylene terephthalate) (PET), PET/poly (ethylene naphthalate) (PET/PEN) polyglactin, polyglycolic acid (PGA), polyglycolic acid/polylactic acid (PGA/PLA), polyimide (PI), polylactic acid (PLA), poly-L-lactide (PLLA), PLLA/PC/polyvinylcarbazole (PLLA/PC/PVCB), poly (β-malic acid)-copolymers (PMLA), polymethacrylate (PMA), poly (methyl methacrylate) (PMMA), polystyrene (PS), polyurethane (PU), poly (vinyl alcohol) (PVA), polyvinylcarbazole (PVCB), polyvinyl chloride (PVC), polyvinylidenedifluoride (PVDF), polyvinylpyrrolidone (PVP), silicone, rayon, or combinations thereof).

In any embodiment described herein, the device (e.g., microclosure or dressing) is tunable without removal of a portion of the device (e.g., without removal of one or more layers of the dressing).

In any embodiment described herein, the adhesive layer includes a continuous layer of one or more adhesive materials or a discontinuous layer of one or more adhesive materials. In further embodiments, the discontinuous layer includes one or more adhesive materials in a random, geometric, or non-geometric arrangement (e.g., an array of one or more adhesive materials). In particular embodiments, the adhesive layer may be tunable (e.g., results in a change in a physical characteristic in the one or more adhesive materials in at least a portion of the microclosure or across the entire microclosure). Exemplary adhesive materials include any described herein, such as a biodegradable adhesive; a pressure sensitive adhesive (e.g., a natural rubber, synthetic rubber (e.g., a styrenebutadiene or styrene-ethylene copolymer), polyvinyl ether, polyurethane, acrylic, silicone, or a ethylenevinyl acetate copolymer); a biocompatible matrix (e.g., collagen (e.g., a collagen sponge), low melting agarose (LMA), polylactic acid (PLA), and/or hyaluronic acid (e.g., hyaluranon)); a photosensitizer (e.g., Rose Bengal, riboflavin-5-phosphate (R-5-P), methylene blue (MB), N-hydroxypyridine-2-(1H)-thione (N-HTP), a porphyrin, or a chlorin, as well as precursors thereof); a photochemical agent (e.g., 1,8 naphthalimide); a synthetic glue (e.g., a cyanoacrylate adhesive, a polyethylene glycol adhesive, or a gelatin-resorcinol-formaldehyde adhesive); or a biologic sealant (e.g., a mixture of riboflavin-5-phosphate and fibrinogen, a fibrin-based sealant, an albumin-based sealant, or a starch-based sealant).

In any embodiment described herein, the devices, apparatuses, and/or methods include one or more therapeutic agents selected from growth factors, analgesics (e.g., an NSAID, a COX-2 inhibitor, an opioid, a glucocorticoid agent, a steroid, or a mineralocorticoid agent, or any described herein), antibiotics, antifungals, antiinflammatory agents, antimicrobials (e.g., chlorhexidine-, iodine-, or silver-based agents, as described herein), antiseptics (e.g., an alcohol, a quaternary ammonium compound, or any described herein), antiproliferative agents, emollients, hemostatic agents, procoagulative agents, anticoagulative agents, immune modulators, proteins, or vitamins. In particular embodiments, the therapeutic agent is a hemostatic agent, a procoagulative agent, an anticoagulative agent, or combinations thereof. In some embodiments, the therapeutic agent is selected from the group of anhydrous aluminum sulfate, anti-fibrinolytic agent(s) (e.g., epsilon aminocaproic acid, tranexamic acid, or the like), anti-platelet agent(s) (e.g., aspirin, dipyridamole, ticlopidine, clopidogrel, or prasugrel), calcium alginate, cellulose, chitosan, coagulation factor(s) (e.g., II, V, VII, VIII, IX, X, XI, XIII, or Von Willebrand factor, as well as activated forms thereof), collagen (e.g., microfibrillar collagen), coumarin derivative(s) or vitamin K antagonist(s) (e.g., warfarin (coumadin), acenocoumarol, atromentin, phenindione, or phenprocoumon), desmopressin, epinephrine, factor Xa inhibitor(s) (e.g., apixaban or rivaroxaban), fibrinogen, heparin or derivatives thereof (e.g., low molecular weight heparin, fondaparinux, or idraparinux), poly-N-acetyl glucosamine, potassium alum, propyl gallate, silver nitrate, thrombin, thrombin inhibitor(s) (e.g., argatroban, bivalirudin, dabigatran, hirudin, lepirudin, or ximelagatran), titanium oxide, or a zeolite (e.g., a calcium-loaded zeolite).

In some embodiments, the kit includes an applicator, where the applicator is configured for positioning the microclosure on a skin region. In some embodiments, the applicator includes a frame or any structure configured to affix a microclosure to the skin region (e.g., a disposable frame or a disposable structure). In some embodiments, the applicator holds the microclosure to allow for aligning, positioning, and/or placing the microclosure on the desired skin region. In yet other embodiments, the applicator is configured to allow for affixing a microclosure (e.g., which may be a tunable microclosure) immediately after or shortly after forming one or more incisions or excisions (e.g., microwounds) in the skin region (e.g., within about 30 seconds, as described herein).

In some embodiments, the kit includes an apparatus for making incisions and/or excisions in a skin region (e.g., a microablation tool, such as a fractional laser microablation tool, a fractional radiofrequency microablation tool, or a fractional ultrasonic microablation tool). In some embodiments, the kit further includes an applicator (e.g., any described herein), where the applicator is structurally configured to attach to the apparatus for making one or more incisions and/or excisions and to release a device (e.g., a microclosure) after making such an incision or excision.

In further embodiments, any of the kits described herein can include one or more of instructions on how to use the device(s), an air blower, a heat gun, a heating pad, one or more therapeutic agents (e.g., any described herein, such as an anticoagulative and/or procoagulative agent, and optionally in combination with a useful dispenser for applying the therapeutic agent, such as a brush, spray, film, ointment, cream, lotion, or gel), one or more wound cleansers (e.g., including any antibiotic, antimicrobial, or antiseptic, such as those described herein, in any useful form, such as a brush, spray, film, ointment, cream, lotion, or gel), one or more debriding agents, one or more removers (e.g., any described herein, such as an apparatus, a chemical agent, a biological agent, a polymeric material, an abrasive material, a macrodressing, an adhesive material, or a mechanical lifting device), and/or other suitable or useful materials.

The present invention features methods of treating skin including: (i) affixing a device to a skin region, where the skin region includes a plurality of incised tissue portions and/or excised tissue portions (e.g,. a plurality of microwounds), where at least two of the tissue portions has an areal dimension that is less than about 1 mm² and/or where at least two of the tissue portions has a dimension that is less than about 1 mm, and where the device maintains a first compressive force and/or provides contraction or expansion of the skin region in one or more directions. In further embodiments, the methods include (ii) adjusting the first compressive force and/or contraction or expansion by exposing the affixed device to one or more external stimuli that result in a change in a physical characteristic of the affixed device.

In some embodiments, the areal dimension is less than or equal to about 1.0 mm² (e.g., less than or equal to about 0.9 mm², 0.8 mm², 0.7 mm², 0.6 mm², 0.5 mm², 0.4 mm², 0.3 mm², 0.2 mm², 0.1 mm², 0.07 mm², 0.05 mm², 0.03 mm², 0.02 mm², 0.01 mm², 0.007 mm², 0.005 mm², 0.003 mm², 0.002 mm², or 0.001 mm²) or between about 0.001 mm² and 1.0 mm² (e.g., as described herein).

In some embodiments, the skin region or treated skin region includes a plurality of incised tissue portions and/or excised tissue portions (e.g., a plurality of microwounds, holes and/or slits). In some embodiments, at least one (e.g., about 2, 3, 4, 5, 6, 7, 8, 9, 10, 15, 20, 25, 30, 35, 40, 45, 50, 75, 100, or more tissue portions, such as between about 2 and 100 tissue portions, as described herein) of the tissue portions (e.g., microwounds) has at least one dimension that is less than about 2.0 mm (e.g., less than or equal to about 1.5 mm, 1 mm, 0.75 mm, 0.5 mm, 0.3 mm, 0.2 mm, 0.1 mm, 0.075 mm, 0.05 mm, or 0.025 mm) or between about 0.025 mm and 2.0 mm (e.g., as described herein). In some embodiments, the plurality of incised tissue portions and/or excised tissue portions include one or more elliptical holes in the skin region. In other embodiments, the plurality of incised tissue portions and/or excised tissue portions includes any useful shape (e.g., a cylinder, hole, slit, elongated strip, or other geometries). In further embodiments, the areal fraction of the skin region to be removed is less than about 70% (e.g., less than about 65%, 60%, 55%, 50%, 45%, 40%, 35%, 30%, 25%, 20%, 10%, or 5%) or between about 5% and 80% (e.g., as described herein). In some embodiments, the plurality of tissue portions are incised or excised in any beneficial pattern within the skin region (e.g., as described herein).

In some embodiments, affixing step (i) is performed within about 30 seconds of incising and/or excising the skin region (e.g., within about 20, 15, 10, 5, 3 seconds or less after forming an incision or excision). In other embodiments, the adjusting step (ii) provides selectively closing or opening the incised tissue portions and/or excised tissue portions. In yet other embodiments, adjusting step (ii) includes adjusting the contraction or expansion across the entire device or a portion of the device. In further embodiments, the method results in controlling pleating in the skin region. In other embodiments, one or more microclosures may be held in a planar configuration at the same time that compression (e.g., a second compression, such as a lateral compression) is applied and therefore promote compression without pleating.

In any embodiment described herein, the devices, apparatuses, and methods are useful for closing discrete microscale incised tissue portions and/or excised tissue portions (e.g., microwounds).

In any embodiment described herein, the devices, apparatuses, and methods are useful for eliminating tissue volume or area, promoting beneficial tissue growth, tightening skin, rejuvenating skin, improving skin texture or appearance, removing skin laxity, and/or expanding tissue volume or area. In some embodiments, the devices, apparatuses, and methods are useful for treating one or more diseases, disorders, or conditions to improve skin appearance, to rejuvenate skin, and/or to tighten skin. Exemplary diseases, disorders, or conditions are described herein and include removal of pigment, veins (e.g., spider veins or reticular veins), and/or vessels in the skin, as well as treatment of acne, allodynia, blemishes, ectopic dermatitis, hyperpigmentation, hyperplasia (e.g., lentigo or keratosis), loss of translucency, loss of elasticity, melasma (e.g., epidermal, dermal, or mixed subtypes), photodamage, rashes (e.g., erythematous, macular, papular, and/or bullous conditions), psoriasis, rhytides (or wrinkles, e.g., crow's feet, age-related rhytides, sun-related rhytides, or heredity-related rhytides), sallow color, scar contracture (e.g., relaxation of scar tissue), scarring (e.g., due to acne, surgery, or other trauma), skin aging, skin contraction (e.g., excessive tension in the skin), skin irritation/sensitivity, skin laxity (e.g., loose or sagging skin or other skin irregularities), striae (or stretch marks), vascular lesions (e.g., angioma, erythema, hemangioma, papule, port wine stain, rosacea, reticular vein, or telangiectasia), or any other unwanted skin irregularities.

In some embodiments, the device includes a component for mechanical fractional ablation and a component for wound closure in a single device. In this manner, treatment can be achieved in one step by the user, thereby saving time and simplifying the treatment process.

In other embodiments, the devices, apparatuses, and methods described herein allow for treatment of uneven surfaces (e.g., the face). In particular, large wound dressings can be difficult to apply conformal to uneven skin surfaces. Thus, the present invention allows for conforming to the skin surface, even if the surface is uneven.

In other embodiments, the devices, apparatuses, and methods described herein allow for immediate assessment of the outcome of the treatment. Compared to energy-based methods, the outcome of the treatment can be immediately visible. For instance, treatment with conventional energy-based devices activate remodeling of the tissue and the end-result is only visible weeks to months after treatment.

In other embodiments, the devices, apparatuses, and methods described herein allow for rapid healing. For instance, compared to surgery, the treatment can be much less invasive and the healing can be, therefore, much faster.

### Definitions

By "about" is meant +/- 10% of any recited value.

By "areal dimension" is meant the two-dimensional area of an entity. The area of the opening of a microwound may be an areal dimension. For example, a circular microwound with a diameter of 0.5 mm would have an areal dimension of about 0.2 mm². If a compressive force is applied to skin surrounding the microwound, then the opening may be closed, thus reducing the microwound areal dimension to substantially zero, even though the underlying microwound below the surface of the skin still exists.

By "incised" tissue portion or "incision" is meant a cut, abrasion, or ablation of tissue, including a tissue portion in a skin region, or the act of cutting, abrading, destroying, or ablating tissue, a skin region, or one or more tissue portions. For example, an incision includes any cut, abrasion, or ablation into tissue, which can result in destruction of tissue or a portion thereof and, thereby, produce one or more holes or slits in the skin region. Exemplary methods of forming incised tissue portions or incisions include use of one or more blades, one or more solid needles, fractional laser ablation, fractional radiofrequency ablation, and/or fractional ultrasonic ablation, any useful tool for forming incisions, or any methods and apparatuses described herein.

By "excised" tissue portion or "excision" is meant a removed tissue, including a tissue portion from a skin region, or the act of removing tissue or one or more tissue portions from a skin region. For example, an excision includes any removed tissue or tissue portion from a skin region, which can result in excised tissue portions having a particular geometry (e.g., a cylindrical geometry) and produce one or more holes (i.e., negative space created by the removal of tissue) in the skin region. Exemplary methods of forming excised tissue portions or excisions include use of one or more hollow needles (optionally include one or more notches, extensions, protrusions, and/or barbs), one or more microaugers, one or more microabraders, any useful tool for forming excisions, or any methods and apparatuses described herein.

By "macrowound dressing" is meant a dressing for a wound with an areal dimension greater than about 4 mm².

By "microclosure" is meant a material, mechanism, or substance that can close, seal, cap, plug, pinch, fill, or otherwise reduce the size of a microwound. Exemplary microclosures include staples, microstaples, circular staples (e.g., circular multiprong staple, circular staple with sharp edge), ring staples, pre-strained staples, sutures, microsutures, wound dressings, tunable wound dressings, microwound dressings, welding, microwelding, glues (e.g., synthetic glues, such as cyanoacrylate, polyethylene glycol, gelatin-resorcinol-formaldehyde, or any described herein), sealants, and collagen crosslinkers (e.g., riboflavin, rose Bengal, or any described herein). In some embodiments, the microclosure has at least one dimension that is less than about 2 mm (e.g., less than or equal to about 1.75 mm, about 1.5 mm, about 1.25 mm, about 1.0 mm, 0.75 mm, about 0.5 mm, about 0.3 mm, about 0.2 mm, about 0.1 mm, or about 0.05 mm) or between about 10 µm to about 2 mm (e.g., including ranges described herein); and/or an areal dimension that is less than about 2 mm² (e.g., less than or equal to about 1.9 mm², 1.8 mm², 1.7 mm², 1.6 mm², 1.5 mm², 1.4 mm², 1.3 mm², 1.2 mm², 1.1 mm², 1 mm², 0.9 mm², 0.8 mm², 0.7 mm², 0.6 mm², 0.5 mm², 0.4 mm², 0.3 mm², 0.2 mm², 0.1 mm², 0.07 mm², 0.05 mm², 0.03 mm², 0.02 mm², 0.01 mm², 0.007 mm², 0.005 mm², 0.003 mm², 0.002 mm², or 0.001 mm²) or between about 0.001 mm² and 2 mm² (e.g., including ranges described herein); and/or a volumetric dimension that is less than about 6 mm³ (e.g., less than or equal to about 5.75 mm³, 5 mm³, 5.25 mm³, 4.75 mm³, 4.5 mm³, 4.25 mm³, 4 mm³, 3.75 mm³, 3.5 mm³, 3.25 mm³, 3 mm³, 2.75 mm³, 2.5 mm³, 2.25 mm³, 2 mm³, 1.75 mm³, 1.5 mm³, 1.25 mm³, 1 mm³, 0.9 mm³, 0.8 mm³, 0.7 mm³, 0.6 mm³, 0.5 mm³, 0.4 mm³, 0.3 mm³, 0.2 mm³, 0.1 mm³, 0.07 mm³, 0.05 mm³, 0.03 mm³, 0.02 mm³, 0.01 mm³, 0.007 mm³, 0.005 mm³, 0.003 mm³, 0.002 mm³, or 0.001 mm³) or between about 0.001 mm³ and 6 mm³ (e.g., including ranges described herein).

By "microwound" is meant an incised tissue or excised tissue portion, incision, abrasion, ablation of tissue, cut, tear, or imperfection in a skin region with an areal dimension less than about 4 mm² and/or a volumetric dimension that is less than about 6 mm³. The microwound may have an areal dimension in a range of about 0.2 mm² to about 4 mm² (e.g., about 0.2 mm² to 0.6 mm², 0.2 mm² to 1.0 mm², 0.2 mm² to 1.6 mm², 0.2 mm² to 2.1 mm², 0.2 mm² to 2.6 mm², 0.2 mm² to 3.0 mm², 0.2 mm² to 3.5 mm², and 0.2 mm² to 4.0 mm², 0.6 mm² to 1.0 mm², 0.6 mm² to 1.6 mm², 0.6 mm² to 2.1 mm², 0.6 mm² to 2.6 mm², 0.6 mm² to 3.0 mm², 0.6 mm² to 3.5 mm², 0.6 mm² to 4.0 mm², 1.0 mm² to 1.6 mm², 1.0 mm² to 2.1 mm², 1.0 mm² to 2.6 mm², 1.0 mm² to 3.0 mm², 1.0 mm² to 3.5 mm², 1.0 mm² to 4.0 mm², 1.6 mm² to 2.1 mm², 1.6 mm² to 2.6 mm², 1.6 mm² to 3.0 mm², 1.6 mm² to 3.5 mm², 1.6 mm² to 4.0 mm², 2.1 mm² to 2.6 mm², 2.1 mm² to 3.0 mm², 2.1 mm² to 3.5 mm², 2.1 mm² to 4.0 mm², 2.6 mm² to 3.0 mm², 2.6 mm² to 3.5 mm², 2.6 mm² to 4.0 mm², 3.0 mm² to 3.5 mm², 3.0 mm² to 4.0 mm², or 3.5 mm² to 4.0 mm²). In some embodiments, a microwound has at least one dimension in a range of about 50 µm to about 2 mm (e.g., about 50 µm to 100 µm, 50 µm to 250 µm, 50 µm to 500 µm, 50 µm to 750 µm, 50 µm to 1 mm, 50 µm to 1.5 mm, 50 µm to 2 mm, 100 µm to 250 µm, 100 µm to 500 µm, 100 µm to 750 µm, 100 µm to 1 mm, 100 µm to 1.5 mm, 100 µm to 2 mm, 250 µm to 500 µm, 250 µm to 750 µm, 250 µm to 1 mm, 250 µm to 1.5 mm, 250 µm to 2 mm, 500 µm to 750 µm, 500 µm to 1 mm, 500 µm to 1.5 mm, 500 µm to 2 mm, 750 µm to 1 mm, 750 µm to 1.5 mm, or 750 µm to 2 mm). In some embodiments, microwounds have an areal dimension less than about 0.2 mm². In some embodiments, a microwound may form a hole in the skin region, where the diameter or width of the hole is less than about 1.0 mm (e.g., less than about 1.0 mm, 750 µm, 500 µm, 250 µm, 100 µm, or 50 µm). The microwound may form a hole in the skin region, where the diameter or width is in a range of about 0.1 mm to about 2 mm (e.g., about 0.1 mm to 0.25 mm, 0.1 mm to 0.5 mm, 0.1 mm to 0.75 mm, 0.1 mm to 1 mm, 0.1 mm to 1.5 mm, 0.1 mm to 2 mm, 0.25 mm to 0.5 mm, 0.25 mm to 0.75 mm, 0.25 mm to 1 mm, 0.25 mm to 1.5 mm, 0.25 mm to 2 mm, 0.5 mm to 0.75 mm, 0.5 mm to 1 mm, 0.5 mm to 1.5 mm, 0.5 mm to 2 mm, 0.75 to 1 mm, 0.75 to 1.5 mm, or 0.75 to 2 mm, or any ranges described herein). In some embodiments, the volumetric dimension that is less than or equal to about 6 mm³ (e.g., as described herein) or between about 0.001 mm³ and 6 mm³ (e.g., as described herein). In particular embodiments, microwounds are discrete incised tissue or excised tissue portions.

By "physical characteristic" is meant a physical property of a device (e.g., a microclosure) or a material included in the device. Exemplary physical characteristics include compression (or compressive force), expansion, tension (e.g., as measured by tensile stress), structure, size, porosity, surface chemistry, bending modulus, fracture or failure strain, resilience, permeability, swelling ratio, elasticity (e.g., as measured by ultimate modulus of elasticity from the end-portion of stress-strain curves that is greater than 10 N/mm²), electric conductivity, plasticity, resilience, resistance (e.g., as measured by creep resistance), strength (e.g., as measured by Young's modulus (e.g., a Young's modulus that is greater than about 1 × 10⁵ N/m), tensile strength (e.g., a tensile strength that is greater than about 2 N/mm²), compressive strength, impact strength, or yield strength), stress (e.g., as measured by compressive stress, shear stress, or tensile stress), load, strain (e.g., as measured by deflection, deformation, strain at failure, or ultimate strain (extension before rupture), e.g., greater than about 30% or from about 30% to 130%), and other parameters, as well as any described herein.

By "pleating" or "skin pleating" is meant any distortion in skin tissue (e.g., in the epidermal and/or dermal layers) that results in puckering and/or folding.

By "tunable" is meant capable of being adjusted, modified, or altered in one or more physical characteristics in response to one or more external stimuli. Any part of the device can be tunable. For instance, in a microclosure, the bulk material can be tunable. In another instance, in a microdressing, the regulatable layer and/or adhesive layer is tunable. In one non-limiting example, a tunable dressing is a dressing including at least one layer, where the structure of the layer changes in response to an external stimulus, such as a change in temperature. In another non-limiting example, a tunable microclosure is a microclosure including at least one material, where the structure of the material changes in response to an external stimulus. The change in one physical characteristic (e.g., change in structure at the molecular, microscopic, or macroscopic level) can exert a change in another physical characteristic (e.g., a change in compressive force or tension exerted by the microclosure) in one or more directions (e.g., in the x-, y-, z-, xy-, xz-, yz-, and/or xyz-direction). In one non-limiting example, a polymeric material can be optimized to facilitate change in structure at the molecular level by altering the structure of the polymer chain (e.g., alterations to the side chain, linker regions, and/or precursor monomers), the particular block of the polymer (e.g., alterations to length, molecular weight, hydrophobicity, or hydrophilicity), or one or more co-polymeric blocks (e.g., alterations to weight percentage ratios or post-polymerization modifications). The extent of change can be either an increase or a decrease in a physical characteristic, as compared to before exposure of the stimulus. Such an increase or decrease can be of any useful extent, e.g., an increase or decrease of at least about 0.5% (e.g., at least about 0.6%, 0.7%, 0.8%, 0.9%, 1.0%, 1.1%, 1.2%, 1.5%, 1.7%, 2.0%, 2.2%, 2.5%, 2.7%, 3%, 3.5%, 4%, 4.5%, 5%, 5.5%, 6%, 6.5%, 7%, 7.5%, 8%, 8.5%, 9%, 9.5%, 10%, 10.5%, 15%, 20%, or more) or from about 0.5% to 20% (e.g., from about 0.5% to 15%, 0.5% to 10.5%, 0.5% to 10%, 0.5% to 9.5%, 0.5% to 9%, 0.5% to 8.5%, 0.5% to 8%, 0.5% to 7.5%, 0.5% to 7%, 0.5% to 6.5%, 0.5% to 6%, 0.5% to 5.5%, 0.5% to 5%, 0.5% to 4.5%, 0.5% to 4%, 0.5% to 3.5%, 0.5% to 3%, 0.5% to 2.7%, 0.5% to 2.5%, 0.5% to 2.2%, 0.5% to 2.0%, 0.5% to 1.7%, 0.5% to 1.5%, 0.5% to 1.2%, 0.5% to 1.1%, 0.5% to 1.0%, 0.5% to 0.9%, 0.5% to 0.8%, 0.5% to 0.7%, 0.5% to 0.6%, 0.7% to 20%, 0.7% to 15%, 0.7% to 10.5%, 0.7% to 10%, 0.7% to 9.5%, 0.7% to 9%, 0.7% to 8.5%, 0.7% to 8%, 0.7% to 7.5%, 0.7% to 7%, 0.7% to 6.5%, 0.7% to 6%, 0.7% to 5.5%, 0.7% to 5%, 0.7% to 4.5%, 0.7% to 4%, 0.7% to 3.5%, 0.7% to 3%, 0.7% to 2.7%, 0.7% to 2.5%, 0.7% to 2.2%, 0.7% to 2.0%, 0.7% to 1.7%, 0.7% to 1.5%, 0.7% to 1.2%, 0.7% to 1.1%, 0.7% to 1.0%, 0.7% to 0.9%, 0.7% to 0.8%, 1.0% to 20%, 1.0% to 15%, 1.0% to 10.5%, 1.0% to 10%, 1.0% to 9.5%, 1.0% to 9%, 1.0% to 8.5%, 1.0% to 8%, 1.0% to 7.5%, 1.0% to 7%, 1.0% to 6.5%, 1.0% to 6%, 1.0% to 5.5%, 1.0% to 5%, 1.0% to 4.5%, 1.0% to 4%, 1.0% to 3.5%, 1.0% to 3%, 1.0% to 2.7%, 1.0% to 2.5%, 1.0% to 2.2%, 1.0% to 2.0%, 1.0% to 1.7%, 1.0% to 1.5%, 1.0% to 1.2%, 1.0% to 1.1%, 1.5% to 20%, 1.5% to 15%, 1.5% to 10.5%, 1.5% to 10%, 1.5% to 9.5%, 1.5% to 9%, 1.5% to 8.5%, 1.5% to 8%, 1.5% to 7.5%, 1.5% to 7%, 1.5% to 6.5%, 1.5% to 6%, 1.5% to 5.5%, 1.5% to 5%, 1.5% to 4.5%, 1.5% to 4%, 1.5% to 3.5%, 1.5% to 3%, 1.5% to 2.7%, 1.5% to 2.5%, 1.5% to 2.2%, 1.5% to 2.0%, 1.5% to 1.7%, 2.0% to 20%, 2.0% to 15%, 2.0% to 10.5%, 2.0% to 10%, 2.0% to 9.5%, 2.0% to 9%, 2.0% to 8.5%, 2.0% to 8%, 2.0% to 7.5%, 2.0% to 7%, 2.0% to 6.5%, 2.0% to 6%, 2.0% to 5.5%, 2.0% to 5%, 2.0% to 4.5%, 2.0% to 4%, 2.0% to 3.5%, 2.0% to 3%, 2.0% to 2.7%, 2.0% to 2.5%, 2.0% to 2.2%, 2.5% to 20%, 2.5% to 15%, 2.5% to 10.5%, 2.5% to 10%, 2.5% to 9.5%, 2.5% to 9%, 2.5% to 8.5%, 2.5% to 8%, 2.5% to 7.5%, 2.5% to 7%, 2.5% to 6.5%, 2.5% to 6%, 2.5% to 5.5%, 2.5% to 5%, 2.5% to 4.5%, 2.5% to 4%, 2.5% to 3.5%, 2.5% to 3%, 2.5% to 2.7%, 3.0% to 20%, 3.0% to 15%, 3.0% to 10.5%, 3.0% to 10%, 3.0% to 9.5%, 3.0% to 9%, 3.0% to 8.5%, 3.0% to 8%, 3.0% to 7.5%, 3.0% to 7%, 3.0% to 6.5%, 3.0% to 6%, 3.0% to 5.5%, 3.0% to 5%, 3.0% to 4.5%, 3.0% to 4%, 3.0% to 3.5%, 4.0% to 20%, 4.0% to 15%, 3.5% to 10.5%, 4.0% to 10%, 4.0% to 9.5%, 4.0% to 9%, 4.0% to 8.5%, 4.0% to 8%, 4.0% to 7.5%, 4.0% to 7%, 4.0% to 6.5%, 4.0% to 6%, 4.0% to 5.5%, 4.0% to 5%, 4.0% to 4.5%, 5.0% to 20%, 5.0% to 15%, 5.0% to 10.5%, 5.0% to 10%, 5.0% to 9.5%, 5.0% to 9%, 5.0% to 8.5%, 5.0% to 8%, 5.0% to 7.5%, 5.0% to 7%, 5.0% to 6.5%, 5.0% to 6%, or 5.0% to 5.5%), as compared to before exposure of a stimulus. For a particular device (e.g., a microclosure), further tunability can be accomplished by any processing or post-processing known in the art (e.g., by using one or more hydrophilic or hydrophobic coatings, hydrogels, foams, colloids, etc.), thereby providing further control of one or more physical characteristics.

By "subject" is meant a human or non-human animal (e.g., a mammal).

By "treating" a disease, disorder, or condition in a subject is meant reducing at least one symptom of the disease, disorder, or condition by affixing a device (e.g., a microclosure) to the subject.

By "prophylactically treating" a disease, disorder, or condition in a subject is meant reducing the frequency of occurrence or severity of (e.g., preventing) a disease, disorder or condition by affixing a device (e.g., a microclosure) to the subject prior to the appearance of a symptom of the disease, disorder, or condition.

Other features and advantages of the invention will be apparent from the following Detailed Description and the claims.

### Brief Description of the Drawings

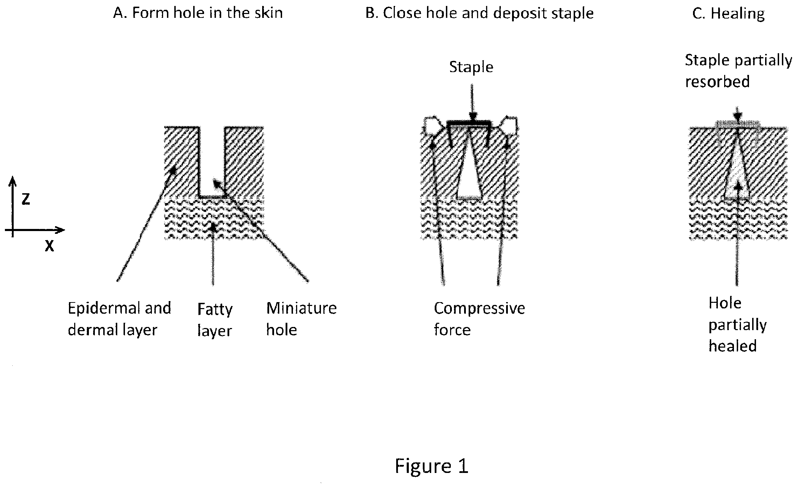

Figures 1A-1C describe an exemplary method of treating skin with a microstaple. This method includes (A) forming a microwound through the dermal and epidermal layer, (B) depositing a microstaple to close a microwound, where the microstaple exerts a compressive force, and (C) healing the skin, where the microstaple can be non-resorbable, resorbable, or partially resorbed during the healing process.

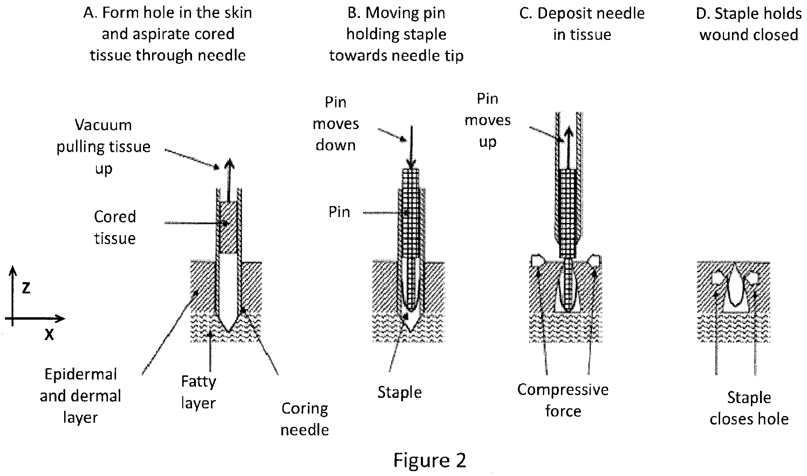

Figures 2A-2D describe an exemplary method of treating skin with a microstaple by closing the microwound internally. The method includes (A) forming a microwound through the epidermal and dermal layer with a coring needle. The cored tissue can detach at the interface with the subcutaneous fat layer. Optionally, a vacuum can be applied in the needle to detach the tissue and to aspirate the cored tissue plug through the needle. (B) While the needle maintains the microwound in an open state, a pin holding the microstaple can be introduced in the needle. The microstaple can be pushed into the formed microwound. (C) The needle can be removed, and a compressive force can be applied on the skin to close the microwound. To engage the microstaple in the tissue, the pin can be moved farther into the lumen of the coring needle, thereby detaching the microstaple from the pin. (D) The microstaple maintains the microwound closed for the duration of the healing process.

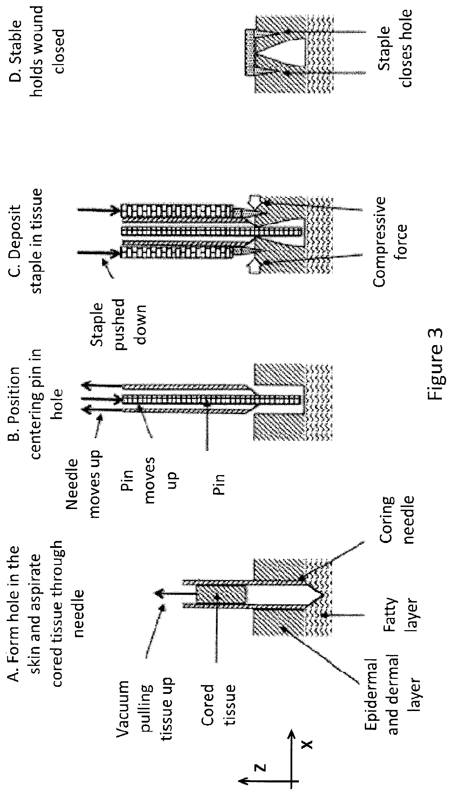

Figures 3A-3D describe an exemplary method of treating skin with a microstaple by closing the microwound externally. The method includes (A) forming a microwound through the epidermal and dermal layer with a coring needle. The cored tissue can detach at the interface with the subcutaneous fat layer. Optionally, a vacuum can be applied in the needle to help detach the tissue and to aspirate the cored tissue plug through the needle. (B) A centering pin can be introduced into the lumen of the needle to maintain the apparatus in alignment with the microwound. The needle is then moved upwards away from the microwound. (C) The microwound can be closed by a compressive force applied on the skin. A microstaple can then be deposited on the skin surface by, e.g., a mechanism co-axial with the needle and the pin. (D) The microstaple maintains the microwound closed for the duration of the healing process.

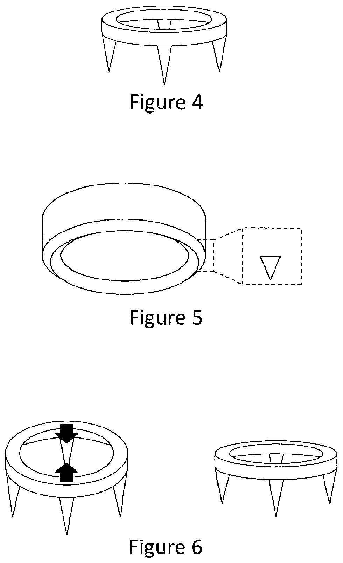

Figure 4 provides an exemplary schematic for a circular, multi-tip microstaple.
Figure 5 provides an exemplary schematic for a circular microstaple with a sharp edge.
Figure 6 provides an exemplary schematic for a circular, pre-constrained microstaple, where arrows represent the closing force of the microstaple.

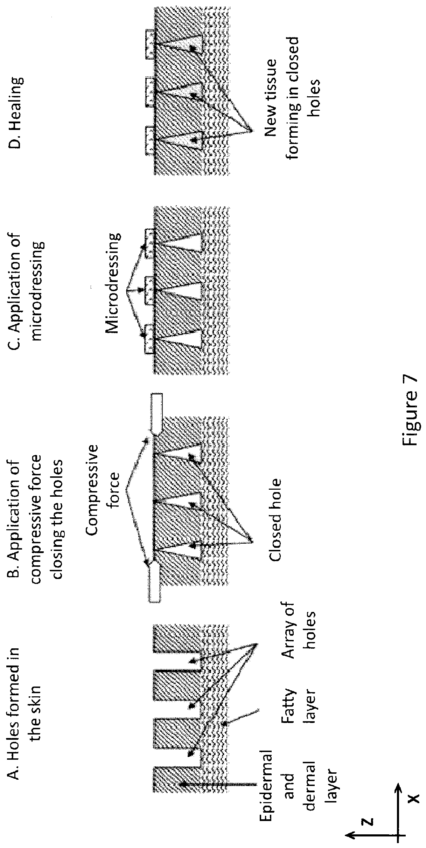

Figures 7A-7D describe an exemplary method of treating skin with a microdressing. The method includes (A) forming a plurality of microwounds through the epidermal and dermal layer (e.g., with a coring needle or any apparatus described herein). (B) The microwounds can be closed by a compressive force applied on the skin, e.g., by the same apparatus that created the microwounds. In one non-limiting example, the compressive force closes the microwounds in a preferred direction. (C) Microdressings can then be applied on the closed microwound to maintain the microwound closed during the healing process, as shown in (D).

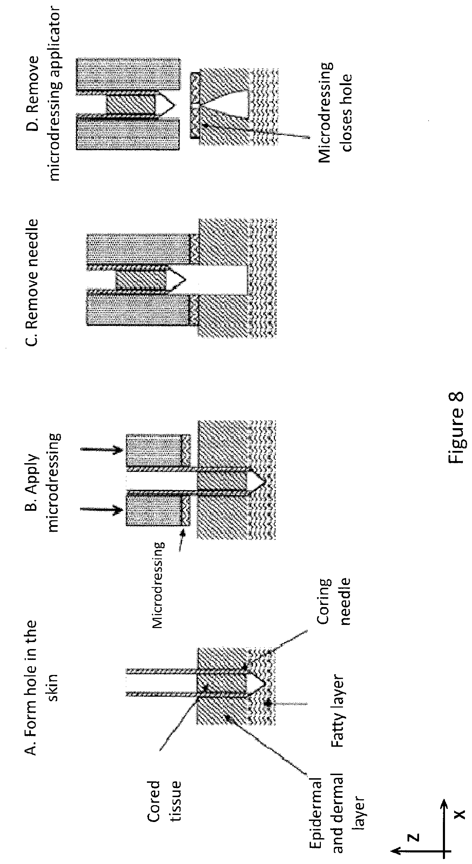

Figures 8A-8D describe an exemplary method of treating skin with a microdressing that is pre-constrained (or pre-stretched) prior to application to the skin. The method includes (A) forming a plurality of microwounds through the epidermal and dermal layer (e.g., with a coring needle or any apparatus described herein). (B) A cylindrical holder, co-axial with the needle, can be moved towards the skin. The microdressing can adhere to the end of the cylindrical holder, such as by an adhesive or an attachment component on the distal surface of the microdressing. Further, the proximal surface of the dressing facing the skin can also include an adhesive, such as any described herein. In particular, the adhesion force on the proximal surface of the dressing is stronger than the adhesion force on the distal surface of the dressing that is close to the cylindrical holder, thereby allowing the microdressing to detach from the holder. (C) The needle can be removed, thereby removing the cored tissue with the needle. The cylindrical holder is also removed, leaving the microdressing in place on the skin. (D) As the microdressing was applied in a pre-stretched state, the microdressing closes the microwound and maintains the microwound closed during the healing process.

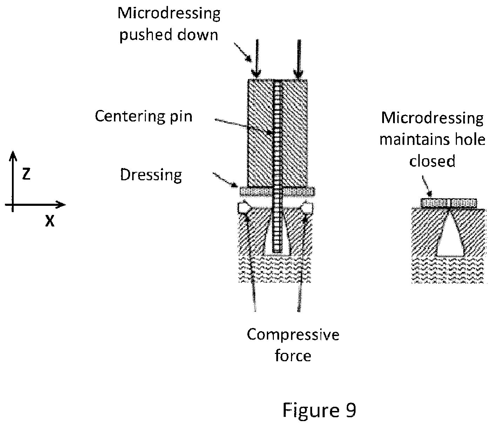

Figure 9 describes an exemplary method of treating skin with a microdressing that is applied with a centering pin. Left: The apparatus for forming microwounds can include a centering pin, which can be inserted into the microwound. Then, the wound can be closed by a compressive force applied on the skin, and a microdressing can be applied on the closed microwound by a cylindrical holder co-axial with the centering pin. Right: The applied microdressing maintains the microwound in a closed state, thereby facilitating the healing process.

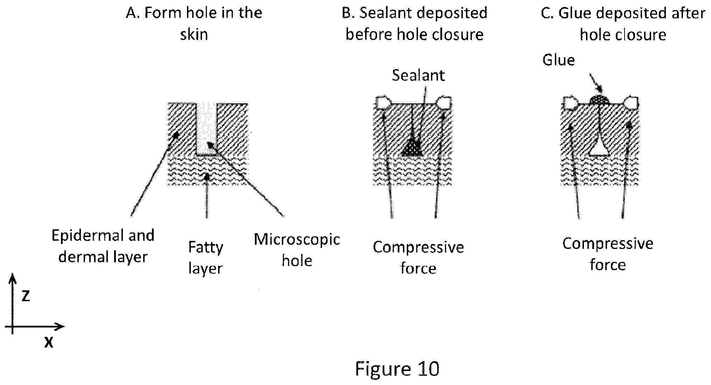

Figures 10A-10C describe an exemplary method of treating skin with a glue or a sealant. This method includes (A) forming a microwound through the dermal and epidermal layer and (B) depositing a sealant before microwound closure (e.g., where the sealant is resorbable, such as any resorbable sealant described herein). Then, a compressive force can be applied. In other embodiments, (C) the sealant can be deposited after microwound closure (e.g., where the sealant is non-resorbable, such as any non-resorbable or minimally resorbable sealant described herein). Then, a compressive force can be applied.

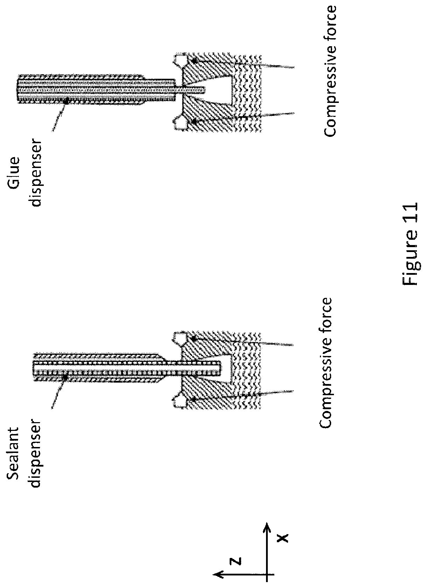

Figure 11 describes exemplary dispensers for a sealant or a glue that is deposited either before or after microwound closure. Left: Dispensing of the sealant in the microwound can include a tube inserted in the microwound through the coring needle prior to needle removal. A compressive force can be applied on the skin to close the microwound prior to depositing the sealant. Right: Dispensing of the glue on the skin surface can include a tube inserted through a coring needle. A centering pin can optionally be located in the tube. A compressive force can be applied on the skin to create a hermetic seal around the centering pin and to prevent glue leakage into the wound. Then, the glue can be dispensed, and the pin can be removed while compressive force is continuously applied on the skin to close the microwound.

### Detailed Description

This invention relates to methods and devices for treating skin (e.g., eliminating tissue volume, tightening skin, and/or reducing skin laxity) by selectively opening or closing a plurality of small wounds (e.g., microwounds) formed by incision or excision of tissue. For example, tissue excision can be performed by fractional ablation of the epidermal and/or dermal layer of the skin with a hollow coring needle, by fractional laser ablation, by fractional radiofrequency ablation, and/or by fractional ultrasonic ablation. Various methods and devices are proposed to close the small wounds, including microclosures which may be tunable or smart microclosures, that allow for titration of the tightening effect after application to the skin of a patient.

In particular embodiments, the present invention provides one or more of the following advantages. First, the methods and devices herein enable visualization of results in real time during the course of the treatment. One can envision asking the patient for feedback in real time during the treatment and adjusting the tightening to the patient preference. Second, the devices include micro-sized features, which can be beneficial for controlling the extent of skin treatment. Third, the methods and devices herein requires less skill than that of a surgeon. One can envision treatment of patients in an outpatient setting, rather than requiring an inpatient, surgical setting. Fourth, the methods and devices herein constitute minimally invasive techniques, which can provide more predictable results and/or risk factors than that for more invasive techniques (e.g., plastic surgery) or non-invasive energy-based techniques (e.g., laser, radiofrequency, or ultrasound). Fifth, the methods and devices herein can allow for less discriminate methods for treating the skin by forming holes or slits because the methods and devices allow for more discriminate control for closing such holes or slits. Sixth, the methods and devices herein can allow for rapid closing of holes or slits after treating the skin (e.g., within a few seconds after treating skin, such as within ten seconds), thereby minimizing the extent of bleeding and/or clotting within the holes or slits. Seventh, the methods and devices herein can be useful for maximizing the tightening effect while minimizing healing time by optimizing tightening (e.g., by controlling the extent of skin pleating, such as by increasing the extent of skin pleating for some applications or skin regions and by decreasing the extent of skin pleating for other applications or skin regions, as described herein). Finally, the methods and devices (e.g., microclosures) herein may be tunable, thereby allowing for titration of tightening after surgical hole or slit formation. For example, the tunable or smart microclosures described herein allow adjustment of the tightening intensity, direction, and spatial distribution after the microclosure has been applied or affixed to the patient's skin. In another example, titratable tightening can be achieved by selectively closing or opening a subset of slits or holes produced in an array.

One additional advantage of the present invention is the retention in the skin of drugs administered into microwounds, in particular, where the drugs are administered in various arrays of microwounds. Absent microenclosures, such administration of drugs suffers from poor dosing control, as a significant fraction of the drug may flow back out of the wound after administration. Use of the microinclosures of the presenet invention can serve to retain drugs after such administration and improve the consistency of dosing.

### Devices for closure of holes

The present invention features methods and devices to treat skin having one or more incised or excised tissue portions. In particular, exemplary devices include selectively opening or closing of microwounds (e.g., holes and/or slits) using a microclosure (e.g., any method or skin closure having at least one dimension of from about 10 µm to about 1 mm after application to a microwound). The invention also includes combinations of one or more different types of microclosures (e.g., one or more of a microstaple, a microdressing, a microweld, a suture, or a sealant), including combinations of these types in an array. Further details are provided below.

### Microstaples and microdressings

The present invention features a microclosure (e.g., a microstaple, a microdressing, or a microweld). Microstaples and microdressings can be formed from any useful material(s) (e.g., a metal, a metal alloy, a plastic, a polymer, such as any described herein, including stimulus-responsive materials). In particular embodiments, the microstaples and microdressings include one or more stimulus-responsive materials that allow for controlling the extent of the first compressive force exerted by the microclosure.

For microstaples, exemplary materials include one or more polymers, metals, alloys, plastics, stimulus-responsive materials, or any other materials described herein. Further, the microstaple can have any useful shape, such as a circle or non-circular (e.g., elliptical) shape (e.g., having one or more dimensions less than about or equal to about 1.0 mm, including any ranges described herein). Microstaples can be resorbable (e.g, bio-resorbable) or not.

The microstaple can have any useful feature that allows for application to the skin. Exemplary features include a tip, a sharpened edge on the perimeter of the staple, a prong, a bevel, a barb, a protrusion, or a point (e.g., including any described herein). The proximal surface of the microstaple can have one or more points (or prongs) (e.g., at least two, three, four, five, six, seven, eight, or more points). The geometry of such points or bevel of a sharp edge can be of any useful geometry (e.g., to allow for a first compression force in one or more directions (e.g., in the x-, y-, z-, xy-, xz-, yz-, and/or xyz-directions) and/or to allow for secure deposition into, on, or around the microwound).

The circumference or length of the microstaple can be such as to allow for depositing into, on, or around the microwound. When the microstaple is to be inserted into the microwound, then the microstaple has a maximum dimension that is less than the x- or y- dimension of the microwound (e.g., less than any x- or y-dimension described herein for a microwound or incised/excised tissue portion). Alternatively, the microstaple can have a maximum dimension that is less than the lumen of the needle used to form the microwound (e.g., any dimension described herein for an inner diameter of a needle). When the microstaple is to be inserted around or onto the microwound, then the microstaple has at least one dimension that is more than the x- or y-dimension of the microwound (e.g., more than any x- or y-dimension described herein for a microwound or incised/excised tissue portion).

For microdressings, exemplary materials include one or more polymers, metals, stimulus-responsive materials, or any other materials described herein. In some embodiments, the microdressing includes an adhesive layer and a regulatable layer. In other embodiments, the microdressing includes an adhesive layer and a pre-stretched or unstretched layer. In yet other embodiments, the microdressing is a tunable microdressing, as described herein.

In particular, exposure of the regulatable layer to one or more external stimuli results in a change in a physical characteristic in the material(s). This change can extend across the entire dressing (e.g., across the entire x-, y-, and/or z-direction of the dressing, including planar and non-planar changes) or in a portion or part of the dressing (e.g., at a localized area of the dressing, which has been locally exposed to a stimulus and thereby results in a change in one or more physical characteristics in the x-, y-, and/or z- direction). Further, the dressing can provide a variable tightening effect across the entire dressing (e.g., varying degrees of tightening across the entire x-, y-, and/or z-direction of the dressing, including planar and non-planar changes) or in a portion or part of the dressing (e.g., varying degrees of tightening at a localized area of the dressing). Additional details regarding dressings are described herein.

### Microwelding

The invention features methods of closing microwounds with microwelding, such as the use of a weld having micro-sized features (e.g., having at least one dimension of from about 10 µm to about 1 mm after application to a microwound).

Microwelding with a laser can be achieved without using a chemical welding agent. For instance, laser welding can be achieved by irradiating a wound and locally activating connective-tissue proteins by thermal effect. This results in a bond between the wound edges, and this effect can be achieved with or without addition of a bonding agent (such as Rose Bengal). Exemplary uses for laser welding include repair of corneal wounds, such as any device, method, agents, and use described in U.S. Pub. Nos. 2013-0045171, 2012-0136387, 2009-0312749, 2008-0009901, and 2007-0239260, as well as U.S. Pat. Nos. 6,562,037; 6,669,694; 6,733,498; and 5,749,895, each of which is incorporated herein by reference.

Such methods includes any described herein for welding tissue (e.g., either with or without a chemical agent, such as a chemical welding agent, a photochemical agent, or a photosensitizer). In one exemplary technique, a photosensitizer is applied to the tissue (e.g., Rose Bengal (RB)), as described herein. The area of application of the photosensitizer determines the size of the microweld. Accordingly, the photosensitizer is applied in an amount and to an area of the skin having at least one dimension of from about 10 µm to about 1 mm. In another exemplary technique, a laser can be used for tissue welding. In yet another exemplary technique, a photochemical agent is applied to the tissue, and then the tissue is irradiated with visible light to produce a seal. The area of application of the photochemical agent and the irradiation area determines the size of the microweld. Accordingly, the photochemical agent is applied in an amount and to an area of the skin having at least one dimension of from about 10 µm to about 1 mm, and the irradiation area has at least one dimension of from about 10 µm to about 1 mm.

### Microgluing

The invention also features methods of closing microwounds with microgluing, such as the use of microclosures including one or more sealants. In some embodiments, the microdressing includes a discrete aliquot of a sealant (e.g., each aliquot has at least one dimension of from about 10 µm to about 1 mm after application to a microwound). In particular embodiments, after deposition of the sealant in or on the microwound, the deposited sealant has an areal dimension that is less than about 1 mm² and/or a volumetric dimension that is less than about 3 mm³.

In some embodiments, the microdressing includes an array of a plurality of discrete aliquots of a sealant on a solid substrate. In use, an applicator can be used to align the array with a plurality of microwounds formed in the skin, thereby allowing for the array of aliquots to be deposited in or on a plurality of microwounds. In particular embodiments, the array includes one or more registration marks that allow for aligning the array with a plurality of microwounds. In some embodiments, the skin treatment device includes an apparatus for making a plurality of microwounds in a skin region, which is adapted to apply the microclosure or the array of microclosures including one or more sealants. In other embodiments, the skin treatment device includes a dispenser (e.g., as described herein) to dispense aliquots of sealant after forming a microwound and optionally applying a compressive force. In further embodiments, the sealant is resorbable. In yet other embodiments, the sealant is non-resorbable.

Exemplary sealants include a biocompatible matrix (e.g., those including at least one of collagen (e.g., a collagen sponge), low melting agarose (LMA), polylactic acid (PLA), and/or hyaluronic acid (e.g., hyaluranon)), a photosensitizer (e.g., Rose Bengal, riboflavin-5-phosphate (R-5-P), methylene blue (MB), N-hydroxypyridine-2-(1H)-thione (N-HTP), a porphyrin, or a chlorin, as well as precursors thereof), a photochemical agent (e.g., 1,8 naphthalimide), a synthetic glue (e.g., a cyanoacrylate adhesive, a polyethylene glycol adhesive, or a gelatin-resorcinol-formaldehyde adhesive), a biologic sealant (e.g., a mixture of riboflavin-5-phosphate and fibrinogen, a fibrin-based sealant, an albumin-based sealant, a collagen-based sealant, a keratin-based sealant, an alginate-based sealant, a chitin-based sealant, a proteoglycan-based sealant, a gelatin-based sealant, or a starch-based sealant), a biodegradable adhesive (e.g., poly(lactic acid), poly(glycolic acid), poly(lactic-co-glycolic acid), a polyester, a polyanhydride, a polyphosphazene, a polyacrylate, or a polymethacrylate), or a tissue glue composed of a mixture of riboflavin-5-phosphate and fibrinogen, as well as any adhesive described herein. Non-limiting examples of resorbable sealants include a biocompatible matrix, a biologic sealant, a biodegradable adhesive, such as any described herein (e.g., a fibrin-based sealant). Non-limiting examples of non-resorbable sealants include a synthetic glue or a tissue glue, such as any described herein (e.g., a cyanoacrylate adhesive).

### Arrays of microclosures

The present invention also features arrays including a plurality of microclosures. As described herein, the microclosure of the invention includes at least one feature having at least one dimension of from about 10 µm to about 1 mm after application to a microwound. When applying multiple devices having such micro-sized features, it may be beneficial to provide an array having the appropriate size and dimensions (e.g., appropriate separation distance between microclosures) to facilitate application of multiple microclosures onto or into a plurality of microwounds. Alternatively, as skin treatment generally requires the formation of a plurality of microwounds, providing microclosures in a multiple format (e.g., attached to a substrate in an array) could allow for simplified loading into an applicator for forming holes and/or depositing microclosures.

The arrays can include any useful random, geometric, or non-geometric arrangement of the microclosures. For instance, such patterns can be consistent with those described herein for a microwound or incised/excised tissue portions (e.g., random, staggered rows, parallel rows, a circular pattern, a tile pattern, fractal-like shapes, a spiral pattern, a square or rectangular pattern, a triangular pattern, a hexagonal pattern, a radial distribution, or a combination of one or more such patterns).

The number of microclosures for the array can be any useful number. For instance, the array can include a number of microclosures consistent with the number of microwounds to be formed in the skin region, such as about 2, 3, 4, 5, 6, 7, 8, 9, 10, 15, 20, 25, 30, 35, 40, 45, 50, 75, 100, or more microwounds or tissue portions, or between about 2 and 100 microwounds or tissue portions (e.g., including ranges described herein).

### Microclosures

The present invention features a microclosure having particular dimensions. In some embodiments, the microclosure has at least one dimension that is less than about 2 mm (e.g., less than or equal to about 1.75 mm, about 1.5 mm, about 1.25 mm, about 1.0 mm, about 0.75 mm, about 0.5 mm, about 0.3 mm, about 0.2 mm, about 0.1 mm, or about 0.05 mm) or between about 10 µm to about 2 mm (e.g., 0.01 mm to 1.75 mm, 0.01 mm to 1.5 mm, 0.01 mm to 1.25 mm, 0.01 mm to 1.0 mm, 0.01 mm to 0.75 mm, 0.01 mm to 0.5 mm, 0.01 mm to 0.3 mm, 0.01 mm to 0.2 mm, 0.01 mm to 0.1 mm, 0.01 mm to 0.05 mm, 0.01 mm to 0.025 mm, 0.02 mm to 2.0 mm, 0.02 mm to 1.75 mm, 0.02 mm to 1.5 mm, 0.02 mm to 1.25 mm, 0.02 mm to 1.0 mm, 0.02 mm to 0.75 mm, 0.02 mm to 0.5 mm, 0.02 mm to 0.3 mm, 0.02 mm to 0.2 mm, 0.02 mm to 0.1 mm, 0.02 mm to 0.05 mm, 0.02 mm to 0.025 mm, 0.03 mm to 2.0 mm, 0.03 mm to 1.75 mm, 0.03 mm to 1.5 mm, 0.03 mm to 1.25 mm, 0.03 mm to 1.0 mm, 0.03 mm to 0.75 mm, 0.03 mm to 0.5 mm, 0.03 mm to 0.3 mm, 0.03 mm to 0.2 mm, 0.03 mm to 0.1 mm, 0.03 mm to 0.05 mm, 0.04 mm to 2.0 mm, 0.04 mm to 1.75 mm, 0.04 mm to 1.5 mm, 0.04 mm to 1.25 mm, 0.04 mm to 1.0 mm, 0.04 mm to 0.75 mm, 0.04 mm to 0.5 mm, 0.04 mm to 0.3 mm, 0.04 mm to 0.2 mm, 0.04 mm to 0.1 mm, 0.04 mm to 0.05 mm, 0.05 mm to 2.0 mm, 0.05 mm to 1.75 mm, 0.05 mm to 1.5 mm, 0.05 mm to 1.25 mm, 0.05 mm to 1.0 mm, 0.05 mm to 0.75 mm, 0.05 mm to 0.5 mm, 0.05 mm to 0.3 mm, 0.05 mm to 0.2 mm, 0.05 mm to 0.1 mm, 0.075 mm to 2.0 mm, 0.075 mm to 1.75 mm, 0.075 mm to 1.5 mm, 0.075 mm to 1.25 mm, 0.075 mm to 1.0 mm, 0.075 mm to 0.75 mm, 0.075 mm to 0.5 mm, 0.075 mm to 0.3 mm, 0.075 mm to 0.2 mm, 0.075 mm to 0.1 mm, 0.1 mm to 2.0 mm, 0.1 mm to 1.75 mm, 0.1 mm to 1.5 mm, 0.1 mm to 1.25 mm, 0.1 mm to 1.0 mm, 0.1 mm to 0.75 mm, 0.1 mm to 0.5 mm, 0.1 mm to 0.3 mm, 0.1 mm to 0.2 mm, 0.3 mm to 2.0 mm, 0.3 mm to 1.75 mm, 0.3 mm to 1.5 mm, 0.3 mm to 1.25 mm, 0.3 mm to 1.0 mm, 0.3 mm to 0.75 mm, 0.3 mm to 0.5 mm, 0.5 mm to 2.0 mm, 0.5 mm to 1.75 mm, 0.5 mm to 1.5 mm, 0.5 mm to 1.25 mm, 0.5 mm to 1.0 mm, 0.5 mm to 0.75 mm, 0.75 mm to 2.0 mm, 0.75 mm to 1.75 mm, 0.75 mm to 1.5 mm, 0.75 mm to 1.25 mm, 0.75 mm to 1.0 mm, 1.0 mm to 2.0 mm, 1.0 mm to 1.75 mm, 1.0 mm to 1.5 mm, 1.0 mm to 1.25 mm, 1.5 mm to 2.0 mm, or 1.5 mm to 1.75 mm).

In other embodiments, the microclosure has an areal dimension that is less than about 2 mm² (e.g., less than or equal to about 1.9 mm², 1.8 mm², 1.7 mm², 1.6 mm², 1.5 mm², 1.4 mm², 1.3 mm², 1.2 mm², 1.1 mm², 1 mm², 0.9 mm², 0.8 mm², 0.7 mm², 0.6 mm², 0.5 mm², 0.4 mm², 0.3 mm², 0.2 mm², 0.1 mm², 0.07 mm², 0.05 mm², 0.03 mm², 0.02 mm², 0.01 mm², 0.007 mm², 0.005 mm², 0.003 mm², 0.002 mm², or 0.001 mm²) or between about 0.001 mm² and 2 mm² (e.g., between about 0.001 mm² and 1.9 mm², 0.001 mm² and 1.8 mm², 0.001 mm² and 1.7 mm², 0.001 mm² and 1.6 mm², 0.001 mm² and 1.5 mm², 0.001 mm² and 1.4 mm², 0.001 mm² and 1.3 mm², 0.001 mm² and 1.2 mm², 0.001 mm² and 1.1 mm², 0.001 mm² and 1.0 mm², 0.001 mm² and 0.9 mm², 0.001 mm² and 0.8 mm², 0.001 mm² and 0.7 mm², 0.001 mm² and 0.6 mm², 0.001 mm² and 0.5 mm², 0.001 mm² and 0.4 mm², 0.001 mm² and 0.3 mm², 0.001 mm² and 0.2 mm², 0.001 mm² and 0.1 mm², 0.001 mm² and 0.07 mm², 0.001 mm² and 0.05 mm², 0.001 mm² and 0.03 mm², 0.001 mm² and 0.02 mm², 0.001 mm² and 0.01 mm², 0.001 mm² and 0.007 mm², 0.001 mm² and 0.005 mm², 0.001 mm² and 0.003 mm², 0.001 mm² and 0.002 mm², 0.002 mm² and 2.0 mm², 0.002 mm² and 1.9 mm², 0.002 mm² and 1.8 mm², 0.002 mm² and 1.7 mm², 0.002 mm² and 1.6 mm², 0.002 mm² and 1.5 mm², 0.002 mm² and 1.4 mm², 0.002 mm² and 1.3 mm², 0.002 mm² and 1.2 mm², 0.002 mm² and 1.1 mm², 0.002 mm² and 1.0 mm², 0.002 mm² and 0.9 mm², 0.002 mm² and 0.8 mm², 0.002 mm² and 0.7 mm², 0.002 mm² and 0.6 mm², 0.002 mm² and 0.5 mm², 0.002 mm² and 0.4 mm², 0.002 mm² and 0.3 mm², 0.002 mm² and 0.2 mm², 0.002 mm² and 0.1 mm², 0.002 mm² and 0.07 mm², 0.002 mm² and 0.05 mm², 0.002 mm² and 0.03 mm², 0.002 mm² and 0.02 mm², 0.002 mm² and 0.01 mm², 0.002 mm² and 0.007 mm², 0.002 mm² and 0.005 mm², 0.002 mm² and 0.003 mm², 0.005 mm² and 2.0 mm², 0.005 mm² and 1.9 mm², 0.005 mm² and 1.8 mm², 0.005 mm² and 1.7 mm², 0.005 mm² and 1.6 mm², 0.005 mm² and 1.5 mm², 0.005 mm² and 1.4 mm², 0.005 mm² and 1.3 mm², 0.005 mm² and 1.2 mm², 0.005 mm² and 1.1 mm², 0.005 mm² and 1.0 mm², 0.005 mm² and 0.9 mm², 0.005 mm² and 0.8 mm², 0.005 mm² and 0.7 mm², 0.005 mm² and 0.6 mm², 0.005 mm² and 0.5 mm², 0.005 mm² and 0.4 mm², 0.005 mm² and 0.3 mm², 0.005 mm² and 0.2 mm², 0.005 mm² and 0.1 mm², 0.005 mm² and 0.07 mm², 0.005 mm² and 0.05 mm², 0.005 mm² and 0.03 mm², 0.005 mm² and 0.02 mm², 0.005 mm² and 0.01 mm², 0.005 mm² and 0.007 mm², 0.007 mm² and 2.0 mm², 0.007 mm² and 1.9 mm², 0.007 mm² and 1.8 mm², 0.007 mm² and 1.7 mm², 0.007 mm² and 1.6 mm², 0.007 mm² and 1.5 mm², 0.007 mm² and 1.4 mm², 0.007 mm² and 1.3 mm², 0.007 mm² and 1.2 mm², 0.007 mm² and 1.1 mm², 0.007 mm² and 1.0 mm², 0.007 mm² and 0.9 mm², 0.007 mm² and 0.8 mm², 0.007 mm² and 0.7 mm², 0.007 mm² and 0.6 mm², 0.007 mm² and 0.5 mm², 0.007 mm² and 0.4 mm², 0.007 mm² and 0.3 mm², 0.007 mm² and 0.2 mm², 0.007 mm² and 0.1 mm², 0.007 mm² and 0.07 mm², 0.007 mm² and 0.05 mm², 0.007 mm² and 0.03 mm², 0.007 mm² and 0.02 mm², 0.007 mm² and 0.01 mm², 0.01 mm² and 2.0 mm², 0.01 mm² and 1.9 mm², 0.01 mm² and 1.8 mm², 0.01 mm² and 1.7 mm², 0.01 mm² and 1.6 mm², 0.01 mm² and 1.5 mm², 0.01 mm² and 1.4 mm², 0.01 mm² and 1.3 mm², 0.01 mm² and 1.2 mm², 0.01 mm² and 1.1 mm², 0.01 mm² and 1.0 mm², 0.01 mm² and 0.9 mm², 0.01 mm² and 0.8 mm², 0.01 mm² and 0.7 mm², 0.01 mm² and 0.6 mm², 0.01 mm² and 0.5 mm², 0.01 mm² and 0.4 mm², 0.01 mm² and 0.3 mm², 0.01 mm² and 0.2 mm², 0.01 mm² and 0.1 mm², 0.01 mm² and 0.07 mm², 0.01 mm² and 0.05 mm², 0.01 mm² and 0.03 mm², 0.01 mm² and 0.02 mm², 0.03 mm² and 2.0 mm², 0.03 mm² and 1.9 mm², 0.03 mm² and 1.8 mm², 0.03 mm² and 1.7 mm², 0.03 mm² and 1.6 mm², 0.03 mm² and 1.5 mm², 0.03 mm² and 1.4 mm², 0.03 mm² and 1.3 mm², 0.03 mm² and 1.2 mm², 0.03 mm² and 1.1 mm², 0.03 mm² and 1.0 mm², 0.03 mm² and 0.9 mm², 0.03 mm² and 0.8 mm², 0.03 mm² and 0.7 mm², 0.03 mm² and 0.6 mm², 0.03 mm² and 0.5 mm², 0.03 mm² and 0.4 mm², 0.03 mm² and 0.3 mm², 0.03 mm² and 0.2 mm², 0.03 mm² and 0.1 mm², 0.03 mm² and 0.07 mm², 0.03 mm² and 0.05 mm², 0.07 mm² and 2.0 mm², 0.07 mm² and 1.9 mm², 0.07 mm² and 1.8 mm², 0.07 mm² and 1.7 mm², 0.07 mm² and 1.6 mm², 0.07 mm² and 1.5 mm², 0.07 mm² and 1.4 mm², 0.07 mm² and 1.3 mm², 0.07 mm² and 1.2 mm², 0.07 mm² and 1.1 mm², 0.07 mm² and 1.0 mm², 0.07 mm² and 0.9 mm², 0.07 mm² and 0.8 mm², 0.07 mm² and 0.7 mm², 0.07 mm² and 0.6 mm², 0.07 mm² and 0.5 mm², 0.07 mm² and 0.4 mm², 0.07 mm² and 0.3 mm², 0.07 mm² and 0.2 mm², 0.07 mm² and 0.1 mm², 0.1 mm² and 2.0 mm², 0.1 mm² and 1.9 mm², 0.1 mm² and 1.8 mm², 0.1 mm² and 1.7 mm², 0.1 mm² and 1.6 mm², 0.1 mm² and 1.5 mm², 0.1 mm² and 1.4 mm², 0.1 mm² and 1.3 mm², 0.1 mm² and 1.2 mm², 0.1 mm² and 1.1 mm², 0.1 mm² and 1.0 mm², 0.1 mm² and 0.9 mm², 0.1 mm² and 0.8 mm², 0.1 mm² and 0.7 mm², 0.1 mm² and 0.6 mm², 0.1 mm² and 0.5 mm², 0.1 mm² and 0.4 mm², 0.1 mm² and 0.3 mm², 0.1 mm² and 0.2 mm², 0.3 mm² and 2.0 mm², 0.3 mm² and 1.9 mm², 0.3 mm² and 1.8 mm², 0.3 mm² and 1.7 mm², 0.3 mm² and 1.6 mm², 0.3 mm² and 1.5 mm², 0.3 mm² and 1.4 mm², 0.3 mm² and 1.3 mm², 0.3 mm² and 1.2 mm², 0.3 mm² and 1.1 mm², 0.3 mm² and 1.0 mm², 0.3 mm² and 0.9 mm², 0.3 mm² and 0.8 mm², 0.3 mm² and 0.7 mm², 0.3 mm² and 0.6 mm², 0.3 mm² and 0.5 mm², 0.3 mm² and 0.4 mm², 0.5 mm² and 2.0 mm², 0.5 mm² and 1.9 mm², 0.5 mm² and 1.8 mm², 0.5 mm² and 1.7 mm², 0.5 mm² and 1.6 mm², 0.5 mm² and 1.5 mm², 0.5 mm² and 1.4 mm², 0.5 mm² and 1.3 mm², 0.5 mm² and 1.2 mm², 0.5 mm² and 1.1 mm², 0.5 mm² and 1.0 mm², 0.5 mm² and 0.9 mm², 0.5 mm² and 0.8 mm², 0.5 mm² and 0.7 mm², 0.5 mm² and 0.6 mm², 0.7 mm² and 2.0 mm², 0.7 mm² and 1.9 mm², 0.7 mm² and 1.8 mm², 0.7 mm² and 1.7 mm², 0.7 mm² and 1.6 mm², 0.7 mm² and 1.5 mm², 0.7 mm² and 1.4 mm², 0.7 mm² and 1.3 mm², 0.7 mm² and 1.2 mm², 0.7 mm² and 1.1 mm², 0.7 mm² and 1.0 mm², 0.7 mm² and 0.9 mm², 0.7 mm² and 0.8 mm², 1.0 mm² and 2.0 mm², 1.0 mm² and 1.9 mm², 1.0 mm² and 1.8 mm², 1.0 mm² and 1.7 mm², 1.0 mm² and 1.6 mm², 1.0 mm² and 1.5 mm², 1.0 mm² and 1.4 mm², 1.0 mm² and 1.3 mm², 1.0 mm² and 1.2 mm², 1.0 mm² and 1.1 mm², 1.3 mm² and 2.0 mm², 1.3 mm² and 1.9 mm², 1.3 mm² and 1.8 mm², 1.3 mm² and 1.7 mm², 1.3 mm² and 1.6 mm², 1.3 mm² and 1.5 mm², 1.3 mm² and 1.4 mm², 1.7 mm² and 2.0 mm², 1.7 mm² and 1.9 mm², or 1.7 mm² and 1.8 mm²).

In yet other embodiments, the microclosure has a volumetric dimension that is less than about 6 mm³ (e.g., less than or equal to about 5.75 mm³, 5 mm³, 5.25 mm³, 4.75 mm³, 4.5 mm³ ,4.25 mm³, 4 mm³, 3.75 mm³, 3.5 mm³, 3.25 mm³, 3 mm³, 2.75 mm³, 2.5 mm³, 2.25 mm³, 2 mm³, 1.75 mm³, 1.5 mm³, 1.25 mm³, 1 mm³, 0.9 mm³, 0.8 mm³, 0.7 mm³, 0.6 mm³, 0.5 mm³, 0.4 mm³, 0.3 mm³, 0.2 mm³, 0.1 mm³, 0.07 mm³, 0.05 mm³, 0.03 mm³, 0.02 mm³, 0.01 mm³, 0.007 mm³, 0.005 mm³, 0.003 mm³, 0.002 mm³, or 0.001 mm³) or between about 0.001 mm³ and 6 mm³ (e.g., between about 0.001 mm³ and 5.75 mm³, 0.001 mm³ and 5 mm³, 0.001 mm³ and 5.25 mm³, 0.001 mm³ and 4.75 mm³, 0.001 mm³ and 4.5 mm³, 0.001 mm³ and 4.25 mm³, 0.001 mm³ and 4 mm³, 0.001 mm³ and 3.75 mm³, 0.001 mm³ and 3.5 mm³, 0.001 mm³ and 3.25 mm³, 0.001 mm³ and 3 mm³, 0.001 mm³ and 2.75 mm³, 0.001 mm³ and 2.5 mm³, 0.001 mm³ and 2.25 mm³, 0.001 mm³ and 2 mm³, 0.001 mm³ and 1.75 mm³, 0.001 mm³ and 1.5 mm³, 0.001 mm³ and 1.25 mm³, 0.001 mm³ and 1 mm³, 0.001 mm³ and 0.9 mm³, 0.001 mm³ and 0.8 mm³, 0.001 mm³ and 0.7 mm³, 0.001 mm³ and 0.6 mm³, 0.001 mm³ and 0.5 mm³, 0.001 mm³ and 0.4 mm³, 0.001 mm³ and 0.3 mm³, 0.001 mm³ and 0.2 mm³, 0.001 mm³ and 0.1 mm³, 0.001 mm³ and 0.07 mm³, 0.001 mm³ and 0.05 mm³, 0.001 mm³ and 0.03 mm³, 0.001 mm³ and 0.02 mm³, 0.001 mm³ and 0.01 mm³, 0.001 mm³ and 0.007 mm³, 0.001 mm³ and 0.005 mm³, 0.001 mm³ and 0.003 mm³, 0.001 mm³ and 0.002 mm³, 0.003 mm³ and 6 mm³, 0.003 mm³ and 5.75 mm³, 0.003 mm³ and 5 mm³, 0.003 mm³ and 5.25 mm³, 0.003 mm³ and 4.75 mm³, 0.003 mm³ and 4.5 mm³, 0.003 mm³ and 4.25 mm³, 0.003 mm³ and 4 mm³, 0.003 mm³ and 3.75 mm³, 0.003 mm³ and 3.5 mm³, 0.003 mm³ and 3.25 mm³, 0.003 mm³ and 3 mm³, 0.003 mm³ and 2.75 mm³, 0.003 mm³ and 2.5 mm³, 0.003 mm³ and 2.25 mm³, 0.003 mm³ and 2 mm³, 0.003 mm³ and 1.75 mm³, 0.003 mm³ and 1.5 mm³, 0.003 mm³ and 1.25 mm³, 0.003 mm³ and 1 mm³, 0.003 mm³ and 0.9 mm³, 0.003 mm³ and 0.8 mm³, 0.003 mm³ and 0.7 mm³, 0.003 mm³ and 0.6 mm³, 0.003 mm³ and 0.5 mm³, 0.003 mm³ and 0.4 mm³, 0.003 mm³ and 0.3 mm³, 0.003 mm³ and 0.2 mm³, 0.003 mm³ and 0.1 mm³, 0.003 mm³ and 0.07 mm³, 0.003 mm³ and 0.05 mm³, 0.003 mm³ and 0.03 mm³, 0.003 mm³ and 0.02 mm³, 0.003 mm³ and 0.01 mm³, 0.003 mm³ and 0.007 mm³, 0.003 mm³ and 0.005 mm³, 0.005 mm³ and 6 mm³, 0.005 mm³ and 5.75 mm³, 0.005 mm³ and 5 mm³, 0.005 mm³ and 5.25 mm³, 0.005 mm³ and 4.75 mm³, 0.005 mm³ and 4.5 mm³, 0.005 mm³ and 4.25 mm³, 0.005 mm³ and 4 mm³, 0.005 mm³ and 3.75 mm³, 0.005 mm³ and 3.5 mm³, 0.005 mm³ and 3.25 mm³, 0.005 mm³ and 3 mm³, 0.005 mm³ and 2.75 mm³, 0.005 mm³ and 2.5 mm³, 0.005 mm³ and 2.25 mm³, 0.005 mm³ and 2 mm³, 0.005 mm³ and 1.75 mm³, 0.005 mm³ and 1.5 mm³, 0.005 mm³ and 1.25 mm³, 0.005 mm³ and 1 mm³, 0.005 mm³ and 0.9 mm³, 0.005 mm³ and 0.8 mm³, 0.005 mm³ and 0.7 mm³, 0.005 mm³ and 0.6 mm³, 0.005 mm³ and 0.5 mm³, 0.005 mm³ and 0.4 mm³, 0.005 mm³ and 0.3 mm³, 0.005 mm³ and 0.2 mm³, 0.005 mm³ and 0.1 mm³, 0.005 mm³ and 0.07 mm³, 0.005 mm³ and 0.05 mm³, 0.005 mm³ and 0.03 mm³, 0.005 mm³ and 0.02 mm³, 0.005 mm³ and 0.01 mm³, 0.005 mm³ and 0.007 mm³, 0.01 mm³ and 6 mm³, 0.01 mm³ and 5.75 mm³, 0.01 mm³ and 5 mm³, 0.01 mm³ and 5.25 mm³, 0.01 mm³ and 4.75 mm³, 0.01 mm³ and 4.5 mm³, 0.01 mm³ and 4.25 mm³, 0.01 mm³ and 4 mm³, 0.01 mm³ and 3.75 mm³, 0.01 mm³ and 3.5 mm³, 0.01 mm³ and 3.25 mm³, 0.01 mm³ and 3 mm³, 0.01 mm³ and 2.75 mm³, 0.01 mm³ and 2.5 mm³, 0.01 mm³ and 2.25 mm³, 0.01 mm³ and 2 mm³, 0.01 mm³ and 1.75 mm³, 0.01 mm³ and 1.5 mm³, 0.01 mm³ and 1.25 mm³, 0.01 mm³ and 1 mm³, 0.01 mm³ and 0.9 mm³, 0.01 mm³ and 0.8 mm³, 0.01 mm³ and 0.7 mm³, 0.01 mm³ and 0.6 mm³, 0.01 mm³ and 0.5 mm³, 0.01 mm³ and 0.4 mm³, 0.01 mm³ and 0.3 mm³, 0.01 mm³ and 0.2 mm³, 0.01 mm³ and 0.1 mm³, 0.01 mm³ and 0.07 mm³, 0.01 mm³ and 0.05 mm³, 0.01 mm³ and 0.03 mm³, 0.01 mm³ and 0.02 mm³, 0.05 mm³ and 6 mm³, 0.05 mm³ and 5.75 mm³, 0.05 mm³ and 5 mm³, 0.05 mm³ and 5.25 mm³, 0.05 mm³ and 4.75 mm³, 0.05 mm³ and 4.5 mm³, 0.05 mm³ and 4.25 mm³, 0.05 mm³ and 4 mm³, 0.05 mm³ and 3.75 mm³, 0.05 mm³ and 3.5 mm³, 0.05 mm³ and 3.25 mm³, 0.05 mm³ and 3 mm³, 0.05 mm³ and 2.75 mm³, 0.05 mm³ and 2.5 mm³, 0.05 mm³ and 2.25 mm³, 0.05 mm³ and 2 mm³, 0.05 mm³ and 1.75 mm³, 0.05 mm³ and 1.5 mm³, 0.05 mm³ and 1.25 mm³, 0.05 mm³ and 1 mm³, 0.05 mm³ and 0.9 mm³, 0.05 mm³ and 0.8 mm³, 0.05 mm³ and 0.7 mm³, 0.05 mm³ and 0.6 mm³, 0.05 mm³ and 0.5 mm³, 0.05 mm³ and 0.4 mm³, 0.05 mm³ and 0.3 mm³, 0.05 mm³ and 0.2 mm³, 0.05 mm³ and 0.1 mm³, 0.05 mm³ and 0.07 mm³, 0.1 mm³ and 6 mm³, 0.1 mm³ and 5.75 mm³, 0.1 mm³ and 5 mm³, 0.1 mm³ and 5.25 mm³, 0.1 mm³ and 4.75 mm³, 0.1 mm³ and 4.5 mm³, 0.1 mm³ and 4.25 mm³, 0.1 mm³ and 4 mm³, 0.1 mm³ and 3.75 mm³, 0.1 mm³ and 3.5 mm³, 0.1 mm³ and 3.25 mm³, 0.1 mm³ and 3 mm³, 0.1 mm³ and 2.75 mm³, 0.1 mm³ and 2.5 mm³, 0.1 mm³ and 2.25 mm³, 0.1 mm³ and 2 mm³, 0.1 mm³ and 1.75 mm³, 0.1 mm³ and 1.5 mm³, 0.1 mm³ and 1.25 mm³, 0.1 mm³ and 1 mm³, 0.1 mm³ and 0.9 mm³, 0.1 mm³ and 0.8 mm³, 0.1 mm³ and 0.7 mm³, 0.1 mm³ and 0.6 mm³, 0.1 mm³ and 0.5 mm³, 0.1 mm³ and 0.4 mm³, 0.1 mm³ and 0.3 mm³, 0.1 mm³ and 0.2 mm³, 0.5 mm³ and 6 mm³, 0.5 mm³ and 5.75 mm³, 0.5 mm³ and 5 mm³, 0.5 mm³ and 5.25 mm³, 0.5 mm³ and 4.75 mm³, 0.5 mm³ and 4.5 mm³, 0.5 mm³ and 4.25 mm³, 0.5 mm³ and 4 mm³, 0.5 mm³ and 3.75 mm³, 0.5 mm³ and 3.5 mm³, 0.5 mm³ and 3.25 mm³, 0.5 mm³ and 3 mm³, 0.5 mm³ and 2.75 mm³, 0.5 mm³ and 2.5 mm³, 0.5 mm³ and 2.25 mm³, 0.5 mm³ and 2 mm³, 0.5 mm³ and 1.75 mm³, 0.5 mm³ and 1.5 mm³, 0.5 mm³ and 1.25 mm³, 0.5 mm³ and 1 mm³, 0.5 mm³ and 0.9 mm³, 0.5 mm³ and 0.8 mm³, 0.5 mm³ and 0.7 mm³, 0.5 mm³ and 0.6 mm³, 1 mm³ and 6 mm³, 1 mm³ and 5.75 mm³, 1 mm³ and 5 mm³, 1 mm³ and 5.25 mm³, 1 mm³ and 4.75 mm³, 1 mm³ and 4.5 mm³, 1 mm³ and 4.25 mm³, 1 mm³ and 4 mm³, 1 mm³ and 3.75 mm³, 1 mm³ and 3.5 mm³, 1 mm³ and 3.25 mm³, 1 mm³ and 3 mm³, 1 mm³ and 2.75 mm³, 1 mm³ and 2.5 mm³, 1 mm³ and 2.25 mm³, 1 mm³ and 2 mm³, 1 mm³ and 1.75 mm³, 1 mm³ and 1.5 mm³, 1 mm³ and 1.25 mm³, 1.5 mm³ and 6 mm³, 1.5 mm³ and 5.75 mm³, 1.5 mm³ and 5 mm³, 1.5 mm³ and 5.25 mm³, 1.5 mm³ and 4.75 mm³, 1.5 mm³ and 4.5 mm³, 1.5 mm³ and 4.25 mm³, 1.5 mm³ and 4 mm³, 1.5 mm³ and 3.75 mm³, 1.5 mm³ and 3.5 mm³, 1.5 mm³ and 3.25 mm³, 1.5 mm³ and 3 mm³, 1.5 mm³ and 2.75 mm³, 1.5 mm³ and 2.5 mm³, 1.5 mm³ and 2.25 mm³, 1.5 mm³ and 2 mm³, 1.5 mm³ and 1.75 mm³, 2.0 mm³ and 6 mm³, 2.0 mm³ and 5.75 mm³, 2.0 mm³ and 5 mm³, 2.0 mm³ and 5.25 mm³, 2.0 mm³ and 4.75 mm³, 2.0 mm³ and 4.5 mm³, 2.0 mm³ and 4.25 mm³, 2.0 mm³ and 4 mm³, 2.0 mm³ and 3.75 mm³, 2.0 mm³ and 3.5 mm³, 2.0 mm³ and 3.25 mm³, 2.0 mm³ and 3 mm³, 2.0 mm³ and 2.75 mm³, 2.0 mm³ and 2.5 mm³, 2.0 mm³ and 2.25 mm³, 2.5 mm³ and 6 mm³, 2.5 mm³ and 5.75 mm³, 2.5 mm³ and 5 mm³, 2.5 mm³ and 5.25 mm³, 2.5 mm³ and 4.75 mm³, 2.5 mm³ and 4.5 mm³, 2.5 mm³ and 4.25 mm³, 2.5 mm³ and 4 mm³, 2.5 mm³ and 3.75 mm³, 2.5 mm³ and 3.5 mm³, 2.5 mm³ and 3.25 mm³, 2.5 mm³ and 3 mm³, 2.5 mm³ and 2.75 mm³, 3.0 mm³ and 6 mm³, 3.0 mm³ and 5.75 mm³, 3.0 mm³ and 5 mm³, 3.0 mm³ and 5.25 mm³, 3.0 mm³ and 4.75 mm³, 3.0 mm³ and 4.5 mm³, 3.0 mm³ and 4.25 mm³, 3.0 mm³ and 4 mm³, 3.0 mm³ and 3.75 mm³, 3.0 mm³ and 3.5 mm³, 3.0 mm³ and 3.25 mm³, 3.5 mm³ and 6 mm³, 3.5 mm³ and 5.75 mm³, 3.5 mm³ and 5 mm³, 3.5 mm³ and 5.25 mm³, 3.5 mm³ and 4.75 mm³, 3.5 mm³ and 4.5 mm³, 3.5 mm³ and 4.25 mm³, 3.5 mm³ and 4 mm³, 3.5 mm³ and 3.75 mm³, 4 mm³ and 6 mm³, 4 mm³ and 5.75 mm³, 4 mm³ and 5 mm³, 4 mm³ and 5.25 mm³, 4 mm³ and 4.75 mm³, 4 mm³ and 4.5 mm³, 4 mm³ and 4.25 mm³, 4.5 mm³ and 6 mm³, 4.5 mm³ and 5.75 mm³, 4.5 mm³ and 5 mm³, 4.5 mm³ and 5.25 mm³, 4.5 mm³ and 4.75 mm³, 5 mm³ and 6 mm³, or 5 mm³ and 5.75 mm³).

The microclosure can have any combination of the dimensions described herein. For instance, in some non-limiting embodiments, the microclosure has at least one dimension that is less than about 2 mm and an areal dimension that is less than about 2 mm². In other embodiments, the microclosure has at least one dimension that is less than about 2 mm and a volumetric dimension that is less than about 6 mm³. In yet other embodiments, the microclosure has at least one dimension that is less than about 2 mm and an areal dimension that is less than about 2 mm² and a volumetric dimension that is less than about 6 mm³. In some embodiments, the microclosure has an areal dimension that is less than about 2 mm² and a volumetric dimension that is less than about 6 mm³.

The present invention features a microclosure including any useful material(s) (e.g., any described herein, such as a stimulus-responsive material). The stimulus-responsive material can be included in the bulk material of the microclosure or within a portion of the microclosure (e.g., in a layer, such as a regulatable layer in a tunable dressing). In particular, exposure of the stimulus-responsive material to one or more external stimuli results in a change in a physical characteristic in the material(s). This change can extend across the entire microclosure (e.g., across the entire x-, y-, and/or z-direction of the microclosure, including planar and non-planar changes) or in a portion or part of the microclosure (e.g., at a localized area of the microclosure, which has been locally exposed to a stimulus and thereby results in a change in one or more physical characteristics in the x-, y-, and/or z- direction). Further, the microclosure can provide a variable tightening effect across the entire microclosure (e.g., varying degrees of tightening across the entire x-, y-, and/or z-direction of the microclosure, including planar and non-planar changes) or in a portion or part of the microclosure (e.g., varying degrees of tightening at a localized area of the microclosure).

Any useful physical characteristic of the device (e.g., microclosure) or material in the device can be changed. Exemplary physical characteristics include compression (or compressive force, e.g., lateral compression), expansion (e.g., lateral expansion), tension (e.g., as measured by tensile stress), structure, size, porosity, surface chemistry, bending modulus, fracture or failure strain, resilience, permeability, swelling ratio, elasticity (e.g., as measured by ultimate modulus of elasticity from the end-portion of stress-strain curves that is greater than 10 N/mm² (e.g., greater than about 15 N/mm², 20 N/mm², 25 N/mm², 30 N/mm², 35 N/mm², or 40 N/mm²) or between about 10 N/mm² and 200 N/mm² (e.g., about 10 N/mm² and 150 N/mm², 10 N/mm² and 100 N/mm², 15 N/mm² and 200 N/mm², 15 N/mm² and 150 N/mm², 15 N/mm² and 100 N/mm², 20 N/mm² and 200 N/mm², 20 N/mm² and 150 N/mm², or 20 N/mm² and 100 N/mm²)), electric conductivity, plasticity, resilience, resistance (e.g., as measured by creep resistance), strength (e.g., as measured by Young's modulus, such as a Young's modulus that is greater than about 1 × 10⁵ Nm⁻² (e.g., greater than about 2.0 × 10⁵ N/m², 2.5 × 10⁵ N/m², 3.5 × 10⁵ N/m², 4 × 10⁵ N/m², 4.5 × 10⁵ N/m² , 5 × 10⁵ N/m², 6 × 10⁵ N/m², 7 × 10⁵ N/m², 8 × 10⁵ N/m², 6 × 10⁵ N/m², or 10 × 10⁵ N/m²), tensile strength, such as a tensile strength that is greater than about 2 N/mm² (e.g., greater than about 5 N/mm², 7 N/mm², 10 N/mm², 15 N/mm², 17 N/mm², 20 N/mm², 25 N/mm², 27 N/mm², 30 N/mm², or 35 N/mm²) or between about 5 N/mm² and 40 N/mm² (e.g., between about 15 N/mm² and 30 N/mm², 15 N/mm² and 35 N/mm², 10 N/mm² and 30 N/mm², or 10 N/mm² and 35 N/mm²), compressive strength, impact strength, or yield strength), stress (e.g., as measured by compressive stress, shear stress, or tensile stress), load (e.g., load per millimeter width of at least 0.1 Newtons at a strain of at least 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or higher), strain (e.g., as measured by deflection, deformation, strain at failure, or ultimate strain (extension before rupture), e.g., greater than about 30% (e.g., greater than about 40%, 50%, 60%, 70%, 75%, 80%, 90%, 95%, 100%, 110%, 115%, or 120%) or from about 30% to 130% (e.g., about 30% to 120%, 30% to 115%, 30% to 110%, 30% to 100%, 30% to 95%, 30% to 90%, 30% to 85%, 30% to 80%, 30% to 75%, 30% to 70%, 30% to 65%, 30% to 60%, 30% to 55%, 30% to 50%, 35% to 130%, 35% to 120%, 35% to 115%, 35% to 110%, 35% to 100%, 35% to 95%, 35% to 90%, 35% to 85%, 35% to 80%, 35% to 75%, 35% to 70%, 35% to 65%, 35% to 60%, 35% to 55%, 35% to 50%, 40% to 130%, 40% to 120%, 40% to 115%, 40% to 110%, 40% to 100%, 40% to 95%, 40% to 90%, 40% to 85%, 40% to 80%, 40% to 75%, 40% to 70%, 40% to 65%, 40% to 60%, 40% to 55%, 40% to 50%, 50% to 130%, 50% to 130%, 50% to 120%, 50% to 115%, 50% to 110%, 50% to 100%, 50% to 95%, 50% to 90%, 50% to 85%, 50% to 80%, 50% to 75%, 50% to 70%, 50% to 65%, 50% to 60%, 50% to 55%, 60% to 130%, 60% to 120%, 60% to 115%, 60% to 110%, 60% to 100%, 60% to 95%, 60% to 90%, 60% to 85%, 60% to 80%, 60% to 75%, 60% to 70%, 60% to 65%, 70% to 130%, 70% to 120%, 70% to 115%, 70% to 110%, 70% to 100%, 70% to 95%, 70% to 90%, 70% to 85%, 70% to 80%, 70% to 75%, 75% to 130%, 75% to 120%, 75% to 115%, 75% to 110%, 75% to 100%, 75% to 95%, 75% to 90%, 75% to 85%, 75% to 80%, 80% to 120%, 80% to 115%, 80% to 110%, 80% to 100%, 80% to 95%, 80% to 90%, or 80% to 85%)), and other parameters.

Further, the extent or intensity of the physical characteristic can be increased or decreased after exposure to one or more stimuli. Exemplary physical characteristics include an increase in tension, a decrease in tension (e.g., of the microclosure), an increase in compressive force (e.g., lateral compressive force that is exerted by the microclosure), a decrease in compressive force (e.g., lateral compressive force that is exerted by the microclosure), compression in one or more directions of the microclosure, and/or expansion in one or more directions of the microclosure.

The change in one or more physical characteristics can be optimized based on the desired response to a stimulus, location of the skin region to be treated, or any other useful parameter. For instance, the change in physical characteristic can be optimized for placement in the eye region, where the eye region includes Langer lines having particular directions, and the directionality of compression or expansion exerted by the microclosure can be parallel to such Langer lines to promote skin tightening.

The directionality of the change in the physical characteristics, relative to the device (e.g., microclosure) or skin region, can also be optimized. In particular embodiments, the direction of skin tightening is determined by the directionality of the physical characteristic change. For instance, the direction of the tensile force or compressive force can be in the x-, y-, and/or z-direction with respect to the device or skin region (see, e.g., Figure 1 for the x-axis, z-axis, and x-z plane for an exemplary device relative to the skin portion; the y-axis would extend along the other plane of the skin, which is not shown). In particular embodiments, the device (e.g., a microclosure or a microdressing having a regulatable layer, a pre-stretched layer, and/or an unstretched layer) contracts or expands in one or more directions (e.g., in planar and/or non-planar directions) after exposure to a stimulus. Such a device may be used for any method described herein, such as to reduce pleating.

The intensity of the change in the physical characteristic(s), as compared to before exposure to one or more stimuli, can also be optimized. Such optimization can include selection of particular materials (e.g., one or more particular shape-memory polymers or alloys) or combinations of such materials to produce the intended effect (e.g., a combination of a rigid polymer with one or more particular shape-memory polymers or alloys), as well as arrangement (e.g., geometric or random arrangement) of such material(s) within a single layer in a device (e.g., within a single regulatable layer) or in separate multiple layers (e.g., in more than one regulatable layers, such as one, two, three, or more layers) in a device to produce the intended directionality and/or intensity of the physical characteristic(s).

The external stimulus to activate or induce the physical characteristic can be any useful stimulus. Exemplary stimulus includes a change in temperature, pH, light, moisture, solvent or chemical exposure, electric field, and/or magnetic field. In particular embodiments, the device includes one or more materials (e.g., in one or more layers) that can be activated by different external stimuli. The regulatable layer can include a first polymer (i.e., responding to stimulus A) and a second polymer (i.e., responding to stimulus B), where stimulus A and stimulus B are different types of stimuli (e.g., temperature and light) or different characteristics of the same stimulus (e.g., two different wavelengths of light). The first and second polymer can be the same polymer that has been modified, shaped, or processed to respond to different stimuli or different polymers having different chemical characteristics.

Furthermore, the change in physical characteristic or exposure of a stimulus can include the entire device or only a portion of the device. For example, the entire microclosure can be exposed to an external stimulus to induce a change in compression over the entire skin region to which the microclosure is affixed. Although the change in compression can occur over the entire skin region, the extent or intensity of compression can vary along the x-, y-, and/or z-axes or within the xy-, xz-, yz-, and/or xyz-planes of the skin region. In another example, the microclosure can be locally exposed to an external stimulus to induce a change in compression over a portion of the device (i.e., thereby resulting in a change in compression over a portion of the skin region). In particular embodiments, the device (e.g., a microclosure having a regulatable layer and/or an unstretched layer) contracts or expands in one or more directions (e.g., in planar and non-planar directions) in a portion of the area of the device after exposure to a stimulus. Such a device may be used for any method described herein, such as to reduce pleating.

Tunability of the microclosure can provide numerous benefits. For instance, such tunability can allow for real-time control of compressing and/or expanding the microclosure after affixation. This level of control can allow for personalized treatment of the patient based on the disease, disorder, or condition to be treated; the optimal cosmetic effect to be achieved; the optimal closure process to be achieved; and/or the timing and extent of the healing process observed for the particular patient. Furthermore, tunability can allow for less discriminate control over how the incisions or excisions in the skin region are made, as well as more discriminate control over selectively closing or opening the incisions or excisions.

The microclosure, which may be a tunable microclosure, can be affixed to the entire treated skin region or in a portion of the treated skin region. Directional or non-directional tightening can be achieved by producing a geometric arrangement of incisions and/or excisions that are treated similarly. Alternatively, such tightening can be achieved by a non-geometric arrangement of incisions and/or excisions in which only some of the incisions and/or excisions are opened or closed using a microclosure, which may be a tunable microclosure.

The microclosure can include an adhesive layer (e.g., formed from any adhesive material described herein). The adhesive layer can be continuous (i.e., a continuous layer of one or more adhesive materials attached to the proximal surface of a microclosure) or discontinuous (i.e., a noncontinuous layer of one or more adhesive materials attached to the proximal surface of a microclosure). The adhesive layer can include any useful arrangement of the adhesive material. For instance, the adhesive layer may be tunable and/or may allow for controlled compression or expansion. In some embodiments, an adhesive layer includes a random, non-geometric, or geometric array of an adhesive material for tunability. In particular embodiments, the array allows for directional or non-directional compression and/or expansion as the microclosure compresses and/or expands. In particular embodiments, the adhesive layer is discontinuous and includes an array of an adhesive material (e.g., an array of dots, where each dot gets closer together as the microclosure compresses and each dot gets further apart as the microclosure expands). Exemplary adhesive materials are described herein and include materials that promote collagen cross-linking, such as riboflavin or Rose Bengal, synthetic glues (e.g., cyanoacrylate, polyethylene glycol, or gelatin-resorcinol-formaldehyde), or biologic sealants (e.g., albumin-based or fibrin-based sealants that promote clotting).

The material(s) of the device can include any useful arrangement or form. Exemplary arrangements include a geometric arrangement of one or more materials within a single layer (e.g., a linear array or a grid of one or more materials in a single regulatable layer; or a linear array or a grid of one or more adhesive materials in a single adhesive layer); a geometric arrangement of one or more materials within multiple layers (e.g., in a multilayer dressing having more than one layer, where each layer includes a linear array or a grid of one or more materials and each linear array or grid is optimized for directional compression or expansion); a random, non-uniform arrangement of one or more materials in a single layer or across a plurality of layers; or combinations thereof. In some embodiments, a layer includes a first array of a first material and a second array of a second material, where each array has a geometric arrangement that promotes directional or non-directional compression or expansion. In particular embodiments, the first array is orthogonal to the second array. The materials can also be in any useful form, e.g., a film, a membrane (e.g., as in temperature shrink wrap), or an actuator having more complex geometries. In other embodiments, an adhesive layer includes an array of an adhesive material, where the array has a random, non-geometric, or geometric arrangement that allows for directional or non-directional compression or expansion as the regulatable layer or microclosure compresses and/or expands. In particular embodiments, the adhesive layer is discontinuous and includes an array of an adhesive material (e.g., an array of dots of an adhesive material).

The material(s) of the device can optionally include one or more actuators in any useful arrangement or form. Such actuators can be embedded in one or more materials and in one or more layers (e.g., in the regulatable layer, the pre-stretched layer, the unstretched layer, and/or the adhesive layer). Furthermore, the actuators can allow for uniform, non-uniform, or variable control (e.g., compression and/or expansion) across the entire device or in a portion of the device. Thus, actuators can be embedded across the entire device, in a portion of the device, in one layer, or in multiple layers. In particular embodiments, the stimulus-responsive material includes one or more actuators that respond to one or more stimuli, where the material includes a plurality of one type of actuator or a plurality of different actuators. The actuators in each layer can be arranged in any useful random, non-geometric, or geometric arrangement. Alternatively, the actuators can be arranged within multiple layers (e.g., in a multilayer dressing having more than one layer, where each layer includes a linear array or a grid of one or more actuators and each linear array or grid is optimized for directional compression or expansion); a random, non-uniform arrangement of one or more actuators in a single layer or across a plurality of layers; or combinations thereof. Exemplary materials including one or more actuators are described herein.

The material(s) or layer(s) in a device (e.g., a microclosure) can include an unstretched layer (e.g., including any material described herein) and an adhesive layer. The unstretched layer can include one or more unstretched materials, including those having sufficient rigidity to hinder stretching and those having one or more stretchable polymers that are not stretched prior to affixing to a skin region.

The material(s) or layer(s) in a device (e.g., a microclosure) can include an adhesive layer, a regulatable layer, as well as one or more additional, optional layers or fasteners (e.g., staples, sutures, etc.). Exemplary optional layers include an occlusion layer (e.g., to control humidity and/or promote wound healing), an absorption layer (e.g., to absorb wound exudate), a reinforcement layer (e.g., to reinforce the layer and optionally formed from low-density polyethylene (LDPE), fluorinated ethylene propylene (FEP), or nylon), and/or a delivery layer (e.g., to delivery one or more therapeutic agents, as described herein).

In particular embodiments, a plurality of microclosures are used in conjunction with a macrodressing adapted to attach on top of the microclosures. In some embodiments, the macrodressing is a tunable dressing (e.g., as described herein), which allows for controlling a first compressive force exerted by the combination of the microclosures and macrodressing. For instance, if the microclosures are a plurality of microstaples, each having an attachment component, then the macrodressing can include one or more structures adapted to engage each attachment component. In use, when the macrodressing is tuned, then the attached microstaples will also be tuned by moving relative to the macrodressing. For instance, if the macrodressing is compressed in the x-direction, then the distance between the microstaples will be compressed in the x-direction. Accordingly, the invention also includes combinations of one or more microclosures with any device herein (e.g., a dressing, such as a macrodressing).

The device (e.g., microclosure) can optionally include an applicator, as described herein. In some embodiments, the applicator is a frame or any other useful structure that provides sufficient support to the microclosure and/or provides a sterile method to affix the microclosure to the treated skin region (e.g., where the microclosure may be a tunable microclosure, or any described herein). In other embodiments, the applicator is configured to attach to an apparatus that forms one or more incisions and/or excisions, where the applicator allows for releasing and/or affixing the microclosure after the formation of such an incision or excision (e.g., within about 30, 25, 20, 15, 10, 5, 3 seconds or less after forming an incision or excision).

The device can be of any cosmetically appealing color, shape, and/or material. For example, the microclosure can be provided in a skin tone color or is transparent or semi-transparent. Such transparent or semi-transparent microclosures can additionally be helpful for visualization, e.g., for real-time tunability of the microclosure and/or for affixing the microclosure to the treated skin region.

Exemplary microclosures and materials for constructing such microclosure are described herein.

### Testing of devices

To optimized function of any of the devices described herein, the appropriate force (e.g., compressive, tensile, and/or lateral force) and/or geometric arrangement of the device (e.g., a microclosure) can be tested by any useful metric. Exemplary metrics include any useful endpoint, such as presence or absence of melanocytes, melanin in keratinocytes, collagen production, elastin, scarring and/or infection, fibroblast activity, inflammation, macrophage and/or leukocyte recruitment, or the relative thickness of the papillary dermis and/or epidermis; melanin index, which is a unitless variable that quantifies the concentration of melanin in skin (e.g., by obtaining a reflectance spectrum and determining the slope of the log of the inverse reflectance values for wavelengths between 620 and 700 nm); erythema index, which is a unitless variable that quantifies the concentration of melanin and/or hemoglobin in skin (e.g., by obtaining an absorption spectrum and determining the log of the ratio of the reflectance at 635 nm and at 565 nm, such as by using a commercially available reflectance instrument from Diastron (Hampshire, U.K.)); transepidermal water loss, which measures the quantity of water that passes from the inside of a body through the epidermal layer; the Glogau wrinkle assessment scale with a scoring system of type I (no wrinkles), type II (wrinkles in motion), type III (wrinkles at rest), and type IV (only wrinkles), as described in Glogau, "Aesthetic and anatomic analysis of the aging skin," Semin. Cutan. Med. Surg. 15(3):134-138 (1996); and/or the Fitzpatrick wrinkle assessment scale (FWAS) or modified FWAS (MWAS) with a scoring system of 0 (no wrinkle: no visible wrinkle, continuous skin line), 0.5 (very shallow yet visible wrinkle), 1 (fine wrinkle: visible wrinkle and slight indentation), 1.5 (visible wrinkle and clear indentation with less than 1 mm wrinkle depth), 2 (moderate wrinkle: clearly visible wrinkle with 1 mm to 2 mm wrinkle depth), 2.5 (prominent visible wrinkle with more than 2 mm and up to 3 mm wrinkle depth), and 3 (deep wrinkle: deep and furrow wrinkle with more than 3 mm wrinkle depth).

### Attachment component

The devices (e.g., microclosures) can include one or more attachment components, which can be adapted to attach (e.g., reversibly or irreversibly attach) to a mechanical lifting device. In some embodiments, the remover or the mechanical lifting device is configured to detach all the microclosure devices once the wound is healed or to detach some of the microclosures to titrate the tightening effect immediately after application of the microclosures.

Exemplary attachment components include a hook, a pincher, an eye, a loop, a post, a microfastener, a slot, a snap fastener, and combinations thereof (e.g., a hook-and-eye combination in a Velcro™ material). Such attachment components can be included on the microclosure by any useful method, including by adhesive bonding, mechanical bonding, ultrasonic bonding, sewing, stitching, serging, edging, and the like. Exemplary attachment components are described in U.S. Pat. Nos. 6,936,039; 6,893,388; 6,669,618; 6,432,098; and 6,251,097, and U.S. Pub. Nos. 2005/0234419; 2005/0215971; 2005/0215970; 2005/0130821 ; 2004/0023771; 2003/0233082; 2003/01.19641 ; 2003/0088220; and 2002/0169431, each of which is incorporated herein by reference.

A mechanical lifting device includes any apparatus, substrate, or component adapted to attach to one or more attachment components to facilitate removal of one or more microclosures. In some embodiments, the remover and/or mechanical lifting device includes heat, optical, radiofrequency, mechanical, and/or ultrasound components to remove one or more microclosures. In particular embodiments, the mechanical lifting device and attachment component of the microclosure are complementarily designed. For instance, the mechanical lifting device and attachment component can together form a hook-and-eye system, such as in a Velcro™ system. In one non-limiting example, the mechanical lifting device is a substrate including one or more hooks, and the microclosure includes an eye adapted to engage to a hook. In another non-limiting example, the mechanical lifting device is a substrate including one or more eyes, and the microclosure includes a hook adapted to engage to an eye.

### Further processing of devices

The devices (e.g., microclosures) can be further processed prior to affixing to the subject. Exemplary processes include sterilization (e.g., with ultrasound, ultraviolet light, heat, and/or plasma); treatment with one or more antimicrobials (e.g., treatment with chlorhexidine gluconate or silver, such as a silver nitrate or Ag⁺ in one or more useful carriers, as described herein); and/or treatment with one or more agents, e.g., to form a coating on the microclosure, where exemplary agents include a biocompatible matrix (e.g., those including at least one of collagen (e.g., a collagen sponge), low melting agarose (LMA), polylactic acid (PLA), and/or hyaluronic acid (e.g., hyaluranon)), a photosensitizer (e.g., Rose Bengal, riboflavin-5-phosphate (R-5-P), methylene blue (MB), N-hydroxypyridine-2-(1H)-thione (N-HTP), a porphyrin, or a chlorin, as well as precursors thereof), a photochemical agent (e.g., 1,8 naphthalimide), a fibrin sealant, a cyanoacrylate adhesive, or a tissue glue composed of a mixture of riboflavin-5-phosphate and fibrinogen

### Methods of skin treatment

The present invention relates to various methods and devices (e.g., microclosures) used to selectively open or close incisions and/or excisions (e.g., all or a portion of such incisions, such as microslits, and/or excisions, such as holes) formed in the skin region by the incised or excised tissue portions. The devices can be affixed to the entire treated skin region or in a portion of the treated skin region, which allow for directional or non-directional tightening by producing a geometric or non-geometric arrangement of incisions and/or excisions that are treated similarly or differently. Further, the devices can provide uniform or non-uniform compression and/or expression across the entire device or a portion thereof. Accordingly, these methods and devices can result in reducing tissue volume or area, promoting beneficial tissue growth, tightening skin, rejuvenating skin, improving skin texture or appearance, removing skin laxity, and/or expanding tissue volume or area.

The methods can include contraction or expansion in one or more directions in at least a portion of the device (e.g., the microclosure). The methods include, for example, affixing a device to a skin region having a plurality of incised tissue portions and/or excised tissue portions (e.g., where at least two of said tissue portions has at least one dimension that is less than about 1 mm or an areal dimension that is less than about 1 mm²). The device provides contraction or expansion of the skin region in one or more directions (e.g., in the x-, y-, z-, xy-, xz-, yz-, and/or xyz-directions, as described herein), where such contraction or expansion can be uniform or non-uniform. Furthermore, contraction or expansion arises by exposing an affixed device to one or more external stimuli (e.g., any described herein) that results in a change in a physical characteristic of the device. In addition, such contraction and/or expansion can be adjusted after affixing the device. For example, after treating the skin and affixing the device, the device can result in expansion of the skin region and then later exposed to an external stimulus to further expand or to compress the skin region. In this manner, the device may be tunable.

The present invention also includes methods of tightening skin in a preferred direction. Directional tightening of the skin (e.g., by compression and/or expansion exerted by the device) can be optimized by using one or more materials in one or more layers of the device. Such compression and/or expansion can be controlled independently (e.g., by use of one or more stimuli).

The present invention also includes optimizing the dimension of the incised or excised tissue portions to promote wound healing. Exemplary dimensions include circular and non-circular holes, such as elliptical holes (e.g., as viewed from the xy-plane). Non-circular holes can be formed by using an apparatus having a non-circular cross-section (e.g., a blade or a tube, such as a hollow tube, having a non-circular cross-section) or by pre-stretching the skin before treatment with an apparatus having a circular cross-section (e.g., a circular coring needle generates an elliptical hole in a non-stretched skin). In some embodiments, the long axis of the ellipse is perpendicular to the pre-stretching direction, where the elliptical hole can generate skin tightening preferentially in the direction of the short axis of the ellipse. Accordingly, the devices of the invention (e.g., a microclosure, as described herein) can be affixed to a skin portion including one or more elliptical holes or one or more incised or excised tissue portions having one or more elliptical geometries.

The methods and devices herein can allow for less discriminate methods for treating the skin by forming holes or slits because the methods and devices allow for more discriminate control for closing or opening such holes or slits. For instance, microclosures can allow for real-time control for compressing or expanding holes or slits. Furthermore, such microclosures may also be tunable microclosures. Exemplary modes of control include the extent of compression or expansion, the directionality of compression or expansion (e.g., in the x-, y-, z-, xy-, yz-, xz-, or xyz-direction), and/or timing of applying the compression or expansion (e.g., within a few seconds, such as within 30, 20, 15, 10, 5, 3 seconds, or less).

### Control of skin pleating

Furthermore, the methods and devices of the invention can be used to control skin pleating. For example, when using microclosure to compress the skin and close holes and/or slits, it may be advantageous to apply an optimal compression level that can be adjusted during the treatment period and after affixing the microclosure. During the setting of the tissue, skin pleating can be beneficial in some instances and should be avoided in other instances. After the excision or incision, the tissue can be compressed or expanded in order to set the tissue. In particular examples, the setting time may be as short as 2-4 days, and the microclosure provides compression or expansion prior to this setting time. In some embodiments, the microclosures may be held in a planar configuration at the same time that lateral compression is applied and therefore promote compression without pleating. Accordingly, the methods and devices or the invention can be used to control the level of compression and/or expansion exerted by the device to increase and/or decrease the extent of skin pleating.

The state of the tissue can provide feedback about the optimal compression level, such that tissue pleating can be controlled. Tissue pleating may affect the wound healing process and hole geometry. Furthermore, in some instances, pleating may prevent conformal adhesion of the device with the treated skin region, thereby affecting the function of a wound microclosure that requires contact with the skin. Accordingly, pleating can be controlled by inspecting the skin periodically and adjusting the microclosure affixed to the skin region (e.g., by exposure of one or more external stimuli, where the microclosure may be a tunable microclosure). Alternatively, the microclosure can control pleating by having limited flexibility (e.g., by including one or more rigid materials or unstretched materials, as described herein) or limited flexibility in particular areas and/or directions.

The methods and devices for skin tightening can also be optimized for conforming to uneven skin surfaces, whether such surfaces arise from a particular disease or condition (e.g., any described herein) or from the anatomical location of the skin region (e.g., in the brow, chin, or breast regions). Such unevenness can occur in any direction or plane, including non-planar and planar unevenness. In some embodiments, the microclosure (e.g., tunable microclosure) includes one or more materials that allow for contraction or expansion of the skin region in one or more directions (e.g., in the x-, y-, z-, xy-, xz-, yz-, and/or xyz-directions, as described herein), as well as in planar and non-planar directions (e.g., in the xy-, xz-, yz-, and/or xyz-planes). When treating uneven skin surfaces, tissue pleating can be a particular concern that should be controlled. Thus, the methods, devices, and microclosures described herein (e.g., which may be a tunable microclosure, or any described herein) can be useful for optimizing compression and/or expansion levels in any useful direction(s) for treating uneven skin surfaces, while controlling pleating.

### Materials

The methods, devices (e.g., microclosures), and apparatuses of the invention can include any useful materials. In a microdressing, the regulatable layer can include one or more stimulus-responsive materials (e.g., a shape-memory material, a shape-memory polymer, a shape-memory alloy, a thermal-responsive material, a pH-responsive material, a light-responsive material, a moisture-responsive material, a solvent-responsive or chemical exposure-responsive material, an electric field-responsive material, a magnetic field-responsive material, an actuator-embedded material, and/or an unstretched material). The adhesive layer can include one or more adhesive materials (e.g., pressure sensitive adhesives).

The materials can include arise from any useful mechanism for compressing and/or expanding the device, as well as any useful stimulus. Such mechanisms include mechanical, hydraulic, and/or pneumatic modes of operation. Exemplary stimulus includes a change in temperature, pH, light, moisture, solvent, chemical exposure, electric field, and/or magnetic field, which can optionally result in mechanical, hydraulic, and/or pneumatic tuning.

The materials can be of any useful form. Exemplary forms include an emulsion, a fiber, a film, a foam, a hydrogel, a solution, a laminate, or any other form that can be further processed, such as shaped, cast, extruded, molded (e.g., by blow molding, injection molding, or resin transfer molding), woven, cross-linked, deposited, laminated, and/or spun (e.g., by wet spinning, electrospinning, and/or melt spinning) to any useful article (e.g., a microclosure or one or more layers within a microdressing).

### Polymers and plastics

The microclosures can be formed from any useful polymer or plastic. Exemplary polymers and plastics include, e.g., alginate, benzyl hyaluronate, carboxymethylcellulose, cellulose acetate, chitosan, collagen, dextran, epoxy, gelatin, hyaluronic acid, hydrocolloids, nylon (e.g., nylon 6 or PA6), pectin, poly (3-hydroxyl butyrate-co- poly (3-hydroxyl valerate), polyacrylate (PA), polyacrylonitrile (PAN), polybenzimidazole (PBI), polycarbonate (PC), polycaprolactone (PCL), polyester (PE), polyethylene glycol (PEG), polyethylene oxide (PEO), PEO/polycarbonate/polyurethane (PEO/PC/PU), poly(ethylene-co-vinyl acetate) (PEVA), PEVA/polylactic acid (PEVA/PLA), poly (ethylene terephthalate) (PET), PET/poly (ethylene naphthalate) (PET/PEN) polyglactin, polyglycolic acid (PGA), polyglycolic acid/polylactic acid (PGA/PLA), polyimide (PI), polylactic acid (PLA), poly-L-lactide (PLLA), PLLA/PC/polyvinylcarbazole (PLLA/PC/PVCB), poly (β-malic acid)-copolymers (PMLA), polymethacrylate (PMA), poly (methyl methacrylate) (PMMA), polystyrene (PS), polyurethane (PU), poly (vinyl alcohol) (PVA), polyvinylcarbazole (PVCB), polyvinyl chloride (PVC), polyvinylidenedifluoride (PVDF), polyvinylpyrrolidone (PVP), silicone, rayon, polytetrafluoroethylene (PTFE), polyether ether ketone (PEEK), or combinations thereof.

### Metals and metal alloys

The microclosures can be formed from any useful metal or metal alloy. Exemplary metals and alloys include stainless steel; titanium; a nickel-titanium (NiTi) alloy; a nickel-titanium-niobium (NiTiNb) alloy; a nickel-iron-gallium (NiFeGa) alloy; a nickel-manganese-gallium (NiMnGa) alloy; a copper-aluminum-nickel (CuAlNi) allow; a copper-zinc (CuZn) alloy; a copper-tin (CuSn) alloy; a copper-zinc-aluminum (CuZnAl) alloy; a copper-zinc-silicon (CuZnSi) alloy; a copper-zinc-tin (CuZnSn) alloy; a copper-manganese alloy; a gold-cadmium (AuCd) alloy; a silver-cadmium (AgCd) alloy; an iron-platinum (FePt) alloy; an iron-manganese-silicon (FeMnSi) alloy; a cobalt-nickel-aluminum (CoNiAl) alloy; a cobalt-nickel-gallium (CoNiGa) alloy; or a titanium-palladium (TiPd) alloy.

### Shape-memory materials

Shape-memory materials (SMMs) can change their physical conformation (or shape) under an external stimulus (e.g., a thermal stimulus). For example, an article formed from an SMM or coated with an SMM can possess a first, desired shape and a second, temporary shape. When the SMM is regulatable by temperature, switching between these two shapes is achieved by heating or cooling above the glass or melting transition temperature of the SMM. SMMs may have a completely reversible transition (e.g., in a material that returns to its original shape) or a partially reversible transition with hysteresis (e.g., resulting in a material requiring additional energy to return to its original shape). SMMs can have multiple external stimulus responses, such as responses to both temperature and light or temperature and magnetic fields.

SMMs include both shape-memory polymers (SMPs) and shape-memory alloys (SMAs). SMPs can be in any useful form, such as in the form of the parent polymer chain, gels, hydrogels, emulsions, or micelles. Exemplary SMPs include shape-memory polyurethane (e.g., a polypropylene glycol) (PPG), 4,4'-diphenylmethane diisocyanate (MDI), and dimethylolpropionic acid (DMPA) (PPG/MDI/DMPA) copolymer, where -NCO is optionally end-capped with methyl ethyl ketoxime (MEKO), or polymers including dimethyloldihydroxyethylene urea (DMDHEU) and/or 1,2,3,4-butane tetra-carboxylic acid (BTCA)); a poly(ethylene terephthalate)/poly(caprolactone) (PET/PCL) block copolymer (e.g., optionally crosslinked with maleic anhydride, glycerin, or dimethyl 5-isophthalate); a polyethylene terephthalate/polyethylene oxide (PET/PEO) block copolymer; an ABA triblock copolymer made from poly(2-methyl-2-oxazoline) and polytetrahydrofuran; a polystyrene and poly(1,4-butadiene) (PS/PBD) block copolymer; a polyethylene glycol/4,4'-diphenylmethane diisocyanate/pentaerythritol (PEG/MDI/PE) copolymer; polynorbornene ((C₇H₁₀)ₓ or Norsorex®, available from Astrotech Advanced Elastomer Products GmbH, Vienna, Austria); organic-inorganic hybrid polymers including polynorbornene units partially substituted by polyhedral oligosilsesquioxane (POSS); an acrylate-based polymer; a styrene-based polymer; an epoxy-based polymer); and shape-memory fibers (e.g., oligomers prepared with polyol as the soft segment and small size diols and MDI as the hard segment).

Exemplary SMAs include a nickel-titanium (NiTi) alloy (e.g., Nitinol™, available from Nitinol Devices & Components, Inc., Fremont, CA, of approximately 55% Ni); a nickel-titanium-niobium (NiTiNb) alloy; a nickel-iron-gallium (NiFeGa) alloy; a nickel-manganese-gallium (NiMnGa) alloy; a copper-aluminum-nickel (CuAlNi) alloy (e.g., 14/14.5 wt.% Al and 3/4.5 wt.% Ni); a copper-zinc (CuZn) alloy (e.g., 38.5/41.5 wt.% Zn); a copper-tin (CuSn) alloy (e.g., approximately 15 at.% Sn); a copper-zinc-aluminum (CuZnAl) alloy; a copper-zinc-silicon (CuZnSi) alloy; a copper-zinc-tin (CuZnSn) alloy; a copper-manganese alloy (e.g., 5/35 at.% Cu); a gold-cadmium (AuCd) alloy (e.g., 46.5/50 at.% Cd); a silver-cadmium (AgCd) alloy (e.g., 44/49 at.% Cd); an iron-platinum (FePt) alloy (e.g., approximately 25 at.% Pt); an iron-manganese-silicon (FeMnSi) alloy (e.g., approximately 25 at.% Pt); a cobalt-nickel-aluminum (CoNiAl) alloy; a cobalt-nickel-gallium (CoNiGa) alloy; or a titanium-palladium (TiPd) alloy.

SMMs can also include shape-memory composites (SMC) and shape-memory hybrids (SHC). SMCs and SMHs are dual component systems that include at least one SMM integrated with conventional materials. Exemplary conventional materials include those useful for preparing wound care dressings, such as any described herein and include, e.g., alginate, benzyl hyaluronate, carboxymethylcellulose, cellulose acetate, chitosan, collagen, dextran, epoxy, gelatin, hyaluronic acid, hydrocolloids, nylon (e.g., nylon 6 or PA6), pectin, poly (3-hydroxyl butyrate-co- poly (3-hydroxyl valerate), polyacrylate (PA), polyacrylonitrile (PAN), polybenzimidazole (PBI), polycarbonate (PC), polycaprolactone (PCL), polyester (PE), polyethylene glycol (PEG), polyethylene oxide (PEO), PEO/polycarbonate/polyurethane (PEO/PC/PU), poly(ethylene-co-vinyl acetate) (PEVA), PEVA/polylactic acid (PEVA/PLA), poly (ethylene terephthalate) (PET), PET/poly (ethylene naphthalate) (PET/PEN) polyglactin, polyglycolic acid (PGA), polyglycolic acid/polylactic acid (PGA/PLA), polyimide (PI), polylactic acid (PLA), poly-L-lactide (PLLA), PLLA/PC/polyvinylcarbazole (PLLA/PC/PVCB), poly (β-malic acid)-copolymers (PMLA), polymethacrylate (PMA), poly (methyl methacrylate) (PMMA), polystyrene (PS), polyurethane (PU), poly (vinyl alcohol) (PVA), polyvinylcarbazole (PVCB), polyvinyl chloride (PVC), polyvinylidenedifluoride (PVDF), polyvinylpyrrolidone (PVP), silicone, rayon, or combinations thereof.

Exemplary SMCs include dual component systems including SMM materials in contact with conventional materials, such that the conventional material applies a force to bend the SMM absent an external stimulus. Upon the addition of an external stimulus, the SMM changes shape, thus overcoming the force applied from the conventional material. The resulting shape transition moves both the SMM and conventional components. In addition, SMHs exhibit the characteristic shape transitions of SMM but are constructed from conventional materials (e.g., non-shape-memory materials). Exemplary SMHs include dual region plastic materials constructed of two conventional polymers layers, where the material bends in response to temperature changes due to the difference in thermal expansion between the two plastic layers. Additional exemplary SMC and SHC materials can be found in, e.g., in Huang et al., "Shape memory materials," Materials Today 13:54-64 (2010), which is hereby incorporated by reference in its entirety.

### Thermal-responsive materials

Thermal-responsive materials can change their physical and chemical properties upon changes in temperature. The transition temperature is the temperature at which the polymer's characteristics change and includes a lower critical solution temperature (LCST) or an upper critical solution temperature (UCST). A common, exemplary response to temperature change is a transition in the hydrophilic/hydrophobic character of the material. A transition to a more hydrophobic state results from changes in the polymer's ability to hydrogen bond with the surrounding environment (e.g., a solvent or solution). For a thermal-responsive polymer dissolved in solution, the temperature response can result in a transition in the polymer's conformation and solvent interaction. This transition includes an expanded state with extensive solvent interaction and a contracted state with limited solvent interaction. In the contracted state, the thermal-responsive polymer will become insoluble and precipitate from solution.

The same above-described transition can occur in other forms (e.g., in gels, such as in hydrogels in which the cross-linked polymer is swollen by a solvent, or in copolymers). Upon exposure to a temperature transition, the conformation of the polymer network changes, thus resulting in reduced solvent interactions and causing a reduction in the gel's volume. Often the transition temperature is independent of the polymer's molecular weight. The transition temperature can be modified with changes to the solvent system. For example, the addition of cosolvents or salts can increase or decrease the transition temperature. For copolymers, the transition temperature in aqueous environments is generally decreased with the addition of more hydrophobic co-monomers or polymer modifying groups. Alternatively, the transition temperature is generally increased by the addition of more hydrophilic co-monomers or polymer modifying groups.

Exemplary thermal-responsive materials include poly-N-isopropylacrylamide (poly-NIPAAm, LCST at 32-37°C); poly-N-vinylcaprolactam (LCST at 25-35°C); poly-N,N-diethylacrylamide (LCST at 25-32°C); other polyalkylacrylamides and co-polymers of polyalkylacrylamides; polyethylene glycol; polyethylene oxide (PEO, LCST at about 85°C); polypropylene oxide (PPO); polymethylvinyl ether (LCST at 34-38°C); and PEO-PPO copolymers. Exemplary thermal-responsive gels or hydrogels include copolymer networks of include poly-N-isopropylacrylamide, poly-N-vinylcaprolactam, poly-N,N-diethylacrylamide, and other polyalkylacrylamides with a cross-linker, such as methylene bisacrylamide. Such thermal-responsive materials can be provided in any form, such as heat shrink films.

### pH-responsive materials

pH-responsive materials can change their physical and chemical properties upon changes in pH. A transition can arise from increased charge density resulting from protonation or deprotonation of a polymer or from decreased charge density resulting from neutralization of the polymer. In general, increasing the charge density results in increased hydrophilicity, which in turn promotes interactions with water, polar solvents, or salts. Decreasing charge density typically makes the polymer more hydrophobic and reduces the interactions with water, polar solvents, and salts.

The nature of the pH transition results from the type of acid/base functionalities present in the material. For example, the presence of amine functionalities (e.g., moieties with a high pKa) results in higher charge densities as the pH decreases and neutralization of the charge as the pH increases. Conversely, the presence of carboxylic acid functionalities (e.g. moieties with a low pKa) results in higher charge densities as the pH increases and neutralization of the charge as the pH decreases. For a pH-responsive polymer in solution, a transition from a higher charge density to a lower charge density (e.g., neutralization of charge) can result in the polymer becoming insoluble and precipitating from solution. An insoluble pH-responsive polymer can dissolve into water as the charge density is increased (e.g. increasing pH for a carboxylic acid moiety containing polymer). The same pH-response transition can occur in numerous forms, such as gels or hydrogels. Typically, an increasing charge density causes the gel network to swell with water, polar solvents, or salts, thus expanding the gel's volume. Conversely, the neutralization of charge results in a reduction of the gel volume due to the elimination of water, polar solvents or salts from the network.

Exemplary pH-responsive polymers include polyacrylic acid, polymethacrylic acid, and methacrylic acid/methyl methacrylate copolymers (Eudragit®, Evonik Industries AG); copolymers of polyacrylic acid and polyvinyl alcohol (PAA/PVA); carboxylic acid derivatives of styrene; derivatives of cellulose such as carboxymethylethylcellulose, cellulose acetate-phthalate and diethylaminoethyl cellulose; diethylaminoethyl methacrylate/methyl methacrylate or butyl methacrylate copolymers (e.g., insoluble at pH 7, soluble at acidic pH); polypyridine; polyallylamine, polyvinylamine, chitosan, and other polyamines; as well as N-dimethylaminoethyl methacrylate, biodegradable copolymers of N,N-dimethylacrylamide, N-tert-butyl acrylamide and N-methylacryoylglycylglycine p-nitrophenyl ester. Exemplary pH-responsive gels or hydrogels include methacrylic acid/methyl methacrylate polymer networks crosslinked with a bifunctional methacrylate, such as 1,4-butanediol dimethacrylate, carboxylic acid derivatives of styrene crosslinked with divinylbenzene; cellulose derivatives crosslinked with multifunctional cross-linking agents such as butanediol diglycidylether; as well as copolymers of polyacrylic acid and polyvinyl alcohol cross-linked with a divinyl group such as 1,4-butanediol dimethacrylate. Exemplary pH-sensitive materials are found, e.g., in Galaev et al., Russian Chem. Reviews 64: 471-489 (1995), which is hereby incorporated by reference in its entirety.

### Light-responsive materials

Light-responsive materials can change their physical and chemical properties upon exposure to electromagnetic radiation. Typically, moieties within the polymer structure undergo a change in response to light of a particular energy. The light provides energy for the moiety to overcome activation energy barriers and transition into a different conformation or state. For example, a copolymer incorporating an azobenzene chromophore has a lower dipole moment (e.g., is less polar) in the trans conformation around the azo double bond. The azobenzene moiety provides a light sensitive "switch," which provides the response to external stimulus. Upon irradiation with light, the double bond can isomerizes to the cis conformation, thus increasing the dipole moment (e.g., making the polymer more polar). The increase in polarity can result in increased solubility in polar solvents. This phenomenon is observed with a dimethylacrylamide-4-phenylazophenylacrylate (7.5 mol%) copolymer. At a temperature slightly above the LCST, the solution is generally cloudy. However, upon UV irradiation, the copolymer's LCST is reduced below the environmental temperature and the solution becomes clear as the copolymer dissolves. Exemplary light-sensitive polymers are found, e.g., in Galaev et al., Russian Chem. Reviews 64:471-489 (1995), which is hereby incorporated by reference in its entirety.

Light-responsive polymers include those having one or more of the following light-responsive switches: cinnamic acid, cinnamylidene acetic acid, azobenzene chromophores (e.g., 4-phenylazobenzene), triarylmethylcyanide, stilbene, or quinone-methide moieties.

### Moisture-responsive materials

Moisture-responsive materials can change their physical and chemical properties upon a change in the environmental humidity or water content. This transition generally involves an increasing or decreasing association with other components in the medium following exposure to water. Essentially, water displaces or increases the volume of the existing medium thus causing changes commiserate with the polymer's hydrophilicity. For example, the grafted polymer, polymethacrylic acid-graft-polyethylene glycol, is collapsed in solutions with a high ethanol/water ratio. Upon addition of water the polymer swells thus increasing the polymers porosity. This volumetric change enables the release of therapeutic compounds after contacting the polymer containing therapeutic compounds with a mucus membrane. This exemplary moisture-sensitive polymer is found in de las Heras Alarcon et al., Chem. Soc. Rev. 34:276-285 (2005), which is hereby incorporated by reference in its entirety.

Exemplary moisture-sensitive polymers include copolymers of ionic monomers, such as acrylamidopropane sulfonic acid sodium salt with neutral monomers, such as acrylamide; pH sensitive polymers, as described above with high charge density; and grafted polymers, such as polymethacrylic acid-graft-polyethylene glycol.

### Solvent-responsive or chemical exposure-responsive materials

Solvent-responsive materials can change their physical and chemical properties upon a change in the solvent or chemical content of the surrounding medium or environment. Similar to the moisture-responsive materials described above, solvent or chemical exposure-responsive materials possess a transition involving an increasing or decreasing association with other components in the medium following exposure to a solvent or chemical. Generally, the solvent responds to displaces or increases the volume of the existing medium, thus causing changes consistent with the polymer's relative compatibility between the exposed solvent and the existing medium. The solvent-responsive material can be a polymer composite with another material or a modified non-polymeric material.

En exemplary chemical-responsive material is a combination of activated carbon and polyaniline were formed into a composite structure or chemiresistive detector. Adsorption of biogenic amines causes a response in the polymer component, which changes the resistance of the composite and yields an electrical signal indicating the presence of the analyte. This exemplary solvent-sensitive polymer composite is found in patent number EP1278061B1, which is hereby incorporated by reference in its entirety

Exemplary solvent or chemical exposure-responsive materials include a polymer/carbon black composite, polyaniline/carbon black composite, gold/para-substituted thiophenol, gold clusters encapsulated with octanethiol, and a dendrimer of poly(amidoamine).

### Electric field-responsive materials

Electric field-responsive materials can change their physical and chemical properties upon changes to the applied electric field. The electric field-responsive materials can be metal or a composite material including a polymer and metal. In general, the electric field stimulates a electric field sensitive component or electric "switch." The polymer component of electric field-sensitive composites can be made from any polymer with the desired polymer properties.

Electric field-responsive materials include those having one or more of the following switches: carbon black, carbon nanotube, metallic Ni powder, short carbon fibers (SCFs), or super-paramagnetic nanoparticles (e.g., magnetite nanoparticles). Electric field-responsive materials can optionally include any composite or material described herein.

### Magnetic field-responsive materials

Magnetic field-responsive materials can change their physical properties upon changes to the applied magnetic field. The magnetic field-responsive materials can be metal or metal polymer composite materials. In general, the magnetic field stimulates a magnetic field sensitive component or magnetic "switch." The polymer component of magnetic field-sensitive composites can be made from any polymer with the desired polymer properties.

Exemplary magnetic field-responsive materials or magnetic switches include magnetite, poly[aniline-co-N-(1-butyric acid)]aniline/iron oxide, polylactide/nanocrystalline magnetite, maghemite, cobalt ferrite, carbonyl iron, ferromagnetic shape-memory alloy, magnetic nanoparticles (e.g., such as iron, cobalt, or iron oxide (e.g., Fe₃O₄)), spinel ferrimagnets (e.g., such as CoFe₂O₄ and MnFe₂O₄), and alloys (e.g., CoPt₃ and FePt). Exemplary polymers for magnetic field-responsive composites include any polymer described herein, e.g., high molecular weight polyacrylic acid, polyethylene glycol, poly(2-vinyl-N-methylpyridinium iodide), polystyrene, polyethyleneimine, and block copolymers of polystyrene, such as poly(styrene-b-butadiene-b-styrene). Exemplary magnetic field-sensitive polymers are found, e.g., in Dai et al., Chem. Soc. Rev. 39:4057-4066 (2010), which is hereby incorporated by reference in its entirety.

### Actuator-embedded materials

Actuator-embedded materials can include one or more micro electro-mechanical systems actuators (or MEMS actuators) to change their physical properties upon exposure to one or more stimuli. Such actuator-embedded materials can result in mechanical, hydraulic, and/or pneumatic control of compression and/or expansion of the device. In some embodiments, the actuator-embedded materials include one or more actuators in combination with one or more polymers (e.g., any described herein, including polyvinylidenedifluoride, polyimide, polyester, rayon, epoxy, or combinations thereof).

Exemplary actuators includes those made from one or more carbon nanotubes (e.g., single-walled carbon nanotube composites having a piezoelectric effect); one or more piezoceramic actuators (e.g., including lead magnesium niobate (PMN), and optionally having one or more interdigitated electrodes, or one or more Pb(ZrₓTi₁₋ₓ)O₃ (PZT) materials (e.g., Ceramic B, PZT-2, PZT-4, PZT-5H, PZT-5A, PZT-4S, or PZT-8M, available from MTC Electro Ceramics, Berkshire, England)); one or more multilayered actuators (e.g., a PZT-based actuator, such as RAINBOW (Reduced And Internally Biased Oxide Wafer); a thin-layered piezoelectric composite material, such as THUNDER (Thin Layer Composite Unimorph ferroelectric DrivER and sensor); a laminate material including piezofibers, interdigitated electrodes, and a polymer (e.g., PVDF or polyimide, such as a Kapton® film), such as an AFC (Active Fiber Composite) developed by MIT University, USA; a macro-fiber composite including uniaxially aligned piezofibers in a polymer matrix, such as LaRC-MFC™ (NASA-Langley Research Center Macro-Fiber Composite); or a composite actuator including a carbon fiber composite layer with near-zero coefficient of thermal expansion (CTE), a PZT ceramic wafer, and a glass/epoxy layer, such as LIPCA (Lightweight Piezo-Composite Actuator)); one or more optical fibers (e.g., quartz-type and single-mode optical fibers, optionally embedded in an epoxy matrix); one or more piezopolymeric films; one or more piezoplates (e.g., a lead zirconate titanate plate that is optionally nickel-plated, e.g., PSI-5A4E or PSI-5H4E, available from Piezo Systems, Inc., Woburn, MA); one or more piezofibers (e.g., one or more carbon fibers and/or glass fibers, as well as composites thereof); one or more shape-memory polymers (e.g., any described herein); or one or more shape-memory alloys (e.g., any described herein, such as a NiTi alloy).

Exemplary actuator-embedded materials include carbon nanotubes in combination with polyvinylidenedifluoride (PVDF, optionally as a melt-blended or electrospun composite); carbon nanotubes in combination with polyimide (PI, optionally as a melt-blended or electrospun composite); unidirectional carbon fiber pre-impregnated sheets, such as XN-50A-RS3C, available from TenCate Corp., Nijverdal, Netherlands; Terfenol-D®, a magnetorestrictive material having terbium, iron, and dysprosium (available from Etrema Products Inc., Ames, IA); a thermally actuated composite in combination with a microelectronic substrate, such as those described in U.S. Pat. No. 6,211,598, which is hereby incorporated by reference in its entirety; a composite material including a nickel-tin shape-memory alloy (e.g., Nitinol™) in a thin film; or a magnetorestrictive composite including layers of Tb-Fe, polyimide, and Sm-Fe. Further exemplary materials are provided in U.S. Pat. No. 6,211,598 and International Pub. Nos. WO 2007/024038, each of which is incorporated by reference in its entirety.

### Pre-stretched and unstretched materials

The microclosures of the invention can include one or more pre-stretched and/or unstretched materials. Such pre-stretched materials include those having one or more stretchable polymers that are stretched prior to affixing to a skin region. Such unstretched materials include those having sufficient rigidity to hinder stretching and those having one or more stretchable polymers that are not stretched prior to affixing to a skin region. Exemplary pre-stretched and unstretched materials include Tegaderm™, available from 3M, St. Paul, MN, which can optionally be stretched after affixing to a skin region.

Unstretched materials have not been dimensionally altered and are in a stable dimensional state. Conversely, a stretched or pre-stretched material has an unstable dimensional state because the material has been dimensionally altered within the material's elastic region by a force. An unstretched material can also be highly rigid or cross-linked (e.g., highly resistant to stretching). Alternatively, an unstretched material can be a naive material, which can be stretched in subsequent use.

Exemplary pre-stretched and unstretched materials include any polymer or material described herein, a conventional material(s) (e.g., as described herein), permanent adhesive(s), highly cross-linked polymeric material(s), material(s) with high rigidity or hardness and low ductility (e.g., carboxymethylcellulose, gelatin, pectin, alginate, polyurethane, polymethacrylate, polyvinylpyrrolidone, nylon, polyethylene, polyacrylate, collagen, silicone, polyglycolic acid/polylactic acid, polyglycolic acid, polyglactin, benzyl hyaluronate, or combinations thereof, in any useful form, such as a film, bandage, gel, or hydrogel), and bioerodable, unstable materials that degrade spontaneously or in reaction to a treatment (e.g., such as any resorbable or biodegradable material, including any described herein).

### Adhesive materials

An adhesive can be used as the device (e.g., as the microclosure itself), as a portion of a device (e.g., within a microdressing, such as in the adhesive layer), or used in combination with any method described herein to promote skin treatment.

The adhesive can be a pressure-sensitive adhesive (PSA). The properties of pressure sensitive adhesives are governed by three parameters, tack (initial adhesion), peel strength (adhesion), and shear strength (cohesion). Pressure-sensitive adhesives can be synthesized in several ways, including solvent-borne, water-borne, and hot-melt methods. Tack is the initial adhesion under slight pressure and short dwell time and depends on the adhesive's ability to wet the contact surface. Peel strength is the force required to remove the PSA from the contact surface. The peel adhesion depends on many factors, including the tack, bonding history (e.g. force, dwell time), and adhesive composition. Shear strength is a measure of the adhesive's resistance to continuous stress. The shear strength is influenced by several parameters, including internal adhesion, cross-linking, and viscoelastic properties of the adhesive. Permanent adhesives are generally resistant to debonding and possess very high peel and shear strength.

Exemplary adhesives include a biocompatible matrix (e.g., those including at least one of collagen (e.g., a collagen sponge), low melting agarose (LMA), polylactic acid (PLA), and/or hyaluronic acid (e.g., hyaluranon); a photosensitizer (e.g., Rose Bengal, riboflavin-5-phosphate (R-5-P), methylene blue (MB), N-hydroxypyridine-2-(1 H)-thione (N-HTP), a porphyrin, or a chlorin, as well as precursors thereof); a photochemical agent (e.g., 1,8 naphthalimide); a synthetic glue (e.g., a cyanoacrylate adhesive, a polyethylene glycol adhesive, or a gelatin-resorcinol-formaldehyde adhesive); or a biologic sealant (e.g., a mixture of riboflavin-5-phosphate and fibrinogen, a fibrin-based sealant, an albumin-based sealant, or a starch-based sealant). In particular embodiments, the adhesive is biodegradable.

Exemplary pressure-sensitive adhesives include natural rubber, synthetic rubber (e.g., styrenebutadiene and styrene-ethylene copolymers), polyvinyl ether, polyurethane, acrylic, silicones, and ethylene-vinyl acetate copolymers. A copolymer's adhesive properties can be altered by varying the composition (via monomer components) changing the glass transition temperature (Tg) or degree of cross-linking. In general, a copolymer with a lower Tg is less rigid and a copolymer with a higher Tg is more rigid. The tack of PSAs can be altered by the addition of components to alter the viscosity or mechanical properties. Exemplary pressure sensitive adhesives are described in Czech et al., "Pressure-Sensitive Adhesives for Medical Applications," in Wide Spectra of Quality Control, Dr. Isin Akyar (Ed., published by InTech), Chapter 17 (2011), which is hereby incorporated by reference in its entirety.

In one exemplary technique, a photosensitizer is applied to the tissue (e.g., Rose Bengal (RB) at concentration of less than 1.0% weight per volume in a buffer, e.g., phosphate buffered saline to form a skin tissue-RB complex), and then the tissue is irradiated with electromagnetic energy to produce a seal (e.g., irradiated at a wavelength of at least 488, at less than 2000 J/cm², and/or at less than 1.5 W/cm², e.g., about 0.6 W/cm²). This exemplary technique is described in U.S. Pat. No. 7,073,510, which is incorporated by reference in its entirety. In another exemplary technique, a laser can be used for tissue welding. In yet another exemplary technique, a photochemical agent is applied to the tissue, and then the tissue is irradiated with visible light to produce a seal. In any of these embodiments, the technique includes use of a bioerodable, unstable material that degrades spontaneously or in reaction to a treatment (e.g., such as any resorbable or biodegradable material, including any described herein).

### Resorbable materials

The microclosures of the invention can include one or more resorbable materials (e.g., bioerodable, unstable materials that degrade spontaneously or in reaction to a treatment. Exemplary resorbable materials include bioresorbable (or bioabsorbable) metallic glass (e.g., Mg-Zn-Ca based metallic glasses, including Mg₆₆Zn₃₀Ca₄ and Mg₇₀Zn₂₅Ca₅; or Ca-Mg-Zn bulk metallic glasses, such as Ca₅₅Mg_{15+X}Zn_{30-X}, Ca₆₀Mg_{10+Y}Zn_{30-Y}, and Ca_{55+Z}Mg_{25-Z}Zn₂₀, where X = 0, 5 and 10; Y = 0, 5, 7.5, 10, and 15; and Z = 0, 5, 7.5, 10, and 15, including Ca₆₅Zn₂₀Mg₁₅); phosphate glass(e.g., P₂O₅-CaO-Na₂O-SiO₂, as described in Carta et al., J. Mater. Chem. 15:2134-2140 (2005), which is incorporated herein in its entirety); bioresorbable polymers, such as polyglycolic acid (PGA), polyglycolic acid/polylactic acid (PGA/PLA, including poly(L-lactide-co-glycolide) (PGA/LPLA) and poly(DL-lactide-co-glycolide) (PGA/DLPLA)), polyimide (PI), polylactic acid (PLA, including poly-L-lactide (PLLA), and poly(L-lactide-co-DL-lactide) (LPLA-DLPLA), and poly(DL-lactide) (DLPLA)), poly ε-caprolactone (PCL), poly(glycolide-co-trimethylene carbonate) (PGA-TMC), poly(dioxanone) (PDO), poly(glycolide-co-trimethylene carbonate-co-dioxanone) (PDO-PGA-TMC), expanded polystyrene (PS), poly(3-hydroxybutyrate) (PHB), polyorthoesters, poly[(carboxy phenoxy propane)-(sebacic acid) (PCPP-SA), poly[pyromellitylimidoalanine-co-1,6-bis(p-carboxyphenoxy) hexane], poly(sebacic acid) (PSA), poly(1-3 bis(p-carboxyphenoxy)propane) (PCPP), poly(1-6 bis(p-carboxy phenxoy)hexane) (PCPH), polypropylene fumarate, tyrosine derived polycarbonates (e.g., tyrosine derived polyarylates, tyrosine containing poly(DTR-PEG carbonate), and tyrosine containing poly(DTR-PEG ether)), poly(alkyl cyano acrylate), polyphosphazenes, polyphosphoesters, poly(aspartic acid), chondroton sulfate, hyaluronic acid (HA), chitosan, alginic acid (e.g., methylcellulose, hydroxypropyl methylcellulose, or hydroxyethyl cellulose alginic acid), cyanophycin, poly((ε-I-lysine), poly-γ-glutamic acid, glycosaminoglycans (e.g., dermatan sulfate, heparin sulfate, or keratin sulfate), a carboxymethylcellulose, a polyurethane, a siloxane, a polysiloxane, or poly(trimethylene carbonate) copolymers (TMC), including homopolymers and copolymers thereof; a biocompatible matrix (e.g., those including at least one of collagen (e.g., a collagen sponge or any described herein), low melting agarose (LMA), polylactic acid (PLA), hyaluronic acid (e.g., hyaluranon); a collagen (e.g., a Type I collagen, a Type II collagen, a Type III collagen, a Type IV collagen, a Type V collagen, a Type VI collagen, a Type VII collagen, or a Type VIII collagen); a glycosaminoglycan (e.g., hyaluronic acid, hyaluronate, chondroitin sulfate, heparin, dermatan sulfate, heparin sulfate, or keratin sulfate); a cellulose (e.g., oxidized regenerated cellulose (ORC) or an ORC:collagen composite; a composite (e.g., an alginate:collagen composite, or a granulated collagen-glycosaminoglycan composite); a polysaccharide (e.g., guar gum, xantham gum, gelatin, chitin, chitosan, chitosan acetate, chitosan lactate, chondroitin sulfate, N,O-carboxymethyl chitosan, cellulose, or a dextran); a biologic sealant (e.g., a mixture of riboflavin-5-phosphate and fibrinogen, a fibrin-based sealant, an albumin-based sealant, a collagen-based sealant, a keratin-based sealant, an alginate-based sealant, a chitin-based sealant, a proteoglycan-based sealant, a gelatin-based sealant, or a starch-based sealant); a biodegradable adhesive (e.g., poly(lactic acid) (PLA), poly(glycolic acid) (PGA), poly(lactic-co-glycolic acid) (PGA/PLA), a polyester, a polyanhydride, a polyphosphazene, a polyacrylate, or a polymethacrylate); or a tissue glue composed of a mixture of riboflavin-5-phosphate and fibrinogen, as well as any adhesive described herein.

### Therapeutic agents

The microclosures and methods of the invention can include one or more useful therapeutic agents.

Exemplary agents include one or more growth factors (e.g., vascular endothelial growth factor (VEGF), platelet-derived growth factor (PDGF), transforming growth factor beta (TGF-β), fibroblast growth factor (FGF), epidermal growth factor (EGF), and keratinocyte growth factor); one or more stem cells (e.g., adipose tissue-derived stem cells and/or bone marrow-derived mesenchymal stem cells); one or more analgesics (e.g., paracetamol/acetaminophen, aspirin, a non-steroidal antiinflammatory drug, as described herein, a cyclooxygenase-2-specific inhibitor, as described herein, dextropropoxyphene, co-codamol, an opioid (e.g., morphine, codeine, oxycodone, hydrocodone, dihydromorphine, pethidine, buprenorphine, tramadol, or methadone), fentanyl, procaine, lidocaine, tetracaine, dibucaine, benzocaine, p-butylaminobenzoic acid 2-(diethylamino) ethyl ester HCI, mepivacaine, piperocaine, dyclonine, or venlafaxine); one or more antibiotics (e.g., cephalosporin, bactitracin, polymyxin B sulfate, neomycin, bismuth tribromophenate, or polysporin); one or more antifungals (e.g., nystatin); one or more antiinflammatory agents (e.g., a non-steroidal antiinflammatory drug (NSAID, e.g., ibuprofen, ketoprofen, flurbiprofen, piroxicam, indomethacin, diclofenac, sulindac, naproxen, aspirin, ketorolac, or tacrolimus), a cyclooxygenase-2-specific inhibitor (COX-2 inhibitor, e.g., rofecoxib (Vioxx®), etoricoxib, and celecoxib (Celebrex®)), a glucocorticoid agent, a specific cytokine directed at T lymphocyte function), a steroid (e.g., a corticosteroid, such as a glucocorticoid (e.g., aldosterone, beclometasone, betamethasone, cortisone, deoxycorticosterone acetate, dexamethasone, fludrocortisone acetate, hydrocortisone, methylprednisolone, prednisone, prednisolone, or triamcinolone) or a mineralocorticoid agent (e.g., aldosterone, corticosterone, or deoxycorticosterone)), or an immune selective antiinflammatory derivative (e.g., phenylalanine-glutamine-glycine (FEG) and its D-isomeric form (feG))); one or more antimicrobials (e.g., chlorhexidine gluconate, iodine (e.g., tincture of iodine, povidone-iodine, or Lugol's iodine), or silver, such as silver nitrate (e.g., as a 0.5% solution), silver sulfadiazine (e.g., as a cream), or Ag⁺ in one or more useful carriers (e.g., an alginate, such as Acticoat® including nanocrystalline silver coating in high density polyethylene, available from Smith & Nephew, London, U.K., or Silvercel® including a mixture of alginate, carboxymethylcellulose, and silver coated nylon fibers, available from Systagenix, Gatwick, U.K.; a foam (e.g., Contreet® Foam including a soft hydrophilic polyurethane foam and silver, available from Coloplast A/S, Humlebæk, Denmark); a hydrocolloid (e.g., Aquacel® Ag including ionic silver and a hydrocolloid, available from Conva Tec Inc., Skillman, NJ); or a hydrogel (e.g., Silvasorb® including ionic silver, available from Medline Industries Inc., Mansfield, MA)); one or more antiseptics (e.g., an alcohol, such as ethanol (e.g., 60-90%), 1-propanol (e.g., 60-70%), as well as mixtures of 2-propanol/isopropanol; boric acid; calcium hypochlorite; hydrogen peroxide; manuka honey and/or methylglyoxal; a phenol (carbolic acid) compound, e.g., sodium 3,5-dibromo-4-hydroxybenzene sulfonate, trichlorophenylmethyl iodosalicyl, or triclosan; a polyhexanide compound, e.g., polyhexamethylene biguanide (PHMB); a quaternary ammonium compound, such as benzalkonium chloride (BAC), benzethonium chloride (BZT), cetyl trimethylammonium bromide (CTMB), cetylpyridinium chloride (CPC), chlorhexidine (e.g., chlorhexidine gluconate), or octenidine (e.g., octenidine dihydrochloride); sodium bicarbonate; sodium chloride; sodium hypochlorite (e.g., optionally in combination with boric acid in Dakin's solution); or a triarylmethane dye (e.g., Brilliant Green)); one or more antiproliferative agents (e.g., sirolimus, tacrolimus, zotarolimus, biolimus, or paclitaxel); one or more emollients; one or more hemostatic agents (e.g., collagen, such as microfibrillar collagen, chitosan, calcium-loaded zeolite, cellulose, anhydrous aluminum sulfate, silver nitrate, potassium alum, titanium oxide, fibrinogen, epinephrine, calcium alginate, poly-N-acetyl glucosamine, thrombin, coagulation factor(s) (e.g., II, V, VII, VIII, IX, X, XI, XIII, or Von Willebrand factor, as well as activated forms thereof), a procoagulant (e.g., propyl gallate), an anti-fibrinolytic agent (e.g., epsilon aminocaproic acid or tranexamic acid), and the like); one or more procoagulative agents (e.g., any hemostatic agent described herein, desmopressin, coagulation factor(s) (e.g., II, V, VII, VIII, IX, X, XI, XIII, or Von Willebrand factor, as well as activated forms thereof), procoagulants (e.g., propyl gallate), antifibrinolytics (e.g., epsilon aminocaproic acid), and the like); one or more anticoagulative agents (e.g., heparin or derivatives thereof, such as low molecular weight heparin, fondaparinux, or idraparinux; an anti-platelet agent, such as aspirin, dipyridamole, ticlopidine, clopidogrel, or prasugrel; a factor Xa inhibitor, such as a direct factor Xa inhibitor, e.g., apixaban or rivaroxaban; a thrombin inhibitor, such as a direct thrombin inhibitor, e.g., argatroban, bivalirudin, dabigatran, hirudin, lepirudin, or ximelagatran; or a coumarin derivative or vitamin K antagonist, such as warfarin (coumadin), acenocoumarol, atromentin, phenindione, or phenprocoumon); one or more immune modulators, including corticosteroids and non-steroidal immune modulators (e.g., NSAIDS, such as any described herein); one or more proteins; one or skin pigmentation modification compounds (e.g., a bleaching or lightening compound , e.g., hydroquinone, or tyrosinase inhibitor (e.g., Kojic acid or any of those inhibitors described in Chang, Int J Mol Sci. Jun 2009; 10(6): 2440-2475, which is incorporated by reference in its entirety), or one or more vitamins (e.g., vitamin A, C, and/or E).

For the skin tightening methods described herein, the use of anticoagulative and/or procoagulative agents may be of particular relevance. For instance, by controlling the extent of bleeding and/or clotting in the incisions and/or excisions, the skin tightening effect can be more effectively controlled. Thus, in some embodiments, the methods and devices herein include one or more anticoagulative agents, one or more procoagulative agents, one or more hemostatic agents, or combinations thereof. In particular embodiments, the therapeutic agent controls the extent of bleeding and/or clotting in the treated skin region, including the use one or more anticoagulative agents (e.g., to inhibit clot formation prior to skin healing or slit/hole closure) and/or one or more hemostatic or procoagulative agents.

### Applicators, including removers

The device, apparatus, and methods of the invention can include one or more microclosures in combination with one or more applicators. Such applicators can be useful for depositing and/or removing one or more microclosures (e.g., including arrays of microclosures), as well as forming one or more microwounds and/or providing a second compressive force (i.e., a compressive force other than the force that may be exerted by the microclosure itself). These functionalities can be present in a single applicator. Alternatively, each applicator or apparatus can perform a particular function, such as one applicator to deposit microclosures, another applicator to remove microclosures (e.g., a remover), as well as another applicator to form microwounds (e.g., an apparatus described herein, such as a microablation tool).

The applicator can include one or more components to facilitate deposition of a microclosure. In some embodiments, the component includes a needle or a pin (e.g., a central pin) adapted to releasably attach a microclosure. In use, the component is inserted into or near a microwound, and the microclosure is detached from the component and deposited into or onto the microwound. In particular embodiments, the needle can include a co-axial holder configured to reversibly attach a microclosure and/or to reversibly stretch or constrain the microclosure. For instance, if the microclosure is a microstaple, then the co-axial holder can maintain the microstaple in a constrained state, which then results in closure of the microstaple when deposited on or within the microwound. In another instance, if the microclosure is a microdressing, then the co-axial holder can maintain the microdressing in a pre-stretched state, which then results in the microdressing exerting a first compressive force when deposited on or within the microwound. Exemplary, non-limiting co-axial holders are described in Figures 3A-3C, 8A-8C, and 9.

In other embodiments, the applicator includes a dispenser for dispensing an aliquot of a sealant. In particular, the dispenser is configured to dispense a volume of sealant sufficient to close a microwound (e.g., a volumetric dimension that is less than or equal to about 6 mm³, 5.75 mm³, 5 mm³, 5.25 mm³, 4.75 mm³, 4.5 mm³, 4.25 mm³, 4 mm³, 3.75 mm³, 3.5 mm³, 3.25 mm³, 3 mm³, 2.75 mm³, 2.5 mm³, 2.25 mm³, 2 mm³, 1.75 mm³, 1.5 mm³, 1.25 mm³, 1 mm³, 0.9 mm³, 0.8 mm³, 0.7 mm³, 0.6 mm³, 0.5 mm³, 0.4 mm³, 0.3 mm³, 0.2 mm³, 0.1 mm³, 0.07 mm³, 0.05 mm³, 0.03 mm³, 0.02 mm³, 0.01 mm³, 0.007 mm³, 0.005 mm³, 0.003 mm³, 0.002 mm³, or 0.001 mm³ and including any ranges described herein). In some embodiments, the dispenser is adapted to dispense a sealant within or onto a microwound. Further, the dispenser can be configured to exert a second compressive force before, during, or after the deposition of the sealant. Exemplary, non-limiting dispensers are described in Figure 11.

The applicator can include one or more components for forming a microwound. For instance, the applicator can include a needle (e.g., any described herein) and a pin within the lumen of the needle, where the microclosure is releasably attached to the pin or the needle. In one non-limiting example, the applicator includes a needle and a pin, where the microclosure is releasably attached to the pin. In use, the needle forms a microwound in the skin, and the pin is inserted into the wound. If the microclosure is a microstaple, then the microstaple is released from the pin and deposited within the microwound.

In another non-limiting example, the applicator includes a needle and a centering pin, where the microclosure is releasably attached to the needle. In particular, this applicator can be used to deposit the microclosure on the surface of the skin and allows for centering of the microclosure relative to the microwound. In use, the needle forms a microwound in the skin, and the centering pin is inserted into the wound. Then, the microclosure is centered relative to the microwound, released from the needle, and deposited onto the microwound.

Exemplary, non-limiting applicators are provided in Figures 2A-2C, 3A-3C, 8A-8D, 9, and 11.

The device, apparatus, and methods of the invention can further include one or more components to remove the microclosure or array of such microclosures. Such components include an apparatus, a chemical agent (e.g., that dissolves, inactivates, or releases the microclosure), a biological agent (e.g., that dissolves, inactivates, or releases the microclosure), a polymeric material, an abrasive material (e.g., that abrades the microclosure and/or treated skin region), a macrodressing (e.g., adapted to attach to the microclosure or array, or a tunable dressing, as described herein), an adhesive material (e.g., an adhesive, such as any described herein, on a substrate), and a mechanical lifting device (e.g., which attaches to the attachment component of the microclosure), as described herein.

### Kits, optionally including one or more applicators

Also described herein are kits for skin tightening or for treating diseases, disorders, and conditions that would benefit from skin restoration or tightening. Accordingly, the present invention includes kits having one or more devices in combination with one or more applicators, as well kits having a combination of two or more devices, where at least one device is a microclosure as described herein.

The kit includes a device, such as any microclosure described herein, and any other useful component. In some embodiments, the kit includes a device (e.g., a microclosure) and an applicator. The applicator can include a frame or any structure configured to affix a device to the skin region, where the frame or structure is optionally disposable. In general, each device or microclosure is configured to be affixed to a skin region, and the applicator can be configured to assist in the affixation of such a device. In some embodiments, the applicator maintains the device in an unstretched state to allow for affixing a device having an unstretched layer. In some embodiments, the applicator maintains the device in a pre-stretched or pre-constrained state to allow for affixing a device having a pre-stretched layer or a pre-constrained microclosure. In other embodiments, the applicator holds the device to allow for aligning, positioning, and/or placing the device on the desired skin region. In yet other embodiments, the applicator is configured to allow for affixing a microclosure immediately after or shortly after forming one or more incisions or excisions in the skin region. In such an embodiment, the applicator is configured to releasably attach to an apparatus for making such an incision or excision (e.g., an apparatus including one or more blades and/or one or more tubes or a microablation tool, such as any described herein).

The applicator can be of any useful shape and/or material (e.g., any material or polymer described herein). In some embodiments, the applicator is a frame that provides sufficient support to the device or microclosure and/or provides a sterile method to affix the device or microclosure. In particular embodiments, the frame includes a rigid plate having one or more view ports (e.g., one or more transparent windows) to allow for positioning of the device. In some embodiments, the frame is structurally configured to attach to an apparatus for making one or more incisions and/or excisions and to release a device (e.g., a microclosure) after making such an incision or excision.

In other embodiments, the applicator includes a liner layer having one or more handles, where the liner layer is attached to the proximal surface of a microclosure. The handles allow for positioning the microclosure over the treated skin region. In some embodiments, the handles are configured to be detached from the microclosure immediately prior to or after affixation. In some embodiments, the applicator includes a releasing layer. Exemplary applicators are provided in U.S. Pub. Nos. 2012/0226306 and 2012/0226214, where each is hereby incorporated by reference in its entirety.

There may be a plurality of devices (e.g., microclosures, such as in an array) in a kit. Within the kit, the microclosure may be packaged individually (e.g., in sets of two or more). In some embodiments, each microclosure includes an applicator, where the dressing and the applicator are configured together in one package. In other embodiments, the kit includes one or more microclosure (e.g., in an array) in combination with one or more applicators, where each of the microclosure(s), arrays(s), and/or applicator(s) is individually packaged. The microclosure(s), arrays(s), and/or applicator(s) are packaged such that they remain sterile until use. In certain embodiments, microclosure(s), arrays(s),, and/or applicator(s) are packaged in plastic sheaths. Further, to prevent contamination of the skin region, microclosure(s), arrays(s), and/or applicator(s) are preferably provided for as disposable and/or single-use items.

The kit can include a microclosure in combination with any other device or apparatus described herein (e.g., a device or apparatus for forming one or more incisions or excisions in a skin region). In some embodiments, the other device or apparatus includes one or more blades and/or one or more needles. In other embodiments, the other device or apparatus includes a microablation tool. Exemplary microablation tools include a fractional laser microablation tool, a fractional radiofrequency microablation tool, or a fractional ultrasonic microablation tool.

The kit can include any other useful components. Exemplary components include instructions on how to use the device(s), an air blower, a heat gun, a heating pad, one or more therapeutic agents (e.g., any described herein, such as an anticoagulative and/or procoagulative agent, and optionally in combination with a useful dispenser for applying the therapeutic agent, such as a brush, spray, film, ointment, cream, lotion, or gel), one or more wound cleansers (e.g., including any antibiotic, antimicrobial, or antiseptic, such as those described herein, in any useful form, such as a brush, spray, film, ointment, cream, lotion, or gel), one or more debriding agents, and/or other suitable or useful materials.

### Methods for treating skin regions

The present invention relates to methods and devices that can be applied to treated skin regions. In particular embodiments, these regions are treated with one or more procedures to improve skin appearance. Accordingly, the devices, microclosures, and methods herein can be useful for skin rejuvenation (e.g., removal of pigment, veins (e.g., spider veins or reticular veins), and/or vessels in the skin) or for treating acne, allodynia, blemishes, ectopic dermatitis, hyperpigmentation, hyperplasia (e.g., lentigo or keratosis), loss of translucency, loss of elasticity, melasma (e.g., epidermal, dermal, or mixed subtypes), photodamage, rashes (e.g., erythematous, macular, papular, and/or bullous conditions), psoriasis, rhytides (or wrinkles, e.g., crow's feet, age-related rhytides, sun-related rhytides, or heredity-related rhytides), sallow color, scar contracture (e.g., relaxation of scar tissue), scarring (e.g., due to acne, surgery, or other trauma), skin aging, skin contraction (e.g., excessive tension in the skin), skin irritation/sensitivity, skin laxity (e.g., loose or sagging skin or other skin irregularities), striae (or stretch marks), vascular lesions (e.g., angioma, erythema, hemangioma, papule, port wine stain, rosacea, reticular vein, or telangiectasia), or any other unwanted skin irregularities.

Such treatments can be include any parts of the body, including the face (e.g., eyelid, cheeks, chin, forehead, lips, or nose), neck, chest (e.g., as in a breast lift), arms, legs, and/or back. Accordingly, the devices on the invention can be arranged or configured to be amenable to the size or geometry of different body regions. Such arrangements and configurations can include any useful shape (e.g., linear, curved, or stellate), size, and/or depth.

In general, the treatment methods includes forming a plurality of microwounds (e.g., as described herein) and applying one or more microclosures to the microwounds. In particular, the microclosure can exert a first compressive force (e.g., thereby treating skin). In other embodiments, the treatment (e.g,. tightening) method includes the steps of (1) forming one or more microwounds, (2) applying a compressive force (e.g., a first and/or second compressive force), (3) applying one or more microclosures, where steps (2) and (3) can occur in any order, (4) titrating the tightening effect (e.g., by removing a portion of a plurality of microclosures or adjusting their tightening effect), and (5) removing one or more microclosures after wound healing. A device may be sued to accomplish a combination of steps. For example, a device according to the invention may facilitate wound formation, compression, and application of microclosures.

These methods can further include applying a second compressive force to the treated skin region. Additional compressive forces (e.g., a second, third, fourth, etc. compressive force) can be applied after the deposition of one or more microclosures. Such additional compressive forces may be helpful in further treating skin, such as any useful endpoint described herein (e.g., reducing tissue volume or area, promoting beneficial tissue growth, tightening skin, rejuvenating skin, improving skin texture or appearance, removing skin laxity, and/or expanding tissue volume or area). These methods can also include removing the microclosure or array (e.g., by using a remover, as described herein). In particular, such removal may be beneficial to control the timing of the skin treatment and/or to remove devices or components that are non-resorbable.

In one exemplary procedure, a plurality of tissue portions are incised into or excised from a skin region in a subject (e.g., about 2, 3, 4, 5, 6, 7, 8, 9, 10, 15, 20, 25, 30, 35, 40, 45, 50, 75, 100, or more tissue portions, such as between about 2 and 100 tissue portions (e.g., between 2 and 10, 2 and 15, 2 and 20, 2 and 25, 2 and 30, 2 and 35, 2 and 40, 2 and 45, 2 and 50, 2 and 75, 5 and 10, 5 and 15, 5 and 20, 5 and 25, 5 and 30, 5 and 35, 5 and 40, 5 and 45, 5 and 50, 5 and 75, 5 and 100, 10 and 20, 10 and 25, 10 and 30, 10 and 35, 10 and 40, 10 and 45, 10 and 50, 10 and 75, 10 and 100, 15 and 20, 15 and 25, 15 and 30, 15 and 35, 15 and 40, 15 and 45, 15 and 50, 15 and 75, 15 and 100, 20 and 25, 20 and 30, 20 and 35, 20 and 40, 20 and 45, 20 and 50, 20 and 75, 20 and 100, 25 and 30, 25 and 35, 25 and 40, 25 and 45, 25 and 50, 25 and 75, 25 and 100, 30 and 35, 30 and 40, 30 and 45, 30 and 50, 30 and 75, 30 and 100, 35 and 40, 35 and 45, 35 and 50, 35 and 75, 35 and 100, 40 and 45, 40 and 50, 40 and 75, 40 and 100, 50 and 75, or 50 and 100)). Such tissue portions can be included in any useful geometric, non-geometric, or random array (e.g., such as those described herein for an array of tubes and/or blades). Such tissue portions can have any useful dimension that promotes wound or skin healing. Non-limiting dimensions of a tissue portion includes at least one dimension that is less than about 2.0 mm (e.g., less than or equal to about 1.5 mm, 1 mm, 0.75 mm, 0.5 mm, 0.3 mm, 0.2 mm, 0.1 mm, 0.075 mm, 0.05 mm, or 0.025 mm) or between about 0.025 mm and 2.0 mm (e.g., between about 0.025 mm and 1.5 mm, 0.025 mm and 1.0 mm, 0.025 mm and 0.75 mm, 0.025 mm and 0.5 mm, 0.025 mm and 0.3 mm, 0.025 mm and 0.2 mm, 0.025 mm and 0.1 mm, 0.025 mm and 0.075 mm, 0.025 mm and 0.05 mm, 0.05 mm and 2.0 mm, 0.05 mm and 1.5 mm, 0.05 mm and 1.0 mm, 0.05 mm and 0.75 mm, 0.05 mm and 0.5 mm, 0.05 mm and 0.3 mm, 0.05 mm and 0.2 mm, 0.05 mm and 0.1 mm, 0.05 mm and 0.075 mm, 0.075 mm and 2.0 mm, 0.075 mm and 1.5 mm, 0.075 mm and 1.0 mm, 0.075 mm and 0.75 mm, 0.075 mm and 0.5 mm, 0.075 mm and 0.3 mm, 0.075 mm and 0.2 mm, 0.075 mm and 0.1 mm, 0.1 mm and 2.0 mm, 0.1 mm and 1.5 mm, 0.1 mm and 1.0 mm, 0.1 mm and 0.75 mm, 0.1 mm and 0.5 mm, 0.1 mm and 0.3 mm, 0.1 mm and 0.2 mm, 0.2 mm and 2.0 mm, 0.2 mm and 1.5 mm, 0.2 mm and 1.0 mm, 0.2 mm and 0.75 mm, 0.2 mm and 0.5 mm, 0.2 mm and 0.3 mm, 0.3 mm and 2.0 mm, 0.3 mm and 1.5 mm, 0.3 mm and 1.0 mm, 0.3 mm and 0.75 mm, 0.3 mm and 0.5 mm, 0.5 mm and 2.0 mm, 0.5 mm and 1.5 mm, 0.5 mm and 1.0 mm, 0.5 mm and 0.75 mm, 0.75 mm and 2.0 mm, 0.75 mm and 1.5 mm, or 0.75 mm and 1.0 mm).

In some embodiments, the incised or excised tissue portions forms a hole in the skin region, where the diameter or width of the hole is less than about 1.0 mm and results in a tissue portion having a diameter or width that is less than about 1.0 mm. In further embodiments, the tissue portion has a diameter or width that is less than about 1.0 mm and a length of more than about 1.0 mm (e.g., about 1.0 mm, 1.5 mm, 2.0 mm. 2.5 mm, 3.0 mm, or 3.5 mm). In particular embodiments, relatively small dimensions of the tissue portions can promote healing while minimizing the formation of scars.

In other embodiments, the incised or excised tissue portions forms a slit in the skin region, where the length or width of the slit is less than about 1.0 mm and results in a tissue portion having a length or width that is less than about 1.0 mm. In further embodiments, the tissue portion has a length or width that is less than about 1.0 mm and a length of more than about 1.0 mm (e.g., about 1.0 mm, 1.5 mm, 2.0 mm. 2.5 mm, 3.0 mm, or 3.5 mm). In particular embodiments, relatively small dimensions of the tissue portions can promote healing while minimizing the formation of scars.

The tissue portion can be of any useful shape. Exemplary shapes include cylinders (i.e., thereby forming round or elongated holes in the skin region), holes (e.g., microholes), slits (e.g., microslits), elongated strips (i.e., thereby forming elongated openings in the skin region), or other geometries including at least dimension that is less than about 1.0 mm (e.g., less than or equal to about 0.75 mm, about 0.5 mm, about 0.3 mm, about 0.2 mm, about 0.1 mm, or about 0.05 mm) or between about 0.05 mm and 1.0 mm (e.g., 0.05 mm and 0.75 mm, 0.05 mm and 0.5 mm, 0.05 mm and 0.3 mm, 0.05 mm and 0.2 mm, 0.05 mm and 0.1 mm, 0.1 mm and 1.0 mm, 0.1 mm and 0.75 mm, 0.1 mm and 0.5 mm, 0.1 mm and 0.3 mm, 0.1 mm and 0.2 mm, 0.2 mm and 1.0 mm, 0.2 mm and 0.75 mm, 0.2 mm and 0.5 mm, 0.2 mm and 0.3 mm, 0.3 mm and 1.0 mm, 0.3 mm and 0.75 mm, 0.3 mm and 0.5 mm, 0.4 mm and 1.0 mm, 0.4 mm and 0.75 mm, 0.4 mm and 0.5 mm, 0.5 mm and 1.0 mm, 0.5 mm and 0.75 mm, 0.6 mm and 1.0 mm, 0.6 mm and 0.75 mm, or 0.75 mm and 1.0 mm). In other embodiments, the incised tissue portion and/or excised tissue portion has an areal dimension (e.g., a cross-sectional dimension in the xy-plane, such as an areal dimension of a circle or non-circular (e.g., elliptical) shape) of less than about or equal to about 1.0 mm² (e.g., less than or equal to about 0.9 mm² , 0.8 mm² , 0.7 mm² , 0.6 mm² , 0.5 mm² , 0.4 mm², 0.3 mm², 0.2 mm², 0.1 mm², 0.07 mm², 0.05 mm², 0.03 mm², 0.02 mm², 0.01 mm², 0.007 mm², 0.005 mm², 0.003 mm², 0.002 mm², or 0.001 mm²) or between about 0.001 mm² and 1.0 mm² (e.g., 0.001 mm² and 0.9 mm², 0.001 mm² and 0.8 mm², 0.001 mm² and 0.7 mm², 0.001 mm² and 0.6 mm², 0.001 mm² and 0.5 mm², 0.001 mm² and 0.4 mm², 0.001 mm² and 0.3 mm², 0.001 mm² and 0.2 mm², 0.001 mm² and 0.1 mm², 0.001 mm² and 0.07 mm², 0.001 mm² and 0.05 mm², 0.001 mm² and 0.03 mm², 0.001 mm² and 0.02 mm², 0.001 mm² and 0.01 mm², 0.001 mm² and 0.007 mm², 0.001 mm² and 0.005 mm², 0.001 mm² and 0.003 mm², 0.001 mm² and 0.002 mm², 0.002 mm² and 1.0 mm², 0.002 mm² and 0.9 mm², 0.002 mm² and 0.8 mm², 0.002 mm² and 0.7 mm², 0.002 mm² and 0.6 mm², 0.002 mm² and 0.5 mm², 0.002 mm² and 0.4 mm², 0.002 mm² and 0.3 mm², 0.002 mm² and 0.2 mm², 0.002 mm² and 0.1 mm², 0.002 mm² and 0.07 mm², 0.002 mm² and 0.05 mm², 0.002 mm² and 0.03 mm², 0.002 mm² and 0.02 mm², 0.002 mm² and 0.01 mm², 0.002 mm² and 0.007 mm², 0.002 mm² and 0.005 mm², 0.002 mm² and 0.003 mm², 0.005 mm² and 1.0 mm², 0.005 mm² and 0.9 mm², 0.005 mm² and 0.8 mm², 0.005 mm² and 0.7 mm², 0.005 mm² and 0.6 mm², 0.005 mm² and 0.5 mm², 0.005 mm² and 0.4 mm², 0.005 mm² and 0.3 mm², 0.005 mm² and 0.2 mm², 0.005 mm² and 0.1 mm², 0.005 mm² and 0.07 mm², 0.005 mm² and 0.05 mm², 0.005 mm² and 0.03 mm², 0.005 mm² and 0.02 mm², 0.005 mm² and 0.01 mm², 0.005 mm² and 0.007 mm², 0.007 mm² and 1.0 mm², 0.007 mm² and 0.9 mm², 0.007 mm² and 0.8 mm², 0.007 mm² and 0.7 mm², 0.007 mm² and 0.6 mm², 0.007 mm² and 0.5 mm², 0.007 mm² and 0.4 mm², 0.007 mm² and 0.3 mm², 0.007 mm² and 0.2 mm², 0.007 mm² and 0.1 mm², 0.007 mm² and 0.07 mm², 0.007 mm² and 0.05 mm², 0.007 mm² and 0.03 mm², 0.007 mm² and 0.02 mm², 0.007 mm² and 0.01 mm², 0.01 mm² and 1.0 mm², 0.01 mm² and 0.9 mm², 0.01 mm² and 0.8 mm², 0.01 mm² and 0.7 mm², 0.01 mm² and 0.6 mm², 0.01 mm² and 0.5 mm², 0.01 mm² and 0.4 mm², 0.01 mm² and 0.3 mm², 0.01 mm² and 0.2 mm², 0.01 mm² and 0.1 mm², 0.01 mm² and 0.07 mm², 0.01 mm² and 0.05 mm², 0.01 mm² and 0.03 mm², 0.01 mm² and 0.02 mm², 0.03 mm² and 1.0 mm², 0.03 mm² and 0.9 mm², 0.03 mm² and 0.8 mm², 0.03 mm² and 0.7 mm², 0.03 mm² and 0.6 mm², 0.03 mm² and 0.5 mm², 0.03 mm² and 0.4 mm², 0.03 mm² and 0.3 mm², 0.03 mm² and 0.2 mm², 0.03 mm² and 0.1 mm², 0.03 mm² and 0.07 mm², 0.03 mm² and 0.05 mm², 0.07 mm² and 1.0 mm², 0.07 mm² and 0.9 mm², 0.07 mm² and 0.8 mm², 0.07 mm² and 0.7 mm², 0.07 mm² and 0.6 mm², 0.07 mm² and 0.5 mm², 0.07 mm² and 0.4 mm², 0.07 mm² and 0.3 mm², 0.07 mm² and 0.2 mm², 0.07 mm² and 0.1 mm², 0.1 mm² and 1.0 mm², 0.1 mm² and 0.9 mm², 0.1 mm² and 0.8 mm², 0.1 mm² and 0.7 mm², 0.1 mm² and 0.6 mm², 0.1 mm² and 0.5 mm², 0.1 mm² and 0.4 mm², 0.1 mm² and 0.3 mm², 0.1 mm² and 0.2 mm², 0.3 mm² and 1.0 mm², 0.3 mm² and 0.9 mm², 0.3 mm² and 0.8 mm², 0.3 mm² and 0.7 mm², 0.3 mm² and 0.6 mm², 0.3 mm² and 0.5 mm², 0.3 mm² and 0.4 mm², 0.5 mm² and 1.0 mm², 0.5 mm² and 0.9 mm², 0.5 mm² and 0.8 mm², 0.5 mm² and 0.7 mm², 0.5 mm² and 0.6 mm², 0.7 mm² and 1.0 mm², 0.7 mm² and 0.9 mm², or 0.7 mm² and 0.8 mm²). In some embodiments, the volumetric dimension is less than or equal to about 6 mm³ (e.g., less than or equal to about 5.75 mm³, 5 mm³, 5.25 mm³ , 4.75 mm³, 4.5 mm³, 4.25 mm³, 4 mm³, 3.75 mm³, 3.5 mm³, 3.25 mm³, 3 mm³, 2.75 mm³, 2.5 mm³, 2.25 mm³, 2 mm³, 1.75 mm³, 1.5 mm³, 1.25 mm³, 1 mm³, 0.9 mm³, 0.8 mm³, 0.7 mm³, 0.6 mm³, 0.5 mm³, 0.4 mm³, 0.3 mm³, 0.2 mm³, 0.1 mm³, 0.07 mm³, 0.05 mm³, 0.03 mm³, 0.02 mm³, 0.01 mm³, 0.007 mm³, 0.005 mm³, 0.003 mm³, 0.002 mm³, or 0.001 mm³) or between about 0.001 mm³ and 6 mm³ (e.g., 0.001 mm³ and 5.75 mm³, 0.001 mm³ and 5 mm³, 0.001 mm³ and 5.25 mm³, 0.001 mm³ and 4.75 mm³, 0.001 mm³ and 4.5 mm³, 0.001 mm³ and 4.25 mm³, 0.001 mm³ and 4 mm³, 0.001 mm³ and 3.75 mm³, 0.001 mm³ and 3.5 mm³, 0.001 mm³ and 3.25 mm³, 0.001 mm³ and 3 mm³, 0.001 mm³ and 2.75 mm³, 0.001 mm³ and 2.5 mm³, 0.001 mm³ and 2.25 mm³, 0.001 mm³ and 2 mm³, 0.001 mm³ and 1.75 mm³, 0.001 mm³ and 1.5 mm³, 0.001 mm³ and 1.25 mm³, 0.001 mm³ and 1 mm³, 0.001 mm³ and 0.9 mm³, 0.001 mm³ and 0.8 mm³, 0.001 mm³ and 0.7 mm³, 0.001 mm³ and 0.6 mm³, 0.001 mm³ and 0.5 mm³, 0.001 mm³ and 0.4 mm³, 0.001 mm³ and 0.3 mm³, 0.001 mm³ and 0.2 mm³, 0.001 mm³ and 0.1 mm³, 0.001 mm³ and 0.07 mm³, 0.001 mm³ and 0.05 mm³, 0.001 mm³ and 0.03 mm³, 0.001 mm³ and 0.02 mm³, 0.001 mm³ and 0.01 mm³, 0.001 mm³ and 0.007 mm³, 0.001 mm³ and 0.005 mm³, 0.001 mm³ and 0.003 mm³, 0.001 mm³ and 0.002 mm³, 0.003 mm³ and 6 mm³, 0.003 mm³ and 5.75 mm³, 0.003 mm³ and 5 mm³, 0.003 mm³ and 5.25 mm³, 0.003 mm³ and 4.75 mm³, 0.003 mm³ and 4.5 mm³, 0.003 mm³ and 4.25 mm³, 0.003 mm³ and 4 mm³, 0.003 mm³ and 3.75 mm³, 0.003 mm³ and 3.5 mm³, 0.003 mm³ and 3.25 mm³, 0.003 mm³ and 3 mm³, 0.003 mm³ and 2.75 mm³, 0.003 mm³ and 2.5 mm³, 0.003 mm³ and 2.25 mm³, 0.003 mm³ and 2 mm³, 0.003 mm³ and 1.75 mm³, 0.003 mm³ and 1.5 mm³, 0.003 mm³ and 1.25 mm³, 0.003 mm³ and 1 mm³, 0.003 mm³ and 0.9 mm³, 0.003 mm³ and 0.8 mm³, 0.003 mm³ and 0.7 mm³, 0.003 mm³ and 0.6 mm³, 0.003 mm³ and 0.5 mm³, 0.003 mm³ and 0.4 mm³, 0.003 mm³ and 0.3 mm³, 0.003 mm³ and 0.2 mm³, 0.003 mm³ and 0.1 mm³, 0.003 mm³ and 0.07 mm³, 0.003 mm³ and 0.05 mm³, 0.003 mm³ and 0.03 mm³, 0.003 mm³ and 0.02 mm³, 0.003 mm³ and 0.01 mm³, 0.003 mm³ and 0.007 mm³, 0.003 mm³ and 0.005 mm³, 0.005 mm³ and 6 mm³, 0.005 mm³ and 5.75 mm³, 0.005 mm³ and 5 mm³, 0.005 mm³ and 5.25 mm³, 0.005 mm³ and 4.75 mm³, 0.005 mm³ and 4.5 mm³, 0.005 mm³ and 4.25 mm³, 0.005 mm³ and 4 mm³, 0.005 mm³ and 3.75 mm³, 0.005 mm³ and 3.5 mm³, 0.005 mm³ and 3.25 mm³, 0.005 mm³ and 3 mm³, 0.005 mm³ and 2.75 mm³, 0.005 mm³ and 2.5 mm³, 0.005 mm³ and 2.25 mm³, 0.005 mm³ and 2 mm³, 0.005 mm³ and 1.75 mm³, 0.005 mm³ and 1.5 mm³, 0.005 mm³ and 1.25 mm³, 0.005 mm³ and 1 mm³, 0.005 mm³ and 0.9 mm³, 0.005 mm³ and 0.8 mm³, 0.005 mm³ and 0.7 mm³, 0.005 mm³ and 0.6 mm³, 0.005 mm³ and 0.5 mm³, 0.005 mm³ and 0.4 mm³, 0.005 mm³ and 0.3 mm³, 0.005 mm³ and 0.2 mm³, 0.005 mm³ and 0.1 mm³, 0.005 mm³ and 0.07 mm³, 0.005 mm³ and 0.05 mm³, 0.005 mm³ and 0.03 mm³, 0.005 mm³ and 0.02 mm³, 0.005 mm³ and 0.01 mm³, 0.005 mm³ and 0.007 mm³, 0.01 mm³ and 6 mm³, 0.01 mm³ and 5.75 mm³, 0.01 mm³ and 5 mm³, 0.01 mm³ and 5.25 mm³, 0.01 mm³ and 4.75 mm³, 0.01 mm³ and 4.5 mm³, 0.01 mm³ and 4.25 mm³, 0.01 mm³ and 4 mm³, 0.01 mm³ and 3.75 mm³, 0.01 mm³ and 3.5 mm³, 0.01 mm³ and 3.25 mm³, 0.01 mm³ and 3 mm³, 0.01 mm³ and 2.75 mm³, 0.01 mm³ and 2.5 mm³, 0.01 mm³ and 2.25 mm³, 0.01 mm³ and 2 mm³, 0.01 mm³ and 1.75 mm³, 0.01 mm³ and 1.5 mm³, 0.01 mm³ and 1.25 mm³, 0.01 mm³ and 1 mm³, 0.01 mm³ and 0.9 mm³, 0.01 mm³ and 0.8 mm³, 0.01 mm³ and 0.7 mm³, 0.01 mm³ and 0.6 mm³, 0.01 mm³ and 0.5 mm³, 0.01 mm³ and 0.4 mm³, 0.01 mm³ and 0.3 mm³, 0.01 mm³ and 0.2 mm³, 0.01 mm³ and 0.1 mm³, 0.01 mm³ and 0.07 mm³, 0.01 mm³ and 0.05 mm³, 0.01 mm³ and 0.03 mm³, 0.01 mm³ and 0.02 mm³, 0.05 mm³ and 6 mm³, 0.05 mm³ and 5.75 mm³, 0.05 mm³ and 5 mm³, 0.05 mm³ and 5.25 mm³, 0.05 mm³ and 4.75 mm³, 0.05 mm³ and 4.5 mm³, 0.05 mm³ and 4.25 mm³, 0.05 mm³ and 4 mm³, 0.05 mm³ and 3.75 mm³, 0.05 mm³ and 3.5 mm³, 0.05 mm³ and 3.25 mm³, 0.05 mm³ and 3 mm³, 0.05 mm³ and 2.75 mm³, 0.05 mm³ and 2.5 mm³, 0.05 mm³ and 2.25 mm³, 0.05 mm³ and 2 mm³, 0.05 mm³ and 1.75 mm³, 0.05 mm³ and 1.5 mm³, 0.05 mm³ and 1.25 mm³, 0.05 mm³ and 1 mm³, 0.05 mm³ and 0.9 mm³, 0.05 mm³ and 0.8 mm³, 0.05 mm³ and 0.7 mm³, 0.05 mm³ and 0.6 mm³, 0.05 mm³ and 0.5 mm³, 0.05 mm³ and 0.4 mm³, 0.05 mm³ and 0.3 mm³, 0.05 mm³ and 0.2 mm³, 0.05 mm³ and 0.1 mm³, 0.05 mm³ and 0.07 mm³, 0.1 mm³ and 6 mm³, 0.1 mm³ and 5.75 mm³, 0.1 mm³ and 5 mm³, 0.1 mm³ and 5.25 mm³, 0.1 mm³ and 4.75 mm³, 0.1 mm³ and 4.5 mm³, 0.1 mm³ and 4.25 mm³, 0.1 mm³ and 4 mm³, 0.1 mm³ and 3.75 mm³, 0.1 mm³ and 3.5 mm³, 0.1 mm³ and 3.25 mm³, 0.1 mm³ and 3 mm³, 0.1 mm³ and 2.75 mm³, 0.1 mm³ and 2.5 mm³, 0.1 mm³ and 2.25 mm³, 0.1 mm³ and 2 mm³, 0.1 mm³ and 1.75 mm³, 0.1 mm³ and 1.5 mm³, 0.1 mm³ and 1.25 mm³, 0.1 mm³ and 1 mm³, 0.1 mm³ and 0.9 mm³, 0.1 mm³ and 0.8 mm³, 0.1 mm³ and 0.7 mm³, 0.1 mm³ and 0.6 mm³, 0.1 mm³ and 0.5 mm³, 0.1 mm³ and 0.4 mm³, 0.1 mm³ and 0.3 mm³, 0.1 mm³ and 0.2 mm³, 0.5 mm³ and 6 mm³, 0.5 mm³ and 5.75 mm³, 0.5 mm³ and 5 mm³, 0.5 mm³ and 5.25 mm³, 0.5 mm³ and 4.75 mm³, 0.5 mm³ and 4.5 mm³, 0.5 mm³ and 4.25 mm³, 0.5 mm³ and 4 mm³, 0.5 mm³ and 3.75 mm³, 0.5 mm³ and 3.5 mm³, 0.5 mm³ and 3.25 mm³, 0.5 mm³ and 3 mm³, 0.5 mm³ and 2.75 mm³, 0.5 mm³ and 2.5 mm³, 0.5 mm³ and 2.25 mm³, 0.5 mm³ and 2 mm³, 0.5 mm³ and 1.75 mm³, 0.5 mm³ and 1.5 mm³, 0.5 mm³ and 1.25 mm³, 0.5 mm³ and 1 mm³, 0.5 mm³ and 0.9 mm³, 0.5 mm³ and 0.8 mm³, 0.5 mm³ and 0.7 mm³, 0.5 mm³ and 0.6 mm³, 1 mm³ and 6 mm³, 1 mm³ and 5.75 mm³, 1 mm³ and 5 mm³, 1 mm³ and 5.25 mm³, 1 mm³ and 4.75 mm³, 1 mm³ and 4.5 mm³, 1 mm³ and 4.25 mm³, 1 mm³ and 4 mm³, 1 mm³ and 3.75 mm³, 1 mm³ and 3.5 mm³, 1 mm³ and 3.25 mm³, 1 mm³ and 3 mm³, 1 mm³ and 2.75 mm³, 1 mm³ and 2.5 mm³, 1 mm³ and 2.25 mm³, 1 mm³ and 2 mm³, 1 mm³ and 1.75 mm³, 1 mm³ and 1.5 mm³, 1 mm³ and 1.25 mm³, 1.5 mm³ and 6 mm³, 1.5 mm³ and 5.75 mm³, 1.5 mm³ and 5 mm³, 1.5 mm³ and 5.25 mm³, 1.5 mm³ and 4.75 mm³, 1.5 mm³ and 4.5 mm³, 1.5 mm³ and 4.25 mm³, 1.5 mm³ and 4 mm³, 1.5 mm³ and 3.75 mm³, 1.5 mm³ and 3.5 mm³, 1.5 mm³ and 3.25 mm³, 1.5 mm³ and 3 mm³, 1.5 mm³ and 2.75 mm³, 1.5 mm³ and 2.5 mm³, 1.5 mm³ and 2.25 mm³, 1.5 mm³ and 2 mm³, 1.5 mm³ and 1.75 mm³, 2.0 mm³ and 6 mm³, 2.0 mm³ and 5.75 mm³, 2.0 mm³ and 5 mm³, 2.0 mm³ and 5.25 mm³, 2.0 mm³ and 4.75 mm³, 2.0 mm³ and 4.5 mm³, 2.0 mm³ and 4.25 mm³, 2.0 mm³ and 4 mm³, 2.0 mm³ and 3.75 mm³, 2.0 mm³ and 3.5 mm³, 2.0 mm³ and 3.25 mm³, 2.0 mm³ and 3 mm³, 2.0 mm³ and 2.75 mm³, 2.0 mm³ and 2.5 mm³, 2.0 mm³ and 2.25 mm³, 2.5 mm³ and 6 mm³, 2.5 mm³ and 5.75 mm³, 2.5 mm³ and 5 mm³, 2.5 mm³ and 5.25 mm³, 2.5 mm³ and 4.75 mm³, 2.5 mm³ and 4.5 mm³, 2.5 mm³ and 4.25 mm³, 2.5 mm³ and 4 mm³, 2.5 mm³ and 3.75 mm³, 2.5 mm³ and 3.5 mm³, 2.5 mm³ and 3.25 mm³, 2.5 mm³ and 3 mm³, 2.5 mm³ and 2.75 mm³, 3.0 mm³ and 6 mm³, 3.0 mm³ and 5.75 mm³, 3.0 mm³ and 5 mm³, 3.0 mm³ and 5.25 mm³, 3.0 mm³ and 4.75 mm³, 3.0 mm³ and 4.5 mm³, 3.0 mm³ and 4.25 mm³, 3.0 mm³ and 4 mm³, 3.0 mm³ and 3.75 mm³, 3.0 mm³ and 3.5 mm³, 3.0 mm³ and 3.25 mm³, 3.5 mm³ and 6 mm³, 3.5 mm³ and 5.75 mm³, 3.5 mm³ and 5 mm³, 3.5 mm³ and 5.25 mm³, 3.5 mm³ and 4.75 mm³, 3.5 mm³ and 4.5 mm³, 3.5 mm³ and 4.25 mm³, 3.5 mm³ and 4 mm³, 3.5 mm³ and 3.75 mm³, 4 mm³ and 6 mm³, 4 mm³ and 5.75 mm³, 4 mm³ and 5 mm³, 4 mm³ and 5.25 mm³, 4 mm³ and 4.75 mm³, 4 mm³ and 4.5 mm³, 4 mm³ and 4.25 mm³, 4.5 mm³ and 6 mm³, 4.5 mm³ and 5.75 mm³, 4.5 mm³ and 5 mm³, 4.5 mm³ and 5.25 mm³, 4.5 mm³ and 4.75 mm³, 5 mm³ and 6 mm³, or 5 mm³ and 5.75 mm³).

When viewed from the top of the skin (i.e., along the z-direction or within the xy-plane of the skin), the shape of the hole can be circular or non-circular (e.g., elliptical). Exemplary shapes of tissue portions are provided in Figures 1A-1C and 3A-3C and its associated text of U.S. Pub. No. 2012/0041430, which are hereby incorporated by reference in its entirety

Any beneficial areal fraction of the skin region can be removed, such as an areal fraction of less than about 70% (e.g., less than about 65%, 60%, 55%, 50%, 45%, 40%, 35%, 30%, 25%, 20%, 10%, or 5%) or such as between about 5% and 80% (e.g., between about 5% and 10%, 5% and 10%, 5% and 20%, 5% and 25%, 5% and 30%, 5% and 35%, 5% and 40%, 5% and 45%, 5% and 50%, 5% and 55%, 5% and 60%, 5% and 65%, 5% and 70%, 5% and 75%, 10% and 10%, 10% and 20%, 10% and 25%, 10% and 30%, 10% and 35%, 10% and 40%, 10% and 45%, 10% and 50%, 10% and 55%, 10% and 60%, 10% and 65%, 10% and 70%, 10% and 75%, 10% and 80%, 15% and 20%, 15% and 25%, 15% and 30%, 15% and 35%, 15% and 40%, 15% and 45%, 15% and 50%, 15% and 55%, 15% and 60%, 15% and 65%, 15% and 70%, 15% and 75%, 15% and 80%, 20% and 25%, 20% and 30%, 20% and 35%, 20% and 40%, 20% and 45%, 20% and 50%, 20% and 55%, 20% and 60%, 20% and 65%, 20% and 70%, 20% and 75%, or 20% and 80%).

Furthermore, the plurality of tissue portions can be incised or excised in any beneficial pattern within the skin region. Exemplary patterns within the skin region include tile patterns or fractal-like shapes, where the array of hollow tubes can be arranged, e.g., in a base, to effectuate such a pattern. For example, a higher density and/or smaller spacing of tissue portions (e.g., slits and/or holes) can be incised or excised in the skin in center of the pattern or in thicker portions of the skin. In another example, the pattern within the skin can be random, staggered rows, parallel rows, a circular pattern, a spiral pattern, a square or rectangular pattern, a triangular pattern, a hexagonal pattern, a radial distribution, or a combination of one or more such patterns of the incised or excised tissue portions. The pattern can arise from modifications to the average length, depth, or width of an incised or excised tissue portion, as well as the density, orientation, and spacing between such incisions and/or excisions (e.g., by using an apparatus having one or more blades or tubes with differing lengths, widths, or geometries that are arranged in a particular density or spacing pattern). Such patterns can be optimized to promote unidirectional, non-directional, or multidirectional contraction or expansion of skin (e.g., in the x-direction, y-direction, x-direction, x-y plane, y-z plane, x-z plane, and/or xyz-plane), such as by modifying the average length, depth, width, density, orientation, and/or spacing between incisions and/or excisions.

Any useful portion of the skin can be incised or excised. Such tissue portions can include epidermal tissue, dermal tissue, and/or cells or tissue proximal to the dermal/fatty layer boundary (e.g., stem cells). In particular embodiments, the incised or excised tissue portions forms a hole in the skin region, where the depth of the hole is more than about 1.0 mm and results in a tissue portion having a length that is more than about 1.0 mm (e.g., about 1.0 mm, 1.5 mm, 2.0 mm. 2.5 mm, 3.0 mm, or 3.5 mm). In particular embodiments, the incised or excised tissue portions forms a slit in the skin region, where the depth of the slit is more than about 1.0 mm and results in a tissue portion having a length that is more than about 1.0 mm (e.g., about 1.0 mm, 1.5 mm, 2.0 mm. 2.5 mm, 3.0 mm, or 3.5 mm). In some embodiments, the tissue portion has a length that corresponds to a typical total depth of the skin layer (e.g., epidermal and dermal layers). Based on the part of the body, the total depth of the epidermal and dermal layers can vary. In some embodiments, the depth of the epidermal layer is between about 0.8 mm to 1.4 mm, and/or the depth of the dermal layer is between about 0.3 mm to 4.0 mm. In other embodiments, the total depth of the skin layer (e.g., epidermal and dermal layers) is between about 1.0 mm and 5.5 mm, thereby resulting in a tissue portion having a length between about 1.0 mm and 5.5 mm (e.g., between about 1.0 mm and 1.5 mm, 1.0 mm and 2.0 mm, 1.0 mm and 2.5 mm, 1.0 mm and 3.0 mm, 1.0 mm and 3.5 mm, 1.0 mm and 4.0 mm, 1.0 mm and 4.5 mm, 1.0 mm and 5.0 mm, 1.5 mm and 2.0 mm, 1.5 mm and 2.5 mm, 1.5 mm and 3.0 mm, 1.5 mm and 3.5 mm, 1.5 mm and 4.0 mm, 1.5 mm and 4.5 mm, 1.5 mm and 5.0 mm, 1.5 mm and 5.5 mm, 2.0 mm and 2.5 mm, 2.0 mm and 3.0 mm, 2.0 mm and 3.5 mm, 2.0 mm and 4.0 mm, 2.0 mm and 4.5 mm, 2.0 mm and 5.0 mm, 2.0 and 5.5 mm, 2.5 mm and 3.0 mm, 2.5 mm and 3.5 mm, 2.5 mm and 4.0 mm, 2.5 mm and 4.5 mm, 2.5 mm and 5.0 mm, 2.5 mm and 5.5 mm, 3.0 mm and 3.5 mm, 3.0 mm and 4.0 mm, 3.0 mm and 4.5 mm, 3.0 mm and 5.0 mm, 3.0 and 5.5 mm, 3.5 mm and 4.0 mm, 3.5 mm and 4.5 mm, 3.5 mm and 5.0 mm, 3.5 and 5.5 mm, 4.0 mm and 4.5 mm, 4.0 mm and 5.0 mm, 4.0 and 5.5 mm, 4.5 mm and 5.0 mm, 4.5 and 5.5 mm, or 5.0 mm and 5.5 mm). In yet other embodiments, the average total depth of the tissue portion or the skin layer (e.g., epidermal and dermal layers) is about 1.5 mm. In yet other embodiments, the average total depth of the tissue portion or the skin layer (e.g., epidermal and dermal layers) is about 3 mm. In further embodiments, the tissue portion does not include a significant amount of subcutaneous tissue, and any apparatus described herein can be optimized (e.g., with one or more stop arrangements) to control the depth of the incision or excision and/or the length of the incised or excised tissue portions.

Incisions can be performed by any useful procedure or component. For example, a plurality of incised tissue portions can be achieved by use of an ablative laser (e.g., an ablative CO₂ laser (about 10600 nm), a superficial fractional CO₂ laser, a fractional Er:YAG laser (about 2940 nm), a fractional Er:YSGG laser (about 2790 nm), an Nd-YAG laser (about 1320 nm), a mid-IR fractional photothermolysis laser, or a fractional deep dermal ablation CO₂ laser), an ultrasonic apparatus, a non-coherent light source, a radiofrequency source, or a plurality of blades (e.g., substantially parallel blades). In some embodiments, the one or more blades can include connected, adjacent blades to provide narrow, elongated openings (or slits) in the skin region. Exemplary procedures and apparatuses including one or more blades are described in Figures 3, 4, 5A-5B, 6A-6B, 7A-7C, 8A-8C, 9, 10, 11A-11B, 14, 15A-15B, and 16A-16D and its associated text in U.S. Pub. No. 2011/0251602, which are incorporated herein by reference.

Excisions can be performed by any useful procedure or component. For example, a plurality of excised tissue portions can be achieved by use of one or more hollow tubes or needles (e.g., where the inner diameter of at least one tube is less than about 0.5 mm, about 0.3 mm, or about 0.2 mm) or one or more solid tubes or needles. Exemplary components for performing excisions include a needle (e.g., a 16 gauge needle having an inner diameter of 1.194 mm; an 18 gauge needle having an inner diameter of 0.838 mm; a 20 gauge needle having an inner diameter of 0.564 mm; a 23 gauge needle having an inner diameter of about 0.337 mm and an outer diameter of about 0.51 mm, thereby resulting in a tissue portion having a dimension (e.g., a width or diameter) of about 0.3 mm; a 25 gauge needle having an inner diameter of about 0.26 mm or a thin-walled 25 gauge needle having an inner diameter of about 0.31 mm and an outer diameter of about 0.51 mm, thereby resulting in a tissue portion having a dimension (e.g., a width or diameter) of about 0.2 mm; a 30 gauge needle having an inner diameter of about 0.159 mm; a 32 gauge needle having an inner diameter of about 0.108 mm; or a 34 gauge needle having an inner diameter of about 0.0826 mm), where such needles can be a hollow biopsy needle or a solid needle; one or more microaugers; or one or more microabraders.

The geometry of the one or more tubes can include at least two points (or prongs) (e.g., at least three, four, five, six, seven, eight, or more points) provided at a distal end of the tube (e.g., to facilitate separation of the tissue portions from the surrounding tissue and/or insertion of the tubes into the skin region), where an angle formed by at least one of the points is about thirty degrees. Exemplary tubes include those having two points (e.g., by grinding in orientations that are 180 degrees apart), three points (e.g., by grinding in orientations that are 120 degrees apart), or four points (e.g., by grinding in orientations that are 90 degrees apart). The points can optionally include a beveled edge (e.g., to further facilitate separation of tissue portions or insertion of tubes).

The points can have any useful geometric configuration. In one example, the tube has a longitudinal axis (i.e., along the length of the tube) and a diameter (i.e., through the cross-section of the tube), as well as a proximal end and the distal end. The distal end can include one or more points, where each point is characterized by angle α (i.e., the angle between each of the opposing lateral sides of the tube that forms the point and the longitudinal axis of the tube). When viewed from the side, the angle formed by a point is characterized by angle 2α. For example, a tip angle of about 30 degrees corresponds to an angle α of about 15 degrees. Furthermore, the angled distal end of the tube can be formed (e.g., by grinding or cutting) at angle α, e.g., to form a second bevel structure at the distal end of a tube, where this second bevel is characterized by angle β and is orthogonal to the primary point (or bevel) characterized by angle α. This second bevel can be provided to reduce the size or width of the point. Exemplary angle α and β includes less than about 20 degrees, 15 degrees, 10, degrees, or 5 degrees (e.g., about 15 degrees, 10 degrees, 6 degrees, 5 degrees, or 3 degrees). See, e.g., Figures 8A-8J and its associated text of U.S. Pub. No. 2011/0313429, which are hereby incorporated by reference in its entirety, for exemplary points, angle α, and angle β.

The tubes can optionally include one or more notches within the lumen of the needle (i.e., if the tube is hollow) and/or extensions on the exterior surface of the needle (e.g., at the distal portion of the needle). Such notches and extensions could be useful to promote cutting of tissue surrounding the incised or excised tissue portions. Exemplary needles having such notches and/or extensions include a microauger, as well as any needles provided in Figures 5A-5E and described its associated text of International Pub. No. WO 2012/103492, which are hereby incorporated by reference in its entirety, for apparatuses having notches and/or extensions.

The tubes can optionally include one or more protrusions or barbs within the lumen of the needle (i.e., if the tube is hollow) to promote retention of fat within the needle. In use, an apparatus including such tubes can be inserted into the subcutaneous fat layer and then withdrawn to remove retained fat tissue. See, e.g., Figures 1A-1C, 2A-2C, 3A, 4, 5A-5C, 6A-6B, 7, and 8A-8C and its associated text of International Pub. No. WO 2013/013196, which are hereby incorporated by reference in its entirety, for apparatuses having protrusions or barbs.

The components for making incisions and/or excisions (e.g., blades and/or tubes) can be provided in any useful arrangement (e.g., a linear array, a radial array, or any described herein) of one or more components (e.g., two, three, four, five, ten, thirty, fifty, hundred, or more). The spacing between each component (e.g., blade and/or tube) can be of any useful dimension, such as between about 1 mm and 50 mm (e.g., between about 1 mm and 40 mm, 1 mm and 30 mm, 1 mm and 25 mm, 1 mm and 20 mm, 1 mm and 15 mm, 1 mm and 10 mm, 1 mm and 5 mm, 1 mm and 3 mm, 3 mm and 50 mm, 3 mm and 40 mm, 3 mm and 30 mm, 3 mm and 25 mm, 3 mm and 20 mm, 3 mm and 15 mm, 3 mm and 10 mm, 3 mm and 5 mm, 5 mm and 50 mm, 5 mm and 40 mm, 5 mm and 30 mm, 5 mm and 25 mm, 5 mm and 20 mm, 5 mm and 15 mm, 5 mm and 10 mm, 10 mm and 50 mm, 10 mm and 40 mm, 10 mm and 30 mm, 10 mm and 25 mm, 10 mm and 20 mm, 10 mm and 15 mm, 15 mm and 50 mm, 15 mm and 40 mm, 15 mm and 30 mm, 15 mm and 25 mm, 15 mm and 20 mm, 20 mm and 50 mm, 20 mm and 40 mm, 20 mm and 30 mm, 20 mm and 25 mm, 30 mm and 50 mm, 30 mm and 40 mm, or 40 mm and 50 mm). Such arrangements can include one or more tubes and/or blades (e.g., about 2, 3, 4, 5, 6, 7, 8, 9, 10, 15, 20, 25, 30, 35, 40, 45, 50, 75, 100, or more tubes and/or blades, such as between about 2 and 100 tubes and/or blades (e.g., between 2 and 10, 2 and 15, 2 and 20, 2 and 25, 2 and 30, 2 and 35, 2 and 40, 2 and 45, 2 and 50, 2 and 75, 5 and 10, 5 and 15, 5 and 20, 5 and 25, 5 and 30, 5 and 35, 5 and 40, 5 and 45, 5 and 50, 5 and 75, 5 and 100, 10 and 20, 10 and 25, 10 and 30, 10 and 35, 10 and 40, 10 and 45, 10 and 50, 10 and 75, 10 and 100, 15 and 20, 15 and 25, 15 and 30, 15 and 35, 15 and 40, 15 and 45, 15 and 50, 15 and 75, 15 and 100, 20 and 25, 20 and 30, 20 and 35, 20 and 40, 20 and 45, 20 and 50, 20 and 75, 20 and 100, 25 and 30, 25 and 35, 25 and 40, 25 and 45, 25 and 50, 25 and 75, 25 and 100, 30 and 35, 30 and 40, 30 and 45, 30 and 50, 30 and 75, 30 and 100, 35 and 40, 35 and 45, 35 and 50, 35 and 75, 35 and 100, 40 and 45, 40 and 50, 40 and 75, 40 and 100, 50 and 75, or 50 and 100)).

Such arrangements of components can be any of various two-dimensional or three-dimensional patterns along a base holding one or more components for making incisions and/or excisions (e.g., blades and/or tubes). The base can be optionally mounted on a roller apparatus having a cylindrical body with a longitudinal rotational axis, where the one or more blades and/or tubes are arranged on the longitudinal surface of the cylindrical body. In some embodiments, the blade or tube extends as substantially coplanar extensions of the cylindrical body. In use, rotation of the cylindrical body along the skin results in the incision or excision of tissue portions by the blade or tubes. Exemplary roller apparatuses are provided in Figures 11A-11B and its associated text in U.S. Pub. No. 2011/0251602, in Figures 3A-3B and its associated text in International Pub. No. WO 2012/103492, which are hereby incorporated by reference in its entirety.

Such components for making incisions and/or excisions (e.g., blades and/or tubes) can include one or more stop arrangements (e.g., one or more collars, which can be coupled to the blade to allow for adjustment along the long axis of the blade or which can be coupled to the outer portion of the tube and be adjusted along the long axis of the tube to control the depth of incision or excision in the biological tissue); one or more sleeves around a portion of a blade and/or a tube, such that the sleeve is slidably translatable along the longitudinal axis of the tube or blade (e.g., to incise or excise tissue portions below the surface of the skin region); a vibrating arrangement (e.g., a piezoelectric element, a solenoid, a pneumatic element, or a hydraulic element) that mechanically couples to at least one blade or hollow tube (e.g., to promote insertion of one or more blades or tubes into the skin region, such as by providing an amplitude of vibration in the range of about 50-500 µm (e.g., between about 100-200 µm) or by providing a frequency of the induced vibrations to be between about 10 Hz and about 10 kHz (e.g., between about 500 Hz and about 2 kHz, or even about 1 kHz)); a suction or pressure system (e.g., by squeezing a flexible bulb or deformable membrane attached thereto or by opening a valve leading from a source of elevated pressure, such as a small pump) to stabilize the surrounding skin region prior to incision or excision and/or to facilitate removal of the skin portions from the tube; a pin within the lumen to the tube to facilitate removal of the skin portions from the tube; one or more actuators for positioning, translating, and/or rotating the one or more blades and/or tubes relative to the skin portion or relative to the optional one or more pins; a housing or frame to stabilize the surrounding skin region prior to incision or excision; one or more actuators for positioning and/or translating the one or more pins relative to the skin portion or relative to one or more tubes; one or more sensors (e.g., force sensors, optical sensors, laser fibers, photodetectors, and/or position sensors) in communication with one or more tubes, blades, pins, actuators, valves, or pressure systems to detect the position of the tubes or pins, the presence of a tissue portion in the tube, the position of the apparatus relative to the treated skin portion; a reciprocating arrangement attached to a base or a substrate having one or more attached blades or tubes (e.g., a motor or actuator configured to repeatedly insert and/or withdrawn one or more blades or tubes); a fluid system coupled to the blades and/or tubes to facilitate removal of incised or excised tissue portions or to irrigate the skin portion, e.g., with saline or a phosphate buffered solution; a heat source (e.g., a resistive heater or current) in communication with the blade and/or tube to promote cauterization or ablation of tissue portions; an optical element (e.g., a lens, a prism, a reflector, etc.) to facilitate viewing of the skin portion beneath the apparatus, tube, or blade; and/or an abrading element optionally mounted on a rotating shaft (e.g., to promote dermabrasion).

Exemplary blades, tubes, pins, apparatuses, and methods are provided in Figures 5A-5B, 6A-6C, 7, and 8A-8B and its associated text of U.S. Pub. No. 2012/0041430; in Figures 8A-8J, 10A-10B, 11, 12, 13A-13B, 14, and 15A-15E and its associated text of U.S. Pub. No. 2011/0313429; in Figures 3, 4, 5A-5B, 6A-6B, 7A-7C, 8A-8C, 9, 10, 11A-11B, 14, 15A-15B, and 16A-D and its associated text in U.S. Pub. No. 2011/0251602; in Figures 1A-1B, 2A-2C, 3A-3B, 4A-4B, 5A-5E, and 6 and its associated text in International Pub. No. WO 2012/103492; in Figures 1, 2, 3, and 4 and its associated text in International Pub. No. WO 2012/103483; in Figures 1, 3, and 4 and its associated text in International Pub. No. WO 2012/103488; in Figures 1A-1C, 2A-2C, 3A, 4, 5A-5C, 6A-6B, 7, and 8A-8C and its associated text of International Pub. No. WO 2013/013196; in Figures 1, 2A-2D, 3, and 4 and its associated text of International Pub. No. WO 2013/013199, which are hereby incorporated by reference in its entirety.

The tubes, blades, pins, and apparatuses can be formed from any useful material and optionally coated or chemically treated to promote incision or excision of a tissue portion and/or to increase precision or effectiveness for treating the skin region. Exemplary materials include metal (e.g., a stainless steel tube, 304 stainless steel, a surgical stainless steel), a biopsy needle, an epoxy, a glass, a polymer, a plastic, a resin, another structurally rigid material, or a similar structure. Exemplary coatings include a lubricant, a low-friction material (e.g., Teflon™), a chromium coating (e.g., ME-92™, such as to increase material strength), a plastic, a polymer (e.g., nylon or polyethylene), a polished metal alloy, or the like.

In particular embodiments, an apparatus for treating skin includes at least one hollow tube including at least two points provided at a distal end thereof and an optional stop arrangement coupled to the outer portion of the tube (e.g., to control and/or limit a distance to which the one tube is inserted into a biological tissue), where the angle formed by at least one of the points is about thirty degrees, where the inner diameter of at least one tube is less than about 1 mm, and where at least one section of the hollow tube is structured to be inserted into a biological tissue to incise or excise at least one tissue therefrom when the tube is withdrawn from the tissue. In other embodiments, the apparatus further includes a pin provided at least partially within the central lumen of a tube, where the pin is controllably translatable in a direction along a longitudinal axis of the one tube and the pin is configured to facilitate removal of at least one tissue portion from the tube. In another embodiment, the apparatus for treating skin includes a plurality of cutting arrangements (e.g., blades) structured to form a plurality of spaced-apart micro-slits (e.g., openings) in tissue, where each of the micro-slits has a length of extension along a surface of the tissue that is less than about 2 mm. In other embodiments, the apparatus includes at least one hollow tube (e.g., needle) configured to be at least partially inserted into a biological tissue; at least one opening provided on a wall of the hollow tube; at least one cutting edge protruding from the wall of the hollow tube proximal to the at least one opening; and a sleeve provided around at least a portion of the tube and configured to be translatable along a longitudinal axis of the tube, where a distance from the longitudinal axis of the tube to an outer edge of the sleeve is at least as large as a distance from the longitudinal axis of the tube to an outer portion of the cutting edge. In yet other embodiments, the apparatus includes a substrate; a plurality of hollow tubes (e.g., needles) affixed to the substrate and configured to be at least partially inserted into a biological tissue; at least one opening provided on or in a wall of each of the hollow tubes; at least one cutting edge protruding from the wall of each of the hollow tubes proximal to the at least one opening; and a sleeve provided around at least a portion of each of the tubes, where each tube is configured to be translatable along a longitudinal axis of a corresponding sleeve, and where a distance from the longitudinal axis of each tube to an outer edge of each corresponding sleeve is at least as large as a distance from the longitudinal axis of the tube to an outer portion of the cutting edge of the tube.

The procedures herein can include one or more optional processes that promote effective incision or excision of tissue portions or that benefit healing. Such optional processes include cooling, freezing, or partially freezing the skin portion prior to skin incision or excision (e.g., by applying a cryospray or by contacting a surface of a skin region with a cooled object for an appropriate duration), where such cooling and/or freezing can, e.g., increase mechanical stability of the tissue portions; treatment with red or near-infrared light of the skin portion to further promote healing of the tissue; and/or treatment with an optical energy source, such as any described herein (e.g., an ablative laser).

Exemplary procedures, methods, and apparatuses are provided in U.S. Pub. Nos. 2012/0041430, 2011/0313429, 2011/0251602, 2012/0226214, 2012/0226306 and 2012/0226214; International Pub. Nos. WO 2012/103492, WO 2012/103483, WO 2012/103488, WO 2013/013199, WO 2013/013196, and WO 2012/119131; Fernandes et al., "Micro-Mechanical Fractional Skin Rejuvenation," Plastic & Reconstructive Surgery 130(5S-1):28 (2012); and Fernandes et al., "Micro-Mechanical Fractional Skin Rejuvenation," Plastic & Reconstructive Surgery 131(2):216-223 (2013), where each is hereby incorporated by reference in its entirety.

### Examples

### Example 1: Method of treating skin regions

A skin region can be treated by any useful method prior to affixing a microclosure. For example, this method can include forming a plurality of small holes or microwounds in the skin through the dermal and epidermal layer. Generally, the dimension of the holes is in the range of 50-500 µm in diameter. Without wishing to be limited by theory, it is envisioned that up to 40% of the treated skin surface (e.g., 10 to 20% of the skin surface) can be removed and that the amount of removed skin determines the extent of the tightening effect. The holes can be formed surgically, for example, by using a hollow coring needle (e.g., any described herein). Alternative forms of energy, e.g., such as laser, non-coherent light, radio-frequency, or ultrasound, can also be used to form the holes. The holes can be circular or have any other preferred shape (e.g., an elongated shape). After the formation of such holes, the methods and devices (e.g., microclosures) described herein (e.g., in the following Examples) can be employed to reduce skin volume, surface, or area, and/or tighten skin.

### Example 2: Exemplary microstaples (Method 1)

After treating the skin to form a plurality of microwounds in a skin portion, one or more miniature staples (or microstaples) can be used to compress the skin. Compression, staple deposition, and wound formation may occur in any order.

Figures 1A-1C describe an exemplary process. First, a microwound is formed through the epidermal and dermal layer. A compressive force is then applied to close the hole in a preferred direction. Without wishing to be limited by mechanism, the compression direction is aligned with the desired direction of tightening (e.g., parallel to the skin in the x-direction as shown in Figure 1, or in any useful direction, such as in the y-, z-, xy-, xz-, yz-, or xyz-direction). A miniature staple is deposited in the tissue and maintains the hole closed for the duration of the wound healing. Without wishing to be limited by mechanism, this duration is sufficient for the formation of a closed epidermal layer and for formation of a new tissue matrix in the hole and is typically less than a week. This procedure can be repeated for each individual hole. In some embodiments, the staple can be bioresorbable. For example, the time for resorption can account for the time to promote sufficient wound healing such that the wound no longer requires the staple to provide mechanical support for healing. Alternatively, the staple can be include one or more non-resorbable materials, which can optionally be removed after wound healing.

The staple can be deposited within or onto the microwound. For example, Figure 1B provides a microstaple deposited on the microwound and at the surface of the skin. Microstaples can also be deposited within the microwound to facilitate healing. Figures 2A-D describe this process. A coring needle can be used to form a hole through the epidermal and dermal layer, where the cored tissue detaches at the interface with the subcutaneous fat layer. A vacuum can optionally be applied in the needle to help detach the tissue and to aspirate the cored tissue plug through the needle. While the needle is holding the hole open, a pin holding the staple can be introduced in the lumen of the needle. The staple can then be pushed into the formed hole. As shown in Figure 2C, the needle can be removed, and a compressive force can be applied on the skin to close the hole. When the pin is moved into the lumen of the needle, the staple can engage into the tissue and then detach from the pin. As shown in Figure 2D, the staple maintains the hole closed for the duration of the healing process. Optionally, the staple material is bio-resorbable, as described herein.

Alternatively, the staple can be deposited externally (i.e., on the skin surface) as described in Figures 3A-3D. The first step is similar to that described for Figure 2A. Then, as shown in Figure 3B, a centering pin can be introduced in the lumen of the needle to maintain the apparatus in alignment with the hole. Next, the needle is moved upwards and out of the hole. A shown in Figure 3C, the hole is closed by a compressive force applied on the skin, and a staple is then deposited on the skin surface by a mechanism that is co-axial with the needle and the pin. Finally, as shown in Figure 3D, the staple holds the wound closed for the duration of the healing process.

The microstaple may be of any useful configuration, shape, and/or design. In particular embodiments, the microstaple maintains a first compressive force in a particular direction (e.g., any described herein). Further, the microstaple can include one or a plurality of prongs or tips (e.g., to maintain a first compressive force in more than one direction or to promote secure attachment of microstaple to the microwound).

In some embodiments, the staple can include a circular geometry (e.g., as described in Figures 4-6). As shown in Figure 4, the internal diameter of the staple is slightly larger than the outer diameter of the needle used to deposit the staple (e.g., using any needle described herein) or slightly larger than a microwound. The tips of the staple engage with the tissue and maintain the microwound in a closed state. The staple preferably has more than two tips (e.g., three, four, five, six, seven, eight, or more tips).

Alternatively, the staple can include a thin circular strip of material with a sharp edge. As shown in Figure 5, the staple can include a sharp edge that engages with the tissue. The entire staple can be inserted into the tissue to maintain the microwound in the closed state. As described above, the internal diameter of the staple can be slightly larger than the outer diameter of the needle and/or the microwound.

In other embodiments, the staple can be pre-constrained as to apply a first compressive force to close the microwound. As shown in Figure 6, an exemplary multi-tip staple can be used, which forms a closed ellipse.

The microstaples can be formed with any useful material. For instance, the material may be resorbable or biodegradable as to not require subsequent removal (e.g,. any such material described herein, such as PGA, PLA, PGA/PLA, or any other herein). In another instance, the material may be non-resorbable (e.g., any such material described herein, such as a metal or metal alloy, e.g., a Ni-Ti alloy).

### Example 3: Exemplary microdressings (Method 2)

The microwounds can be individually closed by miniature wound dressings (or microdressings). Compression, microdressing deposition, and wound formation may occur in any order.

Figures 7A-7D describe this method. First, a plurality of holes are formed through the epidermal and dermal layer. Then, the holes are closed by a compressive force applied on the skin, for example, by the same apparatus that created the holes. The compressive force closes the holes in a preferred direction. Microdressings are then applied on the closed wound and maintain the wound closed during the healing process.

Alternatively, the compressive force can be applied by a pre-stretched microdressing. Figures 8A-8D describe this process. The dressing is pre-constrained (pre-stretched) before adhesion to the skin. Then, an apparatus (e.g., a coring needle) is introduced through the epidermal and dermal layer to core tissue, thereby forming microwounds. A cylindrical component that is co-axial with the needle is moved towards the skin. In Figure 8B, the microdressing adheres to the end of the cylindrical component to facilitate deposition of the dressing after hole formation. The proximal surface of the microdressing (i.e., the surface facing the skin) can include an adhesive or an adhesive layer (e.g., any described herein). In particular embodiments, the adhesion force on the proximal side of the dressing is greater than the adhesion force on distal side of the dressing (i.e., the surface attached to the cylindrical holder). In this manner, the dressing can easily be peeled away from the holder. Next, the needle is removed, which results in removing the cored tissue. The cylindrical holder is also removed, thereby applying the dressing in place on the skin. Finally, as the dressing was applied in a pre-stretched state, the dressing closes the wound and holds the wound closed during the healing process.

The dressing can also be applied to the skin without being pre-stretched (i.e., a microdressing including an unstretched layer). A shown in Figure 9, the apparatus or applicator to apply such a dressing can include a centering pin. Such a pin can be inserted into the microwound, and a compressive force can be applied (e.g., by the applicator or by another device). Then, the microdressing is applied on the closed microwound by a cylindrical holder that is co-axial with the centering pin.

### Example 4: Exemplary method for microwelding (Method 3)

The present invention also includes any useful device to promote skin treatment. Such exemplary devices include energy sources, such as lasers that have been used to weld tissue (with or without photosensitizing dye). The small holes can be closed by laser welding to tighten the skin. Similar to the method described above, the device first forms a hole in the skin, then applies a closing force on the hole and finally welds the hole closed with a laser. Without wishing to be limited by theory, the mechanism of laser skin welding is not fully understood but it is thought that it is affects the structure of the collagen helix and that the affixed lased tissue surface create a chemical bond between denatured collagen. Other energy sources such as radio-frequency or heated probes can be used to weld tissue. Compression, microwelding, and wound formation may occur in any order. Accordingly, the present invention encompasses the use of such devices to form microwelds (i.e., welds having at least one dimension of from about 10 µm to about 1 mm after application to a microwound).

Alternatively, one might take advantage of the rigidity of the thermally injured skin tissue. Fractional laser ablation generates a thermal injury zone around the ablated tissue. This thermal injury zone is rigid and prevents closure of holes generated by fractional laser ablation. In another embodiment, holes can be generated by mechanical fractional ablation (i.e., without generating a thermal injury). The holes are then closed by lateral compression, a probe is inserted in the hole and heated while the hole is closed. A rigid zone will form around the probe that will prevent the holes to re-open.

### Example 5: Exemplary method for microgluing (Method 4)

The microwounds can be individually closed by microgluing (or use of one or more sealants or adhesives). The small holes can be closed with a sealant in a preferred direction. For example, substances promoting collagen cross-linking such as riboflavin or rose Bengal can be applied in the small holes. The holes can then be closed in a preferred direction by applying a compressive force on the skin. Finally, the treated surface can be illuminated with a light source having a wavelength that activates cross-linking by the selected agent. A similar approach can be deployed with synthetic glues (e.g. cyanoacrylate, polyethylene glycol, gelatin-resorcinol-formaldehyde) or biologic sealants (e.g. albumin-based, fibrin-based sealants that promote clotting). Compression, microgluing, and wound formation may occur in any order.

The sealant can be dispensed by a different or the same apparatus that cores the small holes in the skin. After a hole is formed, the apparatus can dispense the sealant in the hole, apply a closing force on the hole, and activate the sealant if necessary (e.g., by emission of light or by a thermal or chemical mechanism). The closing or compressive force can be released after the sealant sets and the apparatus generates another hole. The closing force can be uni-directional or not. In some embodiments, it may be preferable to dispense the sealant after applying the closing force on the hole, for example, to prevent contamination of the inside of the hole by sealant that is not bio-resorbable. In this situation, the apparatus closes the hole first, applies the sealant on the closed hole, activates the sealant if necessary, and releases the closing force after the sealant sets before moving to the next hole. Figures 10A-10C describe this process and shows (A) hole formation, (B) deposition of sealant in the hole before hole closure (e.g., if the sealant is bio-resorbable), and (C) deposition of sealant after hole closure (e.g., if the sealant is not bio-resorbable).

The sealant or adhesive can be applied by any useful apparatus (e.g., a dispenser). Exemplary, non-limiting dispensers are provided in Figure 11. The dispenser can allow for deposition of an adhesive in the hole before hole closure (Figure 11, left) and on the skin surface after hole closure (Figure 11, right). For instance (Figure 11, left), dispensing of the sealant in the hole is achieved by a tube, which is inserted in the lumen of the coring needle prior to needle removal. For this use, the external diameter of the tube is smaller than the internal diameter of the coring needle. A compressive force is applied on the skin to close the hole prior to sealant dispensing. The tube is then removed, and the wound is allowed to set under compression. In another example (Figure 11, right), sealant is dispensed on the skin surface by a tube that is inserted through the lumen of the coring needle. The outer diameter of the dispensing tube is smaller than the inner diameter of the coring needle. A centering pin is located in the sealant dispenser tube. A compressive force is applied on the skin to create a hermetic seal around the centering pin and to prevent sealant leakage in the wound. The sealant is then dispensed, and the pin is removed while compressive force is continuously applied on the skin to close the hole. The wound is then allowed to set under compression.

### Other embodiments

All publications, patent applications, and patents mentioned in this specification are herein incorporated by reference.

Various modifications and variations of the described method and system of the invention will be apparent to those skilled in the art without departing from the scope and spirit of the invention. Although the invention has been described in connection with specific desired embodiments, it should be understood that the invention as claimed should not be unduly limited to such specific embodiments. Indeed, various modifications of the described modes for carrying out the invention that are obvious to those skilled in the fields of medicine, pharmacology, or related fields are intended to be within the scope of the invention.

The following pages 64 to 67 contain specific embodiments.
1. A microclosure comprising a material having at least one dimension of from about 10 µm to about 1 mm after application to a microwound and having no dimension larger than 5 mm, wherein said microclosure maintains a first compressive force when applied to said microwound.
2. The microclosure of 1, wherein said microclosure comprises a microstaple, a microdressing, or a suture.
3. The microclosure of 2, wherein said microstaple has a circular geometry.
4. The microclosure of 3, wherein said microstaple further comprises one or more tips and/or one or more sharp edges.
5. The microclosure of 3 or 4, wherein said microstaple is pre-constrained prior to application to said microwound.
6. The microclosure of 2, wherein said microdressing comprises (i) an adhesive layer and (ii) a regulatable layer that comprises one or more materials, wherein exposure of said regulatable layer to one or more external stimuli results in a change in a physical characteristic in said one or more materials in at least a portion of said microdressing.
7. The microclosure of 6, wherein said change in a physical characteristic comprises an increase in tension of said microdressing, a decrease in tension of said microdressing, an increase in compressive force exerted by said microdressing, a decrease in compressive force exerted by said microdressing, compression in one or more directions of said microdressing, and/or expansion in one or more directions of said microdressing.
8. The microclosure of 2, wherein said microdressing comprises (i) an adhesive layer and (ii) a pre-stretched or unstretched layer.
9. The microclosure of 2, wherein said sealant is resorbable or non-resorbable.
10. The microclosure of 1, wherein said material comprises one or more of a metal, a metal alloy, a plastic, a polymer, a shape-memory polymer, a shape-memory alloy, a thermal-responsive material, a pH-responsive material, a light-responsive material, a moisture-responsive material, a solvent-responsive or chemical exposure-responsive material, an electric field-responsive material, a magnetic field-responsive material, an actuator-embedded material, an unstretched material, a pre-stretched material, an adhesive, a biocompatible matrix, a photosensitizer, a photochemical agent, a synthetic glue, a biologic sealant, a biodegradable adhesive, or a tissue glue.
11. The microclosure of any one of 1-10, further comprising having an areal dimension of less than about 4 mm².
12. The microclosure of any one of 1-11, wherein said first compressive force comprises a compression in one or more directions and/or an expansion in one or more directions, as compared to before application to said microwound.
13. The microclosure of 12, wherein said compression or said expansion is an increase or decrease in the x-axis, y-axis, and/or z-axis of said microclosure, as compared to before application to said microwound.
14. The microclosure of any one of 1-13, further comprising an attachment component adapted to facilitate removal of said microclosure.
15. The microclosure of 14, wherein said attachment component comprises a hook, a pincher, an eye, a loop, a post, a microfastener, a slot, a snap fastener, or a combination thereof.
16. An array comprising a plurality of the microclosures of any one of 1-15.
17. The array of 16, wherein each of said microclosures is separated by at least about 10 µm.
18. The array of 17, wherein each of said microclosures is separated by at least about 100 µm.
19. A skin treatment device comprising an applicator, wherein said applicator is adapted to apply the microclosure of any one of 1-15 or the array of any one of 16-18.
20. The device of 19, wherein said applicator comprises a needle and a pin, wherein said needle or said pin is adapted to releasably attach said microclosure or said array.
21. The device of 20, further comprising a holder that is co-axial to said needle and is adapted to releasably attach said microclosure or said array.
22. The device of 21, wherein said holder is adapted to apply a second compressive force to said microwound or a skin region.
23. The device of 19, wherein said applicator comprises a dispenser adapted to dispense a volume of sealant that is less than or equal to about 3 mm³.
24. The device of any one of 19-23, wherein said applicator is further adapted to make a plurality of microwounds in a skin region.
25. The device of any one of 19-24, wherein said applicator is further adapted to apply a second compressive force in a skin region.
26. The device of any one of 19-25, wherein said applicator is further adapted to remove said microclosure or said array.
27. The device of 26, wherein said microclosure or said array further comprises one or more attachment components adapted to facilitate removal of said microclosure.
28. The device of any one of 19-27, further comprising an apparatus for making a plurality of microwounds in a skin region.
29. The device of 28, wherein said apparatus is a microablation tool.
30. The device of any one of 19-29, further comprising an apparatus for applying a second compressive force in a skin region.
31. The device of any one of 19-30, further comprising an apparatus for removing said microclosure or said array.
32. A kit comprising:
   (a) one or more microclosures of any one of 1-15 or an array of any one of claims 16-18; and
   (b) the device of any one of 19-31,
   wherein said kit optionally comprises a sanitizing wipe, an antibiotic ointment, a macrowound dressing, and/or instructions for use.
33. The kit of 32, further comprising a remover for removing said microclosure or said array, wherein said remover is selected from the group consisting of an apparatus, a chemical agent, a biological agent, a polymeric material, an abrasive material, a macrodressing, an adhesive material, and a mechanical lifting device.
34. The kit of 33, wherein said one or more microclosures comprises an attachment component adapted to attach to said mechanical lifting device.
35. The kit of 33, wherein said apparatus or said mechanical lifting device comprises a heating component, an optical component, a radiofrequency component, a mechanical component, and/or an ultrasound component.
36. The kit of any one of 32-34, wherein said macrowound dressing is a tunable dressing.
37. A method of treating skin comprising:
   (a) forming a plurality of microwounds, each having an areal dimension that is less than about 1 mm² in the region of said skin and/or a volumetric dimension that is less than about 3 mm³ in the region of said skin; and
   (b) applying a plurality of microclosures to said plurality of microwounds, wherein said microclosure comprises a material having at least one dimension of from about 10 µm to about 1 mm after application,
   wherein said microclosure maintains a first compressive force, thereby treating said skin.
38. The method of 37, wherein said treating comprises reducing tissue volume or area, promoting beneficial tissue growth, tightening skin, rejuvenating skin, improving skin texture or appearance, removing skin laxity, and/or expanding tissue volume or area.
39. The method of 37 or 38, further comprising (c) applying a second compressive force to said skin region.
40. The method of any one of 37-39, further comprising (d) removing said microclosure or said array after treating said skin.
41. The method of any one of 37-40, comprising one or more microclosures of any one of 1-15 or an array of any one of 16-18.
42. The methods of any one of 37-40, wherein said one or more microclosures comprise a microweld or sealant.
43. The method of any one of 37-42, wherein, prior to step (b), a drug is administered into said microwound.
44. The method of 43, wherein said drug is administered as the microwounds are being formed.
45. The method of any one of 37-42, wherein the microclosure comprises a skin pigmentation modifying compound.
46. The method of 45, wherein said skin pigmentation modifying compound is a bleaching agent or lightening agent.
47. The method of 46, wherein said lightening agent is hydroquinone.

## Claims

1. A skin treatment device comprising:
(a) a hollow coring needle for forming a plurality of microwounds in the skin, each having an areal dimension less than 4 mm² and/or a volumetric dimension less than 6 mm³, and
(b) an applicator for applying a microdressing to the microwounds,

2. The skin treatment device according to claim 1 further comprising a pin, preferably a centering pin, within the lumen of the needle.

3. The skin treatment device according to claim 1 or 2, wherein the microdressing maintains a first compressive force when applied to said microwound, wherein the first compressive force includes a compression in one or more directions and/or an expansion in one or more directions, as compared to before application to the microwound wherein the compression or the expansion is an increase or decrease of at least 0.5% or from 0.5% to 50%, in the x-axis, y-axis, and/or z-axis of the microdressing, as compared to before application to the microwound.

4. The skin treatment device according to any one of claims 1-3, wherein the microwounds are small slits in the skin having a length or width of less than 1.0 mm.

5. The skin treatment device according to any one of claims 1-3, wherein the microwounds are small holes in skin having a diameter or width less than 1.0 mm and/or an areal dimension less than 4 mm² and/or a volumetric dimension that is less than 6 mm³.

6. The skin treatment device according to any one of claims 1-5, wherein the areal dimension is less than or equal to 1.0 mm², or between 0.001 mm² and 1.0 mm².

7. The skin treatment device according to any one of claims 1-6, wherein the microdressing is disc shaped, the disc having a diameter of less than 2 mm.

8. The skin treatment device according to any one of claims 1-7, wherein the microdressing comprises an adhesive layer and a regulatable layer that comprises one or more materials, wherein exposure of the regulatable layer to one or more external stimuli results in a change in a physical characteristic in the one or more materials in at least a portion of the microdressing.

9. The skin treatment device according to any one of claims 1-7, wherein the microdressing comprises an adhesive layer and a prestretched or unstretched layer.

10. The skin treatment device according to claim 8, wherein the regulatable layer comprises one or more stimulus-responsive materials, such as a shape-memory material, a shape-memory polymer, a shape-memory alloy, a thermalresponsive material, a pH-responsive material, a light-responsive material, a moisture-responsive material, a solvent-responsive or chemical exposure-responsive material, an electric field-responsive material, a magnetic field-responsive material, an actuator-embedded material, and/or an unstretched material.

11. The skin treatment device according to claim 8 or 10, wherein said change in a physical characteristic comprises an increase in tension of said microdressing, a decrease in tension of said microdressing, an increase in compressive force exerted by said microdressing, a decrease in compressive force exerted by said microdressing, compression in one or more directions of said microdressing, and/or expansion in one or more directions of said microdressing.

12. A kit comprising the device according to any one of claims 1-11, wherein said kit optionally comprises a sanitizing wipe, an antibiotic ointment, a macrowound dressing, and/or instructions for use.

13. The kit according to claim 12, wherein said macrowound dressing is a tunable dressing.

14. The kit according to claim 12, comprising one or more drugs, wherein the drug is administered into said microwound and wherein preferably said drug is administered as the microwounds are being formed.
